(19) Europäisches Patentamt
European Patent Office
Office européen des brevets

(11) EP 1 521 744 B1

(12) # FASCICULE DE BREVET EUROPEEN

(45) Date de publication et mention de la délivrance du brevet:
**01.04.2009 Bulletin 2009/14**

(21) Numéro de dépôt: **03760017.8**

(22) Date de dépôt: **12.06.2003**

(51) Int Cl.:
***C07D 233/54*** *(2006.01)* ***A61K 31/4164*** *(2006.01)*
***A61K 31/18*** *(2006.01)* ***A61P 25/04*** *(2006.01)*
***C07D 239/06*** *(2006.01)* ***C07D 409/12*** *(2006.01)*
***C07C 311/16*** *(2006.01)* ***C07D 249/08*** *(2006.01)*

(86) Numéro de dépôt international:
**PCT/FR2003/001763**

(87) Numéro de publication internationale:
**WO 2003/106428 (24.12.2003 Gazette 2003/52)**

(54) **DERIVES D'ARYLSULFONAMIDES ET LEUR UTILISATION EN TANT QUE ANTAGONISTES AU RECEPTEUR B1 DE LA BRADYKININE**

ARYLSULPHONAMIDDERIVAT UND DEREN VERWENDUNG ALS B1-BRADYKININ REZEPTOR ANTAGONISTEN

ARYLSULPHONAMIDE DERIVATIVES AND USE THEREOF AS B1 BRADYKININ RECEPTOR ANTAGONISTS

(84) Etats contractants désignés:
**AT BE BG CH CY CZ DE DK EE ES FI FR GB GR HU IE IT LI LU MC NL PT RO SE SI SK TR**

(30) Priorité: **14.06.2002 FR 0207387**

(43) Date de publication de la demande:
**13.04.2005 Bulletin 2005/15**

(73) Titulaire: **LABORATOIRES FOURNIER S.A.**
**21079 Dijon Cedex (FR)**

(72) Inventeurs:
- **BARTH, Martine**
  **F-21380 Asnières les Dijon (FR)**
- **BONDOUX, Michel**
  **F-21121 Fontaine les Dijon (FR)**
- **DODEY, Pierre**
  **F-21121 Fontaine les Dijon (FR)**
- **MASSARDIER, Christine**
  **F-21000 Dijon (FR)**
- **LUCCARINI, Jean-Michel**
  **F-21000 Dijon (FR)**

(74) Mandataire: **Hubert, Philippe et al**
**Cabinet Beau de Loménie**
**158, rue de l'Université**
**75007 Paris (FR)**

(56) Documents cités:
**WO-A-00/75107** **WO-A-98/03503**
**WO-A-02/076964**



Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

Printed by Jouve, 75001 PARIS (FR)

## Description

La présente invention concerne des nouveaux composés de type arylsulfonamide, leur procédé de préparation et leur utilisation pour obtenir des compositions pharmaceutiques.
Ces nouveaux composés sont utiles en thérapeutique, particulièrement pour le traitement de la douleur.

Art antérieur

On connaît déjà des composés comportant dans leur structure un groupement du type arylsulfonamide. Par exemple on peut citer selon EP 236 163 et EP 236 164 des dérivés N-α-arylsulfonylaminoacyl-p-amidino phényl-alaninamides qui sont des inhibiteurs sélectifs de la thrombine et sont utiles comme anti-thrombotiques. On connaît aussi, selon EP 614 911, des composés de structure assez proche des précédentes, comportant simultanément un groupe arylsulfamoyle et un groupe phénylamidine substitué, qui ont la propriété de se fixer sur les récepteurs du neuropeptide Y et qui peuvent présenter une utilité pour soigner l'hypertension, l'angine de poitrine, l'athérosclérose, la dépression, l'anxiété, l'inflammation, l'allergie ou les surcharges graisseuses.
EP 558 961 suggère également l'utilisation de composés du type arylsulfonamide d'acides aminés substitués pour le traitement de la thrombose en raison de propriétés anticoagulantes.
Des études relatives aux propriétés antithrombotiques de composés présentant dans leur structure un groupe arylsulfonamide et un groupe phénylamidine, ont également été publiées dans Pharmazie 1984 vol. 39 (5) pages 315-317 et Pharmazie 1986 vol 41 (4) p 233-235.
Dans un même domaine d'activité pharmacologique, WO 92/16549 A1 décrit des dérivés de la phénylalanine comportant un groupe arylsulfonamide, qui sont des inhibiteurs de protéinase, notamment des inhibiteurs de la thrombine.
On connaît aussi, selon WO 97/25315, des composés de structure N-(arylsulfonyl)amino-acides, utiles pour traiter les maladies inflammatoires.
Dans un domaine thérapeutique différent, WO 00/34313 décrit des peptides qui peuvent comporter en extrémité de chaîne un groupe arylsulfonyl et qui sont revendiqués pour leur aptitude à inhiber la procollagène-C-protéinase. On connaît également par la publication J. Chem. Soc., Perkin Trans. 1(1986), (9) p 1655-64 des composés de structure proche qui sont présentés comme des inhibiteurs de l'élastase pancréatique porciné.
Parmi les documents de l'art antérieur proposant des éléments de structure de type arylsulfonamide, on peut citer WO 96/40639, WO 97/24349, WO 98/03503, WO 98/24783 et WO 99/00387, relatifs à des composés antagonistes du récepteur $B_2$ de la bradykinine.

## Objet de l'invention

L'invention concerne de nouveaux composés comportant l'enchaînement arylsulfonamide substitué, lesdits composés étant notamment utiles en tant que principes actifs de médicaments destinés au traitement de la douleur, particulièrement les hyperalgésies et les algésies majeures.

## Description

Selon la présente invention, on propose en tant que produit industriel nouveau, un composé de type arylsulfonamide caractérisé en ce qu'il est choisi parmi l'ensemble constitué par

i) les composés de formule :

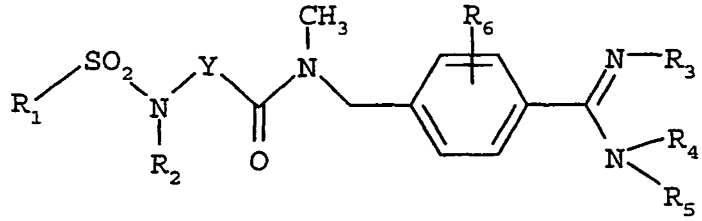

I

dans laquelle

$R_1$ représente un noyau aromatique non substitué ou substitué par un ou plusieurs des atomes ou groupes

d'atomes choisi(s) parmi les halogènes, les groupes alkyle en $C_1$-$C_3$, alcoxy en $C_1$-$C_3$, nitro, cyano, trifluorométhyl ou trifluorométhoxy,
$R_2$ représente un atome d'hydrogène ou un groupe alkyle en $C_1$-$C_4$ éventuellement substitué par un groupe phényle, par un groupe $CONH_2$ ou par un ou plusieurs atomes de fluor,
$R_3$ représente un atome d'hydrogène, un groupe hydroxy, ou forme avec $R_4$ un groupe -CH=N- ou un groupe alkylène en $C_2$-$C_4$ linéaire ou ramifié,
$R_4$ représente un atome d'hydrogène ou forme avec $R_3$ un groupe -CH=N- ou un groupe alkylène en $C_2$-$C_4$ linéaire ou ramifié,
$R_5$ représente un atome d'hydrogène ou un groupe alkyle en $C_1$-$C_3$,
$R_6$ représente un atome d'hydrogène ou un halogène,
Y représente un groupe alkylène en $C_2$-$C_4$, saturé ou insaturé, linéaire ou ramifié, éventuellement interrompu entre deux atomes de carbone par un atome d'oxygène,

ii) les sels d'addition des composés de formule I ci-dessus avec un acide.

Selon l'invention, on préconise aussi un procédé pour la préparation des composés de formule I ainsi que de leurs sels d'addition.

On préconise également l'utilisation d'une substance choisie parmi les composés de formule I et leurs sels d'addition non toxiques pour la préparation d'un médicament, utile en thérapeutique humaine ou animale, destiné à la prévention ou au traitement de pathologies liées à la douleur, notamment les hyperalgésies consécutives à un état inflammatoire ou les algésies majeures liées à d'autres états pathologiques tels que, par exemple, le cancer.

**Description détaillée**

Dans la formule I, on entend par groupe alkyle en $C_1$-$C_4$ une chaîne hydrocarbonée ayant de 1 à 4 atomes de carbone, linéaire, ramifiée, ou bien encore cyclique. Un tel groupe est notamment un groupe méthyle, éthyle, propyle, butyle, 1-méthyl-éthyle, 1-méthylpropyle, 2-méthylpropyle, 1,1-diméthyléthyle, cyclopropyle et cyclopropylméthyle.

On entend par groupe alkyle en $C_1$-$C_4$ substitué par un groupe phényle un groupe alkyle en $C_1$-$C_4$ dont l'un des atomes d'hydrogène est remplacé par un groupe phényle. Un tel groupe est notamment un groupe phénylméthyle, un groupe 2-(phényl)-éthyle, un groupe 1-(phényl)éthyle, un groupe phénylpropyle ou un groupe phénylbutyle.

On entend par groupe alkyle en $C_1$-$C_4$ substitué par un groupe $CONH_2$ un groupe alkyle en $C_1$-$C_4$ dont l'un des atomes de carbone est remplacé par un groupe $CONH_2$. Un tel groupe est par exemple un groupe $-CH_2-CONH_2$, $-(CH_2)_2-CONH_2$, $-CH(CH_3)-CH_2-CONH_2$, ou encore - $(CH_2)_4-CONH_2$.

On entend par halogène un atome de fluor, de chlore ou de brome et, préférentiellement, un atome de fluor ou de chlore.

Par noyau aromatique, on entend un noyau phényle, un noyau 1- ou 2-naphtyle ou un noyau 2- ou 3-thiényle.

Par groupe alcoxy en $C_1$-$C_3$, on comprend un groupe OR dans lequel R est un groupe alkyle en $C_1$-$C_3$, le terme alkyle ayant la signification donnée ci-dessus. Un tel groupe est par exemple un groupe méthoxy, éthoxy, propoxy ou 1-méthyléthoxy.

Par groupe alkylène en $C_2$-$C_4$ saturé, il faut comprendre un groupe $-(CH_2)_n-$ dans lequel n est 2, 3 ou 4 s'il s'agit d'un groupe linéaire ou par exemple un groupe $-CH(CH_3)-CH_2-CH_2-$ou $-C(CH_3)_2-CH_2-$ s'il s'agit d'un groupe ramifié. Dans le cas d'un groupe alkylène interrompu par un atome d'oxygène, on entend par exemple les groupes $-CH_2-CH_2-O-CH_2-$, $-CH_2-O-CH_2-CH_2-$ ou encore $-CH_2-CH_2-O-CH_2-CH_2-$. Par groupe alkylène en $C_2$-$C_4$ insaturé, il faut comprendre un groupe comprenant 2 à 4 atomes de carbone dont 2 consécutifs sont liés par une liaison éthylénique, par exemple un groupe $-CH_2-CH=CH-CH_2-$, $-CH=CH-CH_2-$, $-CH=C(CH_3)-CH_2-$, $CH_2-CH=CH-$ ou $-CH=CH-CH(CH_3)-$.

Par sels d'addition, on entend les sels d'addition obtenus par réaction d'un composé de formule I contenant au moins une fonction basique sous sa forme non salifiée, avec un acide minéral ou organique. De préférence, il s'agira de sels d'addition pharmaceutiquement acceptables.

Parmi les acides minéraux convenant pour salifier un composé basique de formule I, on préfère les acides chlorhydrique, bromhydrique, phosphorique et sulfurique. Parmi les acides organiques convenant pour salifier un composé basique de formule I, on préfère les acides méthanesulfonique, benzènesulfonique, toluènesulfonique, maléïque, fumarique, oxalique, citrique, tartrique, lactique et trifluoroacétique.

Selon un aspect préféré, l'invention a pour objet les composés de formule :

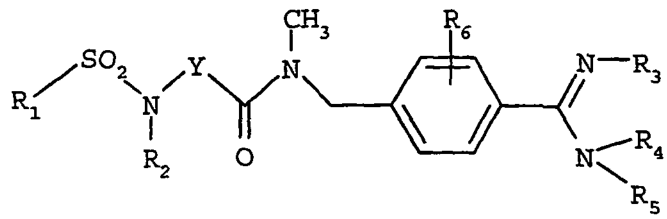

Ia

dans laquelle

$R_1$, $R_2$, $R_5$, $R_6$ et Y sont tels que définis pour les composés de formule I,
$R_3$ représente un atome d'hydrogène, un groupe hydroxy, ou forme avec $R_4$ un groupe alkylène en $C_2$-$C_4$ linéaire ou ramifié,
$R_4$ représent un atome d'hydrogène ou forme avec $R_3$ un groupe alkylène en $C_2$-$C_4$ linéaire ou ramifié.

Parmi les composés de formule I ou Ia, on préfère ceux dans lesquels $R_1$ représente un noyau phényle substitué par un ou plusieurs atomes ou groupes d'atomes choisis parmi un atome d'halogène, de préférence le chlore et les groupes alkyle en $C_1$-$C_3$ et alcoxy en $C_1$-$C_3$.
On préfère également :

- ceux dans lesquels $R_2$ représente un groupe allyle en $C_1$-$C_4$ ;
- ceux dans lesquels $R_3$ et $R_4$ forment ensemble un groupe alkylène en $C_2$-$C_3$ ;
- ceux dans lesquels $R_5$ et $R_6$ représentent chacun un atome d'hydrogène ; et
- ceux dans lesquels Y représente une chaine alkylène en $C_2$-$C_4$ saturée, éventuellement interrompue par un atome d'oxygène, notamment un groupe $-(CH_2)_4-$ ou $-(CH_2)_2-O-CH_2-$.

Selon l'invention, on préconise un procédé général de préparation des composés de l'invention et de leurs sels d'addition, comprenant les étapes consistant à :

1) faire réagir un acide de formule :

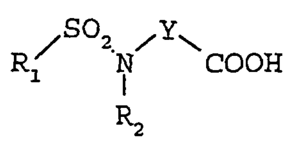

II

dans laquelle

$R_1$ représente un noyau aromatique non substitué ou substitué par un ou plusieurs des atomes ou groupes d'atomes choisi(s) parmi les halogènes, les groupes alkyle en $C_1$-$C_3$, alcoxy en $C_1$-$C_3$, nitro, cyano, trifluorométhyl ou trifluorométhoxy,
$R_2$ représente un atome d'hydrogène, un groupe alkyle en $C_1$-$C_4$ éventuellement substitué par un groupe phényle, par un groupe $CONH_2$ ou par un ou plusieurs atomes de fluor,
et Y représente un groupe alkylène en $C_2$-$C_4$, saturé ou insaturé, linéaire ou ramifié, éventuellement interrompu entre deux atomes de carbone par un atome d'oxygène,
avec une amine de formule :

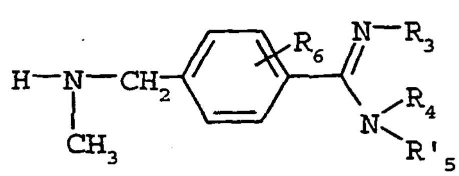

III

dans laquelle

$R_3$ représente un atome d'hydrogène ou forme avec $R_4$ un groupe alkylène en $C_2$-$C_4$ linéaire ou ramifié,

$R_4$ représente un atome d'hydrogène ou forme avec $R_3$ un groupe alkylène en $C_2$-$C_4$ linéaire ou ramifié,

$R'_5$ représente un groupe alkyle en $C_1$-$C_3$, un atome d'hydrogène ou un groupe amino-protecteur, par exemple le groupe Boc (1,1- diméthyl-éthoxycarbonyle),

$R_6$ représente un atome d'hydrogène ou un halogène,

la réaction étant conduite dans un solvant comme par exemple le dichlorométhane, en présence d'au moins un agent activateur tel que notamment le 1-(3-diméthylaminopropyl)-3-éthylcarbodiimide (EDCI) et le 1-hydroxy-7-azabenzotriazole (HOAT), à une température généralement comprise entre la température ambiante (soit environ 15°C) et 60°C et de préférence pendant environ 2 à 15 heures pour obtenir l'amide de formule

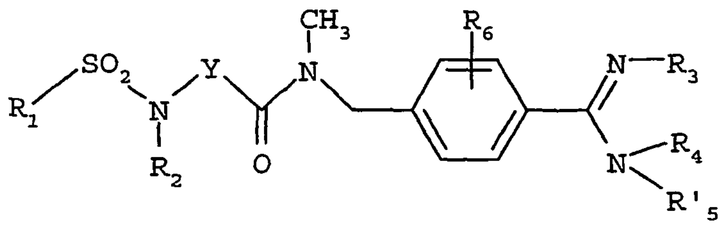

IV

dans laquelle $R_1$, $R_2$, $R_3$, $R_4$, $R'_5$, $R_6$ et Y conservent la même signification que dans les produits de départ,

2) si nécessaire, lorsque le substituant $R'_5$ est un groupe amino-protecteur, faire réagir le composé de formule IV de façon à éliminer le groupe amino protecteur et le remplacer par un atome d'hydrogène, par exemple si $R'_5$ représente le groupe Boc, par action de l'acide trifluoroacétique en présence d'anisole, et ainsi obtenir le composé de formule I dans lequel $R_5$ représente un atome d'hydrogène,

3) si nécessaire, faire réagir le composé de formule IV ou I obtenu ci-dessus, avec un acide minéral ou organique, pour obtenir le sel d'addition du composé de formule IV ou I.

Selon un autre procédé d'obtention, les composés de formule I selon l'invention dans lesquels $R_3$, $R_4$ et $R_5$ représentent un atome d'hydrogène, peuvent être obtenus en effectuant les étapes consistant à :

1) faire réagir un acide de formule :

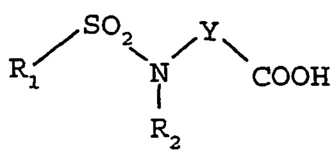

II

dans laquelle

$R_1$ représente un noyau aromatique non substitué ou substitué par un ou plusieurs des atomes ou groupes d'atomes choisi(s) parmi les halogènes, les groupes alkyle en $C_1$-$C_3$, alcoxy en $C_1$-$C_3$, trifluorométhyl ou trifluorométhoxy,

$R_2$ représente un atome d'hydrogène, un groupe alkyle en $C_1$-$C_4$ éventuellement substitué par un groupe phényle, par un groupe $CONH_2$ ou par un ou plusieurs atomes de fluor,

et Y représente un groupe alkylène en $C_2$-$C_4$, saturé ou insaturé, linéaire ou ramifié, éventuellement interrompu entre deux atomes de carbone par un atome d'oxygène,

avec une amine de formule :

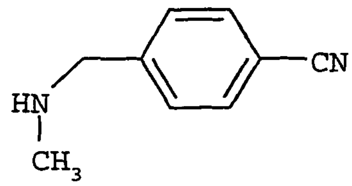

V

la réaction étant conduite dans des conditions analogues à celles décrites précédemment (étape 1 du procédé général), pour obtenir le composé de formule :

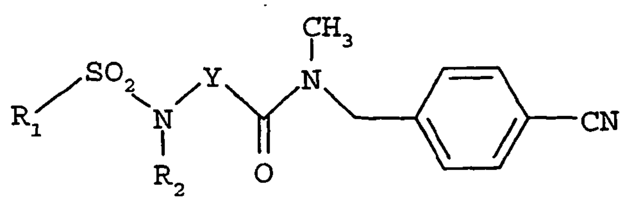

VI

dans laquelle $R_1$, $R_2$, et Y conservent la même signification que dans l'acide de départ,

2) faire réagir le composé de formule VI ci-dessus avec l'hydroxylamine, dans un solvant comme par exemple le diméthylsulfoxide (DMSO) et en présence de triéthylamine, à une température généralement proche de la température ambiante, pendant environ 2 à 15 heures, pour obtenir le composé de formule :

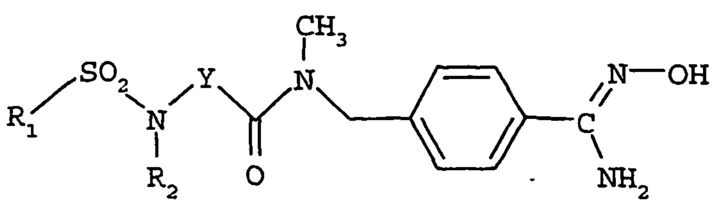

VII

dans laquelle $R_1$, $R_2$ et Y restent inchangés,

3) faire réagir le composé de formule VII avec l'anhydride acétique, dans un solvant tel que par exemple le dichlorométhane, à une température généralement proche de la température ambiante et pendant environ 2 à 15 heures, pour obtenir le composé de formule :

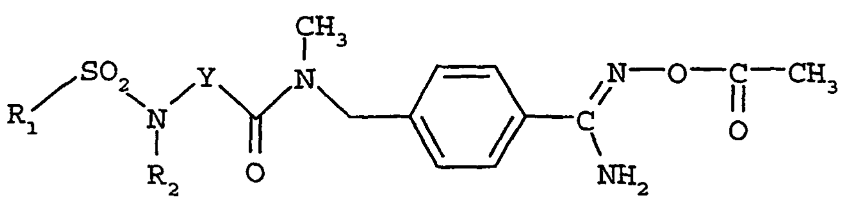

VIII

4) effectuer une réduction par hydrogénation catalytique du composé de formule VIII, par exemple en présence de charbon palladié, dans un solvant tel que par exemple le méthanol, à une température proche de la température ambiante, pour obtenir le composé de formule :

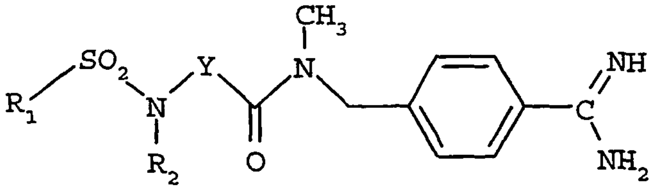

IX

dans laquelle $R_1$, $R_2$ et Y restent inchangés,
5) si nécessaire, obtenir le sel du composé de formule IX par addition de l'acide approprié.

Les composés de formule I dans lesquels $R_3$ et $R_4$ forment ensemble un groupe -CH=N-peuvent être obtenus au départ du composé de formule VI, en faisant réagir successivement :

1) le sulfure d'hydrogène, en utilisant par exemple la pyridine comme solvant et en travaillant à température ambiante, de façon à obtenir un groupe thioamide (aminothioxométhyle) en lieu et place du groupe cyano initial,
2) l'iodure de méthyle, dans un solvant tel que l'acétone, afin d'obtenir un groupe iminométhylthiométhyl en lieu et place du groupe thioamide,
3) la formylhydrazine, dans un solvant tel que l'éthanol, pour obtenir le cycle triazole en lieu et place du groupe iminométhylthiométhyl précédent.

Selon un autre mode de préparation, les composés de formule I selon l'invention peuvent être obtenus en effectuant les étapes consistant à :

1) faire réagir une amine de formule :

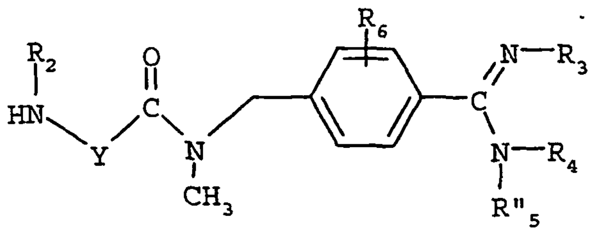

X

dans laquelle

$R_2$ représente un atome d'hydrogène, un groupe alkyle en $C_1$-$C_4$ éventuellement substitué par un groupe phényle, par un groupe $CONH_2$ ou par un ou plusieurs atomes de fluor,
$R_3$ représente un atome d'hydrogène ou forme avec $R_4$ un groupe alkylene en $C_2$-$C_4$ linéaire ou ramifié,
$R_4$ représente un atome d'hydrogène ou forme avec $R_3$ un groupe alkylène en $C_2$-$C_4$ linéaire ou ramifié,
$R''_5$ représente un groupe amino protecteur, notamment le groupe Boc
$R_6$ représente un atome d'hydrogène ou un halogène,
Y représente un groupe alkylène en $C_2$-$C_4$, linéaire ou ramifié, éventuellement interrompu par un atome d'oxygène,
avec un chlorure d'arylsulfonyle de formule :

$R_1$-$SO_2Cl$ XI

dans laquelle $R_1$ représente $R_1$ un noyau aromatique non substitué ou substitué par un ou plusieurs des atomes ou groupes d'atomes choisi(s) parmi les halogènes, les groupes alkyle en $C_1$-$C_3$, alcoxy en $C_1$-$C_3$, nitro, cyano, trifluorométhyl ou trifluorométhoxy,
la réaction étant conduite dans un solvant tel que par exemple le dichlorométhane, en présence d'une base aprotique telle que la triéthylamine, à température ambiante et pendant environ 1 à 12 heures, pour obtenir le composé de formule :

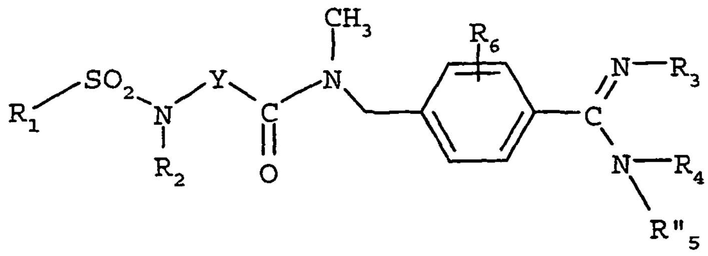

IV

dans laquelle $R_1$, $R_2$, $R_3$, $R_4$, $R''_5$, $R_6$ et Y restent inchangés.

2) faire réagir le composé de formule IV ci-dessus de façon à éliminer le groupe amino protecteur $R''_5$ et le remplacer par un atome d'hydrogène, par exemple, si $R''_5$ représente le groupe Boc, par action de l'acide trifluoroacétique en présence d'anisole, et ainsi obtenir le composé de formule I dans lequel $R_5$ représente un atome d'hydrogène.

Les composés de formule II :

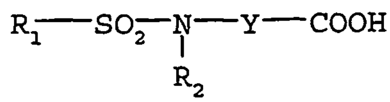

II

peuvent être obtenus notamment par action d'un chlorure d'arylsulfonyle

$R_1$-$SO_2Cl$ XI

avec une amine

$R_2$ - $NH_2$ XII,

puis réaction de l'amide obtenue successivement avec l'hydrure de sodium et un acide halogéné de formule

X-Y-COOH XIII

dans laquelle X représente un halogène, préférentiellement le brome, et Y représente un groupe alkylène en $C_2$ - $C_4$, pour obtenir l'acide de formule II.

Les composés de formule III :

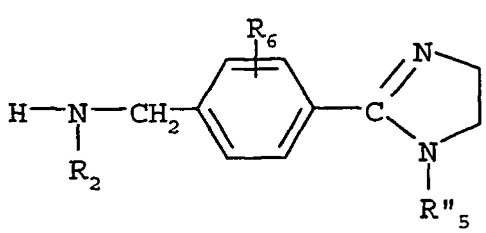

III

peuvent être obtenus selon un procédé consistant à :

1) faire réagir une amine de formule

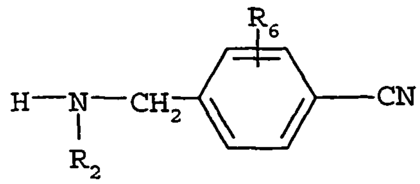

XIV

avec le chloroformiate de benzyle, dans un solvant tel que par exemple le dichlorométhane et en présence d'une base aprotique pour obtenir le composé de formule :

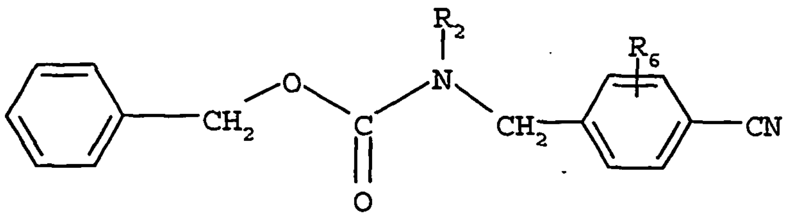

XV

2) faire réagir le composé de formule XV ci-dessus avec l'éthylènediamine, de façon à obtenir le composé de formule :

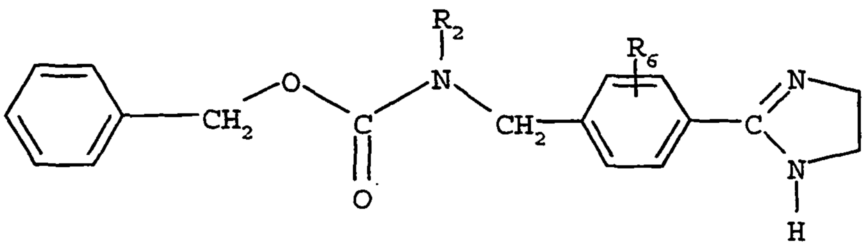

XVI

3) faire réagir le composé de formule XVI avec le dicarbonate de di-*tert*-butyle, dans un solvant et en présence d'une base aprotique, de façon à obtenir le produit de formule :

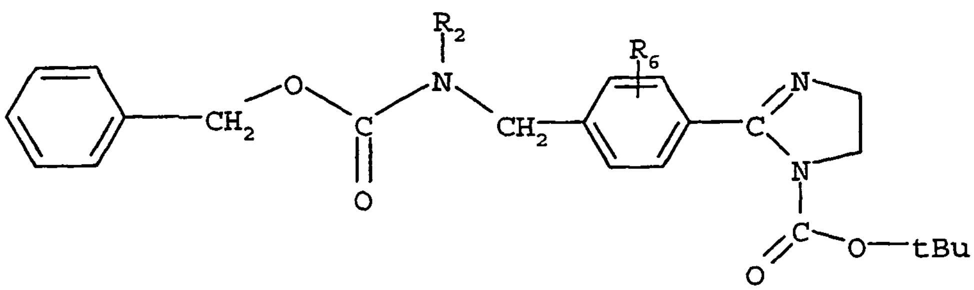

XVII

4) effectuer une déprotection partielle du composé de formule XVII, par hydrogénation catalytique par exemple en présence de charbon palladié, de façon à obtenir l'amine attendue de formule III
dans laquelle $R''_5$ est le groupe protecteur Boc

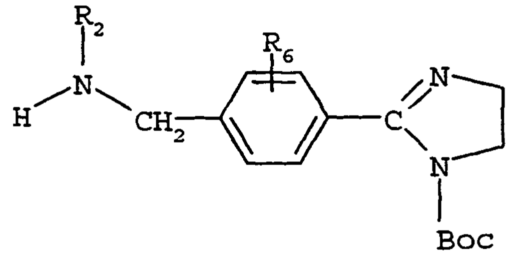

III

L'invention sera mieux comprise à la lecture des exemples de préparation ainsi que des résultats d'essais pharmacologiques réalisés avec des composés selon l'invention.

Parmi les abréviations utilisées dans les descriptions suivantes, M signifie mole, mM signifie millimole ($10^{-3}$ mole). DCM signifie dichlorométhane, DMSO signifie diméthylsulfoxyde.

**PREPARATION I**

**Acide [(4-cyanophényl)méthyl]méthylcarbamique, phénylméthyl ester**

On prépare un mélange de 7 g (47,9 mM) de [(4-cyanophényl)méthyl]méthanamine dans 60 ml de DCM et on ajoute 5,8 g (57,5 mM) de triéthylamine. Le mélange est refroidi à 0 ˚C et on ajoute goutte à goutte une solution de 9,8 g (57,5 mM) de chloroformiate de benzyle dans 20 ml de DCM. Le mélange est ensuite agité pendant 20 heures à température ambiante puis lavé par une solution d'acide chlorhydrique 0,1 N, puis par de l'eau, séché sur sulfate de sodium et concentré sous pression réduite. Le résidu est purifié par chromatographie sur gel de silice en éluant à l'aide d'un mélange toluène/acétate d'éthyle (95/5 ; v/v). On obtient ainsi 11,4 g du produit attendu sous forme d'une huile (rendement = 87 %).
$n_D^{22}$ = 1,564

**PREPARATION II**

**Acide [[4,(4,5-dihydro-1*H*-imidazol-2-yl)phényl]méthyl]méthylcarbamique, phénylméthyl ester**

On prépare un mélange de 11,3 g (40 mM) du composé obtenu selon la préparation I dans 40 ml d'éthylènediamine et on ajoute 0,64 g (20 mM) de fleur de soufre. Le mélange réactionnel est agité pendant 2 heures à 100 ˚C puis refroidi. On ajoute de l'eau et on extrait par l'acétate d'éthyle. La phase organique est lavée à l'eau, séchée sur sulfate de sodium et concentrée sous pression réduite. Le résidu est purifié par chromatographie sur gel de silice en éluant à l'aide d'un mélange dichlorométhane/méthanol/ammoniaque (95/5/0,05 ; v/v /v). On obtient ainsi 11 g de produit attendu sous forme d'un solide blanc (rendement = 85 %).
F=84˚C

**PREPARATION III**

**Acide 4,5-dihydro-2-[4-[[méthyl[(phénylméthoxy)carbonyl]amino]méthyl]phényl]-1*H*-imidazole-1-carboxylique, 1,1-diméthyléthyl ester**

On prépare une solution de 3,22 g (10 mM) du composé obtenu selon la préparation II dans 45 ml de DCM, et on ajoute 1,34 g (11 mM) de N,N-diméthylaminopyridine, puis, goutte à goutte, une solution de 2,4 g (11 mM) de di-*tert*-butyl dicarbonate dans 45 ml de DCM. Le mélange réactionnel est agité pendant 2 heures à température ambiante puis lavé à l'aide d'une solution d'acide chlorhydrique 0,5 N, puis avec de l'eau. La phase organique est séchée sur sulfate de sodium puis concentrée sous pression réduite. Le résidu est cristallisé dans l'éther isopropylique puis filtré et séché. On obtient ainsi 4 g du produit attendu sous forme de fins cristaux blancs (rendement = 94 %).
F=124˚C

**PREPARATION IV**

**Acide 4,5-dihydro-2-[4-[(méthylamino)méthyl]phényl]-1*H*-imidazole-1-carboxylique, 1,1-diméthyléthyl ester**

On prépare un mélange de 4,23 g (10 mM) du composé obtenu selon la préparation III dans 80 ml de méthanol et on ajoute 0,4 g de charbon palladié (à 10 % Pd). Le mélange est agité sous atmosphère d'hydrogène à température ambiante et pression atmosphérique pendant 2 heures. Le catalyseur est éliminé par filtration puis le filtrat est concentré sous pression réduite. Le résidu et purifié par chromatographie sur gel de silice en éluant à l'aide d'un mélange dichlorométhane/méthanol/ammoniaque (90/10/0,1 ; v/v/v). On obtient ainsi 2,5 g du produit attendu sous forme d'un solide blanc cassé (rendement = 90 %).
F=65°C

**PREPARATION V**

**N-méthyl-2,4-dichloro-3-méthylbenzènesulfonamide**

On prépare une suspension de 2,55 g (37,8 mM) de chlorhydrate de méthanamine dans 120 ml de dichlorométhane (DCM) et on ajoute 7,5 g de chlorure de 2,4-dichloro-3-méthylbenzènesulfonyle en solution dans 30 ml de DCM et 10,5 ml de triéthylamine ; le mélange réactionnel est maintenu sous agitation pendant 15 heures à température ambiante puis lavé avec une solution d'acide chlorhydrique 1N, avec une solution de bicarbonate de sodium, puis à l'eau. La phase organique est séchée sur sulfate de magnésium puis concentrée sous pression réduite. Le résidu solide obtenu est purifié par chromatographie sur gel de silice en éluant à l'aide d'un mélange cyclohexane/acétate d'éthyle (8/2, v/v). On obtient ainsi 6,2 g du composé attendu sous forme d'un solide blanc (rendement = 85%).
F = 118°C

**PREPARATION VI**

**Acide 5-[[(2,4-dichloro-3-méthylphényl)sulfonyl]méthylamino]pentanoïque, éthyl ester**

On prépare une solution de 1g (3,9 mM) du composé obtenu selon la préparation V dans 30 ml de diméthylformamide puis on ajoute 1,63 g (11,8 mM) de carbonate de potassium puis 987 mg (4,7 mM) de 5-bromopentanoate d'éthyle. Le mélange réactionnel est agité à température ambiante pendant 15 heures, puis additionné d'eau et extrait par l'acétate d'éthyle. La phase organique est lavée à l'eau plusieurs fois puis séchée sur sulfate de magnésium et concentrée sous pression réduite. On obtient ainsi 1,5 g du produit attendu sous forme d'une huile incolore (rendement quantitatif).
RMN $^1$H (300 MHz, DMSO) δ : 7,83 (d, 1H) ; 7,65 (d, 1H) ; 4,04 (q, 2H) ; 3,19 (t, 2H) ; 2,80 (s, 3H) ; 2,49 (s, 3H) ; 2,28 (t, 2H) ; 1,49 (m, 4H) ; 1,17 (t, 3H).

**PREPARATION VII**

**Acide 5-[[(2,4-dichloro-3-méthylphényl)sulfonyl]méthylamino]pentanoique.**

On prépare une solution de 1,45 g (3,79 mM) de l'ester obtenu selon la préparation VI dans 15 ml de tétrahydrofurane (THF) et on ajoute 320 mg (7,56 mM) de lithine et 30 ml d'eau. Le mélange réactionnel est agité pendant 15 heures à température ambiante. Le THF est chassé sous pression réduite et la phase aqueuse résiduelle est additionnée de 50 ml d'eau, acidifiée à l'aide d'une solution d'acide chlorhydrique N, puis extraite à l'aide d'acétate d'éthyle. La phase organique obtenue est lavée à l'eau, séchée sur sulfate de magnésium et concentrée sous pression réduite. On obtient ainsi 1,3 g de l'acide attendu sous forme d'une huile incolore (rendement = 97%).
RMN $^1$H (300 MHz, DMSO) δ : 12,0 (s large, 1H) ; 7,83 (d, 1H) ; 7,64 (d, 1H) ; 3,20 (t, 2H) ; 2,80 (s, 3H) ; 2,57 (s, 3H) ; 2,20 (t, 2H) ; 1,49 (m, 4H).

**PREPARATION VIII**

**Acide 2-[4-[[[5-[[(2,4-dichloro-3-méthylphényl)sulfonyl]méthylamino]-1-oxopentyl]méthylamino]méthyl]phényl]-4,5-dihydro-1*H*-imidazole-1-carboxylique, 1,1-diméthyléthyl ester.**

On prépare une solution de 200 mg (0,565 mM) de l'acide obtenu selon la préparation VII, dans 15 ml de dichlorométhane, puis on ajoute 120 mg de EDCI et 90 mg de HOAT. Le mélange est agité à température ambiante

pendant 20 min puis on ajoute 163 mg (0,565 mM) de l'amine obtenue selon la préparation IV, en solution dans 3 ml de dichlorométhane. Le mélange réactionnel est maintenu sous agitation à température ambiante pendant 15 heures, puis dilué par 40 ml de dichlorométhane. Cette phase organique est lavée à l'eau puis séchée et concentrée sous pression réduite. Le produit huileux obtenu est purifié par chromatographie sur gel de silice en éluant à l'aide d'un mélange dichlorométhane/méthanol (98/2, v/v).
On obtient ainsi 260 mg du composé attendu sous forme d'une huile incolore (rendement = 73%)
RMN $^1$H (300 MHz, DMSO) δ : 7,80 (t, 1H) ; 7,60 (m, 1H) ; 7,40 (m, 2H) ; 7,20 (m, 2H) ; 4,52 (d, 2H) ; 3,84 (m, 4H) ; 3,20 (m, 2H) ; 2,88 (s, 3H) ; 2,81 (s, 3H) ; 2,79 (m, 2H) ; 2,49 (s, 3H) ; 2,38 (m, 2H) ; 2,30 (m, 4H) ; 1,17 (s, 9R).

## Exemple 1

**5-[[(2,4-dichloro-3-méthylphényl)sulfonyl]méthylamino]-N-[[4-(4,5-dihydro-1*H*-imidazol-2-yl)phényl]méthyl]-N-méthyl-pentanamide, trifluoroacétate**

On prépare un mélange de 240 mg du composé obtenu selon la préparation VIII, 5 ml d'acide trifluoroacétique, 5 ml de dichlorométhane et 41 mg d'anisole et on maintient ce mélange sous agitation pendant 15 heures à température ambiante. Les solvants sont chassés sous pression réduite une première fois, puis une seconde fois en présence de toluène. L'huile résiduelle est agitée avec de l'éther isopropylique que l'on élimine ensuite. Le résidu huileux est repris par de l'eau pure. La solution est filtrée et le filtrat est lyophilisé. On obtient ainsi 240 mg du produit attendu sous forme d'un solide cotonneux blanc (rendement = 97 %).
F = 83°C
En opérant de façon analogue à la préparation VI, on obtient les composés suivants :

## PREPARATION IX

**Acide 4-[[(2,4-dichloro-3-méthylphényl)sulfonyl]méthylamino]butanoïque, éthyl ester (non isolé)**

## PREPARATION X

**N-[(2,4-dichloro-3-méthylphényl)sulfonyl)-N-méthyl-β-alanine, éthyl ester**

(huile incolore, rendement = 43%)
RMN $^1$H (300 MHz, DMSO) δ : 7,83 (d, 1H) ; 7,66 (d, 1H) ; 4,04 (q, 2H) ; 3,46 (t, 2H) ; 2,85 (s, 3H) ; 2,59 (t, 2H) ; 2,5 (s, 3H) ; 1,17 (t, 3H).

## PREPARATION XI

**Acide 4-[[(2,4-dichloro-3-méthylphényl)sulfonyl]méthylaminol-(2E)-2-butènoïque, éthyl ester**

(solide blanc, rendement = 53%)
F = 90°C
En opérant de façon analogue à la préparation VII, on obtient les composés suivants :

## PREPARATION XII

**Acide 4-[[(2,4-dichloro-3-méthylphényl)sulfonyl]méthylamino]butanoïque**

(solide blanc, rendement quantitatif)
F = 104°C

## PREPARATION XIII

**N-[(2,4-dichloro-3-méthylphényl)sulfonyl]-N-méthyl-β-alanine**

(solide blanc, rendement = 98%).
F = 119 °C

**PREPARATION XIV**

**Acide 4-[[(2,4-dichloro-3-méthylphényl)sulfonyl]méthylamino]-(2E)-2-butènoïque**

(solide blanc, rendement = 47%).
F = 160°C

En opérant de façon analogue à la préparation VIII, on obtient les composés suivants :

**PREPARATION XV**

**Acide 2-[4-[[[4-[[(2,4-dichloro-3-méthylphényl)sulfonyl]méthylamino]-1-oxobutyl]méthylamino]méthyl]phényl]-4,5-dihydro-1*H*-imidazole-1-carboxylique, 1,1-diméthyléthyl ester**

(huile, rendement = 34%)
RMN $^1H$ (300 MHz, DMSO) δ : 7,95 (m, 1H) ; 7,82 (m, 1H) ; 7,41 (m, 2H) ; 7,21 (m, 2H) ; 4,52 (d, 2H) ; 3,84 (m, 4H) ; 3,22 (m, 2H) ; 2,85 (m, 6H) ; 2,49 (s, 3H) ; 2,24 (m, 2H) ; 1,79 (m, 2H) ; 1,17 (s, 9H).

**PREPARATION XVI**

**Acide 2-[4-[[[3-[[(2,4-dichloro-3-méthylphényl)sulfonyl]méthylamino]-1-oxopropyl]méthylamino]méthyl]phényl]-4,5-dihydro-1*H*-imidazole-1-carboxylique, 1,1-diméthyléthyl ester**

(mousse écrue, rendement = 55%)
F = 50°C

**PREPARATION XVII**

**Acide 2-[4-[[[(2E)-4-[[(2,4-dichloro-3-méthylphényl)sulfonyl]méthylamino]-1-oxo-2-butènyl]méthylamino]méthyl]phényl]-4,5-dihydro-1*H*-imidazole-1-carboxylique, 1,1-diméthyléthyl ester.**

(solide blanc, rendement = 74%)
F = 65°C

En opérant de façon analogue à l'exemple 1, on obtient les 3 composés suivants :

**Exemple 2**

**4-[[(2,4-dichloro-3-méthylphényl)sulfonyl]méthylamino]-N-[[4-(4,5-dihydro-1*H*-imidazol-2-yl)phényl]méthyl]-N-méthyl-butanamide, trifluoroacétate**

(solide blanc, rendement = 92%)
F = 90°C

**Exemple 3**

**3-[[(2,4-dichloro-3-méthylphényl)sulfonyl]méthylamino]-N-[[4-(4,5-dihydro-1*H*-imidazol-2-yl)phényl]méthyl]-N-méthyl-propanamide, trifluoroacétate**

(solide blanc, rendement = 87%)
F = 65°C

## Exemple 4

**4-[[(2,4-dichloro-3-méthylphényl)sulfonyl]méthylamino]-N-[[4-(4,5-dihydro-1*H*-imidazol-2-yl)phényl]méthyl]-N-méthyl-(2 E)-2-butènamide, trifluoroacétate**

### PREPARATION XXV

**Acide 4-[[(2,3-dichlorophényl)sulfonyl]méthylamino]butanoïque, éthyl ester**

(huile jaune, rendement = 69%)
RMN $^1H$ (250 MHz, DMSO) $\delta$ : 7,93 (m, 2H) ; 7,57 (t, 1H) ; 4,03 (q, 2H) ; 3,24 (t, 2H) ; 2,84 (s, 3H) ; 2,29 (t, 2H) ; 1,75 (m, 2H) ; 1,17 (t, 3H).

### PREPARATION XXVI

**Acide 5-[[(2,3-dichlorophényl)sulfonyl]méthylamino]pentanoique, éthyl ester**

(non isolé)

### PREPARATION XXVII

**Acide 4-[[(2,3-dichlorophényl)sulfonyl]méthylamino]-(2E)-2-butènoïque, éthyl ester**

(solide blanc, rendement = 72%)
F = 98°C

### PREPARATION XXVIII

**Acide 5-[[(2,6-dichlorophényl)sulfonyl]méthylamino]pentanoïque, éthyl ester**

(non isolé)

### PREPARATION XXIX

**Acide 4-[[(2,6-dichlorophényl)sulfonyl]méthylamino]-(2E)-2-butènoïque, éthyl ester**

(solide blanc, rendement = 81%)
F = 84°C

### PREPARATION XXX

**Acide 5-[méthyl(1-naphtalénylsulfonyl)amino]pentanoïque, éthyl ester**

(huile incolore, rendement = 93%)
RMN $^1H$ (250 MHz, DMSO) $\delta$ : 8,58 (d, 1H) ; 8,27 (d, 1H) ; 8,08 (m, 2H) ; 7,69 (m, 3H) ; 4,04 (q, 2H) ; 3,16 (t, 2H) ; 2,77 (s, 3H) ; 2,25 (t, 2H) ; 1,45 (m, 4H) ; 1,16 (t, 3H).

### PREPARATION XXXI

**Acide 4-[méthyl(1-naphtalénylsulfonyl)amino]-(2E)-2-butènoïque, éthyl ester**

(solide blanc, rendement = 54%)
F = 75°C

En opérant de façon analogue à la préparation VII, on obtient les composés suivants :

**PREPARATION XXXII**

**Acide 5-[[(2-chlorophényl)sulfonyl]méthylamino]pentanoïque**

(solide blanc, rendement = 75%)
F = 120°C

**PREPARATION XXXIII**

(solide blanc, rendement = 100%)
F = 55°C

En opérant de façon analogue à la préparation V, on obtient les composés suivants :

**PREPARATION XVIII**

**2-chloro-N-méthyl-benzènesulfonamide**

(huile jaune, rendement = 94%)
RMN $^1H$ (300 MHz, DMSO) δ : 7,95 (d, 1H) ; 7,65 (m, 3H) ; 7,54 (t, 1H) ; 2,47 (s, 3H).

**PREPARATION XIX**

**2,3-dichloro-N-méthylbenzènesulfonamide**

(solide écru, rendement = 76%)
F = 126°C

**PREPARATION XX**

**2,6-dichloro-N-méthylbenzènesulfonamide**

(solide blanc, rendement = 99%)
F = 110°C

**PREPARATION XXI**

**N-méthyl-1-naphtalènesulfonamide**

(solide beige, rendement = 94%)
RMN $^1H$ (250 MHz, DMSO) δ : 8,63 (m, 1H) ; 8,23 (d, 1H) ; 8,10 (m, 2H) ; 7,72 (q, 1H) ; 7,66 (m, 3H) ; 2,42 (d, 3H).

En opérant de façon analogue à la préparation VI, on obtient les composés suivants :

**PREPARATION XXII**

**Acide 5-[[(2-chlorophényl)sulfonyl]méthylamino]-pentanoïque, éthyl ester**

(non isolé)

**PREPARATION XXIII**

**Acide 4-[[(2-chlorophényl)sulfonyl]méthylamino]-(2E)-2-butènoïque, éthyl ester**

(huile incolore, rendement = 78%)
RMN $^1H$ (250 MHz, DMSO) δ: 8,02 (d, 1H) ; 7,71 (m, 2H) ; 7,60 (m, 1H) ; 6,79 (m, 1H) ; 6,04 (d, 1H) ; 4,08 (d, 2H) ; 3,67 (s, 3H) ; 2,80 (s, 3H).

**PREPARATION XXIV**

**N-[(2,3-dichlorophényl)sulfonyl]-N-méthyl-β-alanine, éthyl ester**

(huile, rendement = 74%)
RMN $^1$H (250 MHz, DMSO) δ: 7,94 (m, 2H) , 7,57 (m, 1H) ; 4,02 (q, 2H) ; 3,48 (t, 2H) ; 2,88 (s, 3H) ; 2,59 (t, 2H) ; 1,17 (t, 3H).

**Acide 4-[[(2-chlorophényl)sulfonyl]méthylamino]-(2E)-2-butènoïque**

(solide blanc, rendement = 50%)
F =136°C

**PREPARATION XXXIV**

**N-[(2,3-dichlorophényl)sulfonyl]-N-méthyl-β-alanine**

(solide blanc, rendement = 74%)
F = 141°C

**PREPARATION XXXV**

**Acide 4-[[(2,3-dichlorophényl)sulfonyl]méthylamino]butanoïque**

(solide blanc, rendement = 78%)
F = 114°C

**PREPARATION XXXVI**

**Acide 5-[[(2,3-dichlorophényl)sulfonyl]méthylamino]pentanoïque**

(huile incolore, rendement = 63%)
RMN $^1$H (250 MHz, DMSO) δ - 12,0 (s large, 1H) ; 7,94 (d, 2H) ; 7,56 (t, 1H) ; 3,25 (t, 2H) ; 2,73 (s, 3H) ; 2,24 (t, 2H) ; 1,50 (m, 4H).

**PREPARATION XXXVII**

**Acide 4-[[(2,3-dichlorophényl)sulfonyl]méthylaminol-(2E)-2-butènoïque**

(huile incolore, rendement = 60%)
RMN $^1$H (250 MHz, DMSO) δ : 7,96 (m, 2H) ; 7,58 (t, 1H) ; 6,58 (m, 1H) ; 5,90 (d, 1H) ; 4,04 (d, 2H) ; 2,81 (s, 3H).

**PREPARATION XXXVIII**

**Acide 5-[[(2,6-dichlorophényl)sulfonyl]méthylamino]pentanoïque**

(solide blanc écru, rendement = 59%)
F = 105°C

**PREPARATION XXXIX**

**Acide 4-[[(2,6-dichlorophényl)sulfonyl]méthylamino]-(2E)-2-butènoïque**

(solide blanc, rendement = 48%)
F=158°C

## PREPARATION XL

**Acide 5-[méthyl(1-naphtalénylsulfonyl)amino]pentanoïque**

(huile incolore, rendement = 74%)
RMN $^1H$ (300 MHz, DMSO) δ : 12,0 (s large, 1H) ; 8,62 (d, 1H) ; 8,58 (d, 1H) ; 8,25 (m, 2H) ; 8,08 (m, 3H) ; 3,16 (t, 2H) ; 2,77 (s, 3H) ; 2,20 (t, 2H) ; 1,48 (m, 4H).

## PREPARATION XLI

**Acide 4-[méthyl(1-naphtalénylsulfonyl)amino]-(2E)-2-butènoïque**

(solide blanc, rendement = 38%)
RMN $^1H$ (250 MHz, DMSO) δ : 12,5 (s large, 1H) ; 8,60 (d, 1H) ; 8,29 (d, 1H) ; 8,13 (m, 2H) ; 7,75 (m, 3H) ; 6,64 (m, 1H) ; 5,90 (d, 1H) ; 4,03 (d, 2H) ; 2,78 (s, 3H).
En opérant de façon analogue à la préparation VIII, on obtient les composés suivants :

## PREPARATION XLII

**Acide 2-[4-[([5-[[(2-chlorophényl)sulfonyl]méthylamino]-1-oxopentyl] méthylamino]méthyl]phényl]-4,5-di-bydro-1*H*-imidazole-1-carboxylique, 1,1-diméthyléthyl ester**

(huile, rendement = 65%)
RMN $^1H$ (300 MHz, DMSO) δ: 7,97 (t, 1H) ; 7,66 (m, 2H) ; 7,54 (m, 1H) ; 7,44 (m, 2H) ; 7,20 (m, 2H) ; 4,55 (d, 2H) ; 3,84 (m, 4H) ; 3,20 (m, 2H) ; 2,85 (m, 6H) ; 2,36 (m, 2H) ; 1,50 (m, 4H) ; 1,17 (d, 9H).

## PREPARATION XLIII

**Acide 2-[4-[[[(2E)-4-[[(2-chlorophényl)sulfonyl]méthylamino]-1-oxo-2-butènyl]méthylamino]méthyl]phényl]-4,5-dihydro-1*H*-imidazole-1-carboxylique,1,1-diméthyléthyl ester.**

(pâte incolore, rendement = 82%)
RMN $^1H$ (300 MHz, DMSO) δ: 8,03 (t, 1H) ; 7,67 (m, 2H) ; 7,51 (m, 1H) ; 7,42 (m, 2H) ; 7,20 (m, 2H) ; 6,60 (m, 2H) ; 4,60 (d, 2H) ; 4,05 (m, 2H) ; 3,84 (m, 4H) ; 2,88 (m, 6H) ; 1,18 (s, 9H).

## PREPARATION XLIV

**Acide 2-[4-[[[3-[[(2,3-dichlorophényl)sulfonyl]méthylamino]-1-oxopropyl] méthylamino]méthyl]phényl]-4,5-di-bydro-1*H*-imidazole-1-carboxylique, 1,1-diméthyléthyl ester**

(solide blanc, rendement = 42%)
F = 65°C

## PREPARATION XLV

**Acide 2-[4-[[[4-[[(2,3-dichlorophényl)sulfonyl]méthylamino]-1-oxobutyl] méthylamino]méthyl]phényl]-4,5-di-hydro-1*H*-imidazole-1-carboxylique, 1,1-diméthyléthyl ester**

(solide blanc, rendement = 68%)
F = 50°C

## PREPARATION XLVI

**Acide 2-[4-[[[5-[[(2,3-dichlorophényl)sulfonyl]méthylamino]-1-oxopetyl] méthylamino]méthyl]phényl]-4,5-di-hydro-1*H*-imidazole-1-carboxylique, 1,1-diméthyléthyl ester**

(solide blanc, rendement = 47%)
F = 50°C

**PREPARATION XLVII**

**Acide 2-[4-[[[(2E)-4-[[(2,3-dichlorophényl)sulfonyl]méthylamino]-1-oxo-2-butényl] méthylamino]méthyl]phényl]-4,5-dihydro-1*H*-imidazole-1-carboxylique, 1,1-diméthyléthyl ester**

(pâte blanche, rendement = 69%)
RMN $^1$H (250 MHz, DMSO) δ : 8,02 (m, 2H) ; 7,59 (m, 1H) ; 7,43 (m, 2H) ; 7,21 (m, 2H) ; 6,60 (m, 2H) ; 4,70 (d, 2H) ; 4,00 (m, 2H) ; 3,82 (m, 4H) ; 2,86 (m, 6H) ; 1,18 (s, 9H).

**PREPARATION XLVIII**

**Acide 2-[4-[[[5-[[(2,6-dichlorophényl)sulfonyl]méthylamino]-1-oxopentyl] méthylamino]méthyl]phényl]-4,5-dihydro-1*H*-imidazole-1-carboxylique,1,1-diméthyléthyl ester**

(huile, rendement = 5 1 %)
RMN $^1$H (250 MHz, DMSO) δ : 7,67 (m, 2H) ; 7,64 (m, 1H) ; 7,42 (m, 2H) ; 7,20 (m, 2H) ; 4,55 (d, 2H) ; 3,84 (m, 4H) ; 3,23 (m, 2H) ; 2,83 (m, 6H) ; 2,39 (m, 2H) ; 1,55 (m, 4H) ; 1,17 (s, 9H).

**PREPARATION IL**

**Acide 2-[4-[[[(2E)-4-[[(2,6-dichlorophényl)sulfonyl]méthylamino]-1-oxo-2-butènyl] méthylamino]méthyl]phényl]-4,5-dihydro-1*H*-imidazole-1-carboxylique, 1,1-diméthyléthyl ester**

(solide blanc, rendement = 70%)
F = 70°C

**PREPARATION L**

**Acide 2-[4-[[[5-[méthyl[(1-naphtalényl)sulfonyl]amino]-1-oxopentyl] méthylamino] méthyl]phényl]-4,5-dihydro-1*H*-imidazole-1-carboxylique, 1,1-diméthyléthyl ester**

(solide blanc, rendement = 70%)
RMN $^1$H (300 MHz, DMSO) δ: 8,60 (m, 1H) ; 8,25 (d, 1H) ; 8,07 (m, 2H) ; 7,65 (m, 3H) ; 7,43 (m, 2H) ; 7,24 (m, 2H) ; 4,53 (d, 2H) ; 3,84 (m, 4H) ; 3,17 (m, 2H) ; 2,80 (m, 6H) ; 2,35 (m, 2H) ; 1,48 (m, 4H) ; 1,17 (s, 9H).

**PREPARATION LI**

**Acide 2-[4-[[[(2E)-4-[méthyl[(1-naphtalényl)sulfonyl]amino]-1-oxo-2-butènyl] méthylamino]méthyl]phényl]-4,5-dihydro-1*H*-imidazole-1-carboxylique,1,1-diméthyléthyl ester**

(pâte incolore, rendement = 79%)
RMN $^1$H (300 MHz, DMSO) δ : 8,60 (t, 1H) ; 8,26 (d, 1H) ; 8,13 (m, 2H) ; 7,70 (m, 3H) ; 7,42 (d, 2H) ; 7,16 (m, 2H) ; 6,55 (m, 2H) ; 4,54 (s, 2H) ; 4,00 (m, 2H) ; 3,84 (m, 4H) ; 2,70 (m, 6H) ; 1,16 (d, 9H).
En opérant de façon analogue à l'exemple 1, on obtient les composés suivants :

**Exemple 5**

**5-[[(2-chlorophényl)sulfonyl]méthylamino]-N-[[4-(4,5-dihydro-1*H*-imidazol-2-yl) phényl]méthyl]-N-méthyl-pentanamide, trifluoroacétate**

(solide blanc, rendement = 98%)
F = 60°C

**Exemple 6**

**(2E)-4-[[(2-chlorophényl)sulfonyl]méthylamino]-N-[[4-(4,5-dihydro-1*H*-imidazol-2-yl)phényl]méthyl]-N-méthyl-2-butènamide, trifluoroacétate**

(solide blanc, rendement = 90%)
F = 72°C

**Exemple 7**

**3-[[(2,3-dichlorophényl)sulfonyl]méthylamino]-N-[[4-(4,5-dihydro-1*H*-imidazol-2-yl)phényl]méthyl]-N-méthyl-propanamide, trifluoroacétate**

(solide blanc, rendement = 84%)
F = 60°C

**Exemple 8**

**4-[[(2,3-dichlorophényl)sulfonyl]méthylamino]-N-[[4-(4,5-dihydro-1*H*-imidazol-2-yl)phényl]méthyl]-N-méthyl-butanamide, trifluoroacétate**

(solide blanc, rendement = 9 1 %)
F = 62°C

**Exemple 9**

**5-[[(2,3-dichlorophényl)sulfonyl]méthylamino]-N-[[4-(4,5-dihydro-1*H*-imidazol-2-yl)phényl]méthyl]-N-méthyl-pentanamide, trifluoroacétate**

(solide blanc, rendement = 80%)
F = 64°C

**Exemple 10**

**(2E)-4-[[(2,3-dichlorophényl)sulfonyl]méthylamino]-N-[[4-(4,5-dihydro-1*H*-imidazol-2-yl)phényl]méthyl]-N-méthyl-2-butènamide, trifluoroacétate**

(solide blanc, rendement = 94%)
F = 66°C

**Exemple 11**

**5-[[(2,6-dichlorophényl)sulfonyl]méthylamino]-N-[[4-(4,5-dihydro-1*H*-imidazol-2-yl)phényl]méthyl]-N-méthyl-pentanamide, trifluoroacétate**

(solide écru, rendement = 65%)
F = 50°C

**Exemple 12**

**(2E)-4-[[(2,6-dichlorophényl)sulfonyl]méthylamino]-N-[[4-(4,5-dihydro-1*H*-imidazol-2-yl)phényl]méthyl]-N-méthyl-2-butènamide, trifluoroacétate**

(solide beige, rendement = 98%)
F = 83°C

## Exemple 13

**5-[méthyl[(1-naphtalényl)sulfonyl]amino]-N-[[4-(4,5-dihydro-1*H*-imidazol-2-yl)phényl]méthyl]-N-méthyl-pentanamide, trifluoroacétate**

(solide blanc, rendement = 97%)
RMN $^1$H (250 MHz, DMSO) δ : 10,5 (d, 2H) ; 8,60 (t, 1H) ; 8,26 (d, 1H) ; 8,09 (m, 2H) ; 7,90 (m, 2H) ; 7,70 (m, 3H) ; 7,45 (d, 2H) ; 4,60 (d, 2H) ; 4,01 (s, 4H) ; 3,20 (m, 2H) ; 2,85 (m, 6H) ; 2,40 (m, 2H) ; 1,52 (m, 4H).

## Exemple 14

**(2E)-4-[méthyl[(1-naphtalényl)sulfonyl]amino]-N-[[4-(4,5-dihydro-1*H*-imidazol-2-yl)phényl]méthyl]-N-méthyl-2-butènamide, trifluoroacétate**

(solide beige, rendement = 99%)
F = 77°C

En opérant de façon analogue à la préparation V, on obtient les composés suivants :

## PREPARATION LII

**2-chloro-N-cyclopropyl-benzènesulfonamide**

(solide blanc, rendement = 82%)
F = 117°C

## PREPARATION LIII

**N-cyclopropyl-2,3-dichloro-benzènesulfonamide**

(solide écru, rendement = 50%)
F = 140°C

## PREPARATION LIV

**N-cyclopropyl-2,6-dichloro-benzènesulfonamide**

(solide blanc, rendement = 99%)
F = 76°C

## PREPARATION LV

**2,3-dichloro-N-(1-méthyléthyl)-benzènesulfonamide**

(solide blanc, rendement = 93%)
F=131°C

## PREPARATION LVI

**2,6-dichloro-N-(1-méthyléthyl)-benzènesulfonamide**

(solide blanc, rendement = 99%)
F = 105°C

## PREPARATION LVII

**N-(2-amino-2-oxoéthyl)-2,4-dichloro-3-méthylbenzènesulfonamide**

(huile, rendement = 95%)

RMN $^1H$ (300 MHz, DMSO) δ : 8,04 (s, 1H) ; 7,82 (d, 1H) ; 7,62 (d, 1H) ; 7,12 (s, 1H) ; 7,03 (s, 1H) ; 3,50 (s, 2H) ; 2,48 (s, 3H).

**PREPARATION LVIII**

**N-(3-amino-3-oxopropyl)-2,4-dichloro-3-méthylbenzènesulfonamide**

(solide blanc, rendement = 81%)
F = 163°C

**PREPARATION LIX**

**2,3-dichloro-N-(2,2,2-trifluoroéthyl)-benzènesulfonamide**

(solide écru, rendement = 76%)
F = 107°C

**PREPARATION LX**

**2,6-dichloro-N-(2,2,2-trifluoroéthyl)-benzènesulfonamide**

(solide blanc, rendement = 99%)
F = 106°C

**PREPARATION LXI**

**2,4-dichloro-3-méthyl-N-(2-phényléthyl)-benzènesulfonamide**

(solide blanc, rendement = 81%)
F = 75°C

En opérant de façon analogue à la préparation VI, on obtient les composés suivants :

**PREPARATION LXII**

**Acide 5-[[(2-chlorophényl)sulfonyl]cyclopropylamino]pentanoïque, éthyl ester**

(solide blanc, rendement = 82%)
F = 117°C

**PREPARATION LXIII**

**Acide 5-[cyclopropyl[(2,3-dichlorophényl)sulfonyl]amino]pentanoïque, éthyl ester**

(huile jaune, rendement = 98%)
RMN $^1H$ (250 MHz, $DMSO_3$) δ : 8,00 (m, 2H) ; 7,58 (t, 1H) ; 4,05 (q, 2H) ; 3,42 (t, 2H) ; 2,50 (m, 1H) ; 2,33 (t, 2H) ; 1,59 (m, 4H) ; 1,18 (t, 3H) ; 0,60 (m, 2H) ; 0,40 (m, 2H).

**PREPARATION LXIV**

**Acide 5-[cyclopropyl[(2,6-dichlorophényl)sulfonyl]amino]pentanoïque, éthyl ester**

(non isolé)

**PREPARATION LXV**

**Acide 5-[[(2,6-dichlorophényl)sulfonyl](1-méthyléthyl)amino]pentanoïque, éthyl ester**

(non isolé)

**PREPARATION LXVI**

**Acide 5-[(2-amino-2-oxoéthyl)[(2,4-dichloro-3-méthylphényl)sulfonyl] amino]pentanoïque, éthyl ester**

(huile jaune, rendement = 95%)
RMN $^1$H (300 MHz, DMSO) δ : 7,91 (d, 1H) ; 7,64 (d, 1H) ; 7,15 (s, 1H) ; 7,01 (s, 1H) ; 4,04 (m, 2H) ; 3,95 (s, 2H) ; 3,27 (t, 2H) ; 2,49 (s, 3H) ; 2,15 (t, 2H) ; 1,62 (m, 1H) ; 1,20 (m, 3H) ; 1,17 (t, 3H).

**PREPARATION LXVII**

**Acide 5-[(3-amino-3-oxopropyl)[(2,4-dichlorophényl)sulfonyl]amino]pentanoïque, éthyl ester**

(huile incolore, rendement = 99%)
RMN $^1$H (300 MHz, DMSO) δ : 7,84 (d, 1H) ; 7,65 (d, 1H) ; 7,34 (s, 1H) ; 6,84 (s, 1H) ; 4,04 (m, 2H) ; 3,51 (m, 2H) ; 3,25 (m, 2H) ; 2,50 (s, 3H) ; 2,30 (m, 2H) ; 2,22 (t, 2H) ; 1,44 (m, 4H) ; 1,17 (t, 3H).

En opérant de façon analogue à la préparation VI mais en remplaçant l'ester éthylique bromé par un ester *t*-butylique iodé, on obtient les composés suivants :

**PREPARATION LXVIII**

**Acide [2-[[(2-chlorophényl)sulfonyl]cyclopropylamino]éthoxy]acétique, 1,1-diméthyléthyl ester**

(huile jaune, rendement = 52%)
RMN $^1$H (250 MHz, DMSO) δ : 8,00 (d, 1H) ; 7,68 (d, 2H) ; 7,57 (m, 1H) ; 3,98 (s, 2H) ; 3,69 (t, 2H) ; 3,52 (t, 2H) ; 2,50 (m, 1H) ; 1,43 (s, 9H) ; 0,56 (m, 2H) ; 0,45 (m, 2H).

**PREPARATION LXIX**

**Acide [2-[cyclopropyl[(2,3-dichlorophényl)sulfonyl]amino]éthoxy]acétique, 1,1-diméthyléthyl ester**

(huile incolore, rendement = 78%)
RMN $^1$H (250 MHz, DMSO) δ : 8,00 (m, 2H) ; 7,60 (t, 1H) ; 4,08 (s, 2H) ; 3,69 (t, 2H) ; 3,56 (t, 2H) ; 2,56 (m, 1H) ; 1,42 (s, 9H) ; 0,60 (m, 2H) ; 0,50 (m, 2H).

**PREPARATION LXX**

**Acide [2-[cyclopropyl[(2,6-dichlorophényl)sulfonyl]amino]éthoxy]acétique, 1,1-diméthyléthyl ester**

(solide écru, rendement = 40%)
F = 76°C

**PREPARATION LXXI**

**Acide [2-[[(2,4-dichloro-3-méthylphényl)sulfonyl](2-phényléthyl)amino]éthoxy]acétique, 1,1-diméthyléthyl ester**

(huile incolore, rendement = 77%)
RMN $^1$H (250 MHz, DMSO) δ : 7,86 (m, 1H) ; 7,60 (m, 1H) ; 7,17 (m, 6H) ; 3,94 (s, 2H) ; 3,56 (m, 6H) ; 2,80 (m, 2H) ; 2,44 (d, 3H) ; 1,42 (s, 9H).

En opérant de façon analogue à la préparation VIII, on obtient les composés suivants :

**PREPARATION LXXII**

**Acide 5-[[(2-chlorophényl)sulfonyl]cyclopropylamino]pentanoïque**

(solide blanc, rendement = 86%)
F = 104°C

**PREPARATION LXXIII**

**Acide 5-[cyclopropyl[(2,3-dichlorophényl)sulfonyl]amino]pentanoïque**

(solide écru, rendement = 79%)
F = 115°C

**PREPARATION LXXIV**

**Acide 5-[cyclopropyl[(2,6-dichlorophényl)sulfonyl]amino]pentanoïque**

(solide jaune, rendement = 53%)
F = 146°C

**PREPARATION LXXV**

**Acide 5-[[(2,6-dichlorophényl)sulfonyl](1-méthyléthyl)amino]pentanoïque**

(huile, rendement = 36%)
RMN $^1H$ (300 MHz, DMSO) δ : 12,0 (s large, 1H) ; 7,67 (d, 2H) ; 7,56 (t, 1H) ; 3,98 (m, 1H) ; 3,97 (t, 2H) ; 2,17 (t, 2H) ; 1,48 (m, 4H) ; 1,06 (d, 6H).

**PREPARATION LXXVI**

**Acide 5-[(2-amino-2-oxoéthyl)[(2,4-dichloro-3-méthylphényl) sulfonyl]amino]pentanoïque**

(solide blanc, rendement = 63%)
F = 160°C

**PREPARATION LXXVII**

**Acide 5-[(3-amino-3-oxopropyl)[(2,4-dichlorophényl)sulfonyl]amino]pentanoïque**

(solide blanc, rendement = 93%)
F = 161°C

**PREPARATION LXXVIII**

**Acide [2-[[(2-chlorophényl)sulfonyl]cyclopropylamino]éthoxylacétique**

On prépare une solution de 210 mg (0,5 mM) de l'ester obtenu selon la préparation LXVIII dans 10 ml de dichlorométhane, on ajoute 3 ml d'acide trifluoroacétique et on maintient ce mélange réactionnel sous agitation pendant 16 heures, à température ambiante. Le milieu réactionnel est ensuite concentré sous pression réduite, repris par 30 ml de toluène et à nouveau concentré sous pression réduite. Le résidu cristallise pendant le séchage. On obtient ainsi le produit attendu sous forme d'un solide blanc (rendement = 99%).
F= 115°C

En opérant de façon analogue à la préparation LXXVIII, on obtient les composés suivants :

**PREPARATION LXXIX**

**Acide [2-[cyclopropyl[(2,3-dichlorophényl)sulfonyl]amino]éthoxy]acétique**

(solide jaune, rendement = 99%)
F = 96°C

**PREPARATION LXXX**

**Acide [2-[cyclopropyl[(2,6-dichlorophényl)sulfonyl]amino]éthoxy]acétique**

(solide blanc, rendement = 88%)
F = 98°C

**PREPARATION LXXXI**

**Acide [2-[[(2,4-dichloro-3-méthylphényl)sulfonyl](2-phényléthyl)amino]éthoxy]acétique**

(huile jaune, rendement = 77%)
RMN $^1H$ (300 MHz, DMSO) δ : 12,0 (s large, 1H) ; 7,87 (d, 1H) ; 7,60 (d, 1H) ; 7,15 (m, 5H) ; 3,91 (s, 2H) ; 3,56 (m, 6H) ; 2,81 (t, 2H) ; 2,43 (s, 3H).

**PREPARATION LXXXII**

**N-[(2,6-dichlorophényl)sulfonyl]-N-méthyl-β-alanine**

On prépare une solution de 480 mg (2 mM) du composé obtenu selon la préparation XX dans 7 ml de diméthylformamide et on ajoute 120 mg (4 mM) d'hydrure de sodium à 80% dans l'huile. Le mélange est agité 2 min à température ambiante puis on ajoute 306 mg (2 mM) d'acide 3-bromopropanoïque. Le milieu réactionnel est maintenu sous agitation pendant 1 heure à température ambiante, puis 20 heures à 70°C. Après refroidissement, le mélange est hydrolysé sur 50 ml d'eau glacée. Cette phase aqueuse est extraite à l'acétate d'éthyle, puis acidifiée jusqu'à pH 2 à l'aide d'acide chlorhydrique N et extraite à l'acétate d'éthyle. Cette dernière phase organique est lavée à l'eau puis séchée sur sulfate de sodium, puis concentrée sous pression réduite. On obtient ainsi l'acide attendu sous forme d'un solide blanc amorphe (rendement = 70%).
F = 115°C

En opérant de façon analogue à la préparation LXXXII, on obtient les composés suivants :

**PREPARATION LXXXIII**

**N-[(2,3-dichlorophényl)sulfonyl]-N-méthyl-β-alanine**

(solide blanc, rendement = 70%)
F = 115°C

**PREPARATION LXXXIV**

**N-cyclopropyl-N-[(2,6-dichlorophényl)sulfonyl]-β-alanine**

(solide écru, rendement = 55%)
F = 190°C

**PREPARATION LXXXV**

**N-[(2,3-dichlorophényl)sulfonyl]-N-(1-méthyléthyl)-β-alanine**

(solide écru, rendement = 47%)
F = 115°C

**PREPARATION LXXXVI**

**N-[(2,6-dichlorophényl)sulfonyl]-N-(1-méthyléthyl)-β-alanine**

(solide blanc, rendement = 53%)
F = 138°C

**PREPARATION LXXXVII**

**N-[(2,3-dichlorophényl)sulfonyl]-N-(2,2,2-trifluoroéthyl)-β-alanine**

(solide écru, rendement = 92%)
F = 125°C

**PREPARATION LXXXVIII**

**N-[(2,6-dichlorophényl)sulfonyl]-N-(2,2,2-trifluoroéthyl)-β-alanine**

(solide blanc, rendement = 77%)
F = 100°C

En opérant de façon analogue à la préparation V, on obtient le composé suivant :

**PREPARATION LXXXIX**

**N-méthyl-2-(trifluorométhyl)benzènesulfonamide**

(solide blanc, rendement = 99%)
F = 107°C

**PREPARATION XC**

**2,4-dichloro-3-méthylbenzènesulfonamide**

On prépare une solution de 5g (16,8 mM) de 2,4-dichloro-N-(1,1-diméthyléthyl)-3-méthylbenzènesulfonamide dans 100 ml de dichlorométhane et on ajoute 50 ml d'acide trifluoroacétique. Le mélange réactionnel est agité à température ambiante pendant 20 heures. On ajoute ensuite 20 ml d'acide chlorhydrique 10 N et on maintient sous agitation pendant 4 heures à température ambiante. Le mélange est ensuite concentré sous pression réduite. Le résidu solide est purifié par chromatographie sur gel de silice en éluant au moyen d'un mélange toluène-acétate d'éthyle (9/1 ; v/v). On obtient ainsi 3,74 g du produit attendu (rendement = 92%).
F = 210°C

En opérant de façon analogue à la préparation VI, on obtient les composés suivants :

**PREPARATION XCI**

**Acide (2E)-4-[méthyl[[2-(trifluorométhyl)phényl]sulfonyl]amino]-2-butènoïque, 1,1-diméthyléthyl ester**

(huile jaune, rendement = 44%)
RMN $^1H$ (250 MHz, DMSO) δ : 8,04 (m, 2H) ; 7,90 (m, 2H) ; 6,66 (dt, 1H) ; 5,88 (dt, 1H) ; 4,07 (dd, 2H) ; 2,84 (s, 3H) ; 1,44 (s, 9H).

**PREPARATION XCII**

**Acide (2E)-4-[[(2,4-dichloro-3-méthylphényl)sulfonyl]amino]-2-butènoïque, 1,1-diméthyléthyl ester**

(huile jaune, rendement = 20%)
RMN$^1H$ (300 MHz , DMSO) δ : 8,33 (s, 1H) ; 7,81 (d, 1H) ; 7,63 (d, 1H) ; 6,49 (dt, 1H) ; 5,69 (dt, 1H) ; 3,70 (dd, 2H) ; 2,48 (s, 3H) ; 1,38 (s, 9H).

En opérant de façon analogue à la préparation LXXIII, on obtient les composés suivants :

**PREPARATION XCIII**

**Acide (2E)-4-[[(2,4-dichloro-3-méthylphényl)sulfonyl]amino]-2-butènoïque**

(solide blanc, rendement = 99%)
F = 154°C

**PREPARATION XCIV**

**Acide 4-[méthyl[[2-(trifluorométhyl)phényl]sulfonyl]amino]-2-butènoïque**

(solide blanc, rendement = 99%)
F = 184°C

En opérant de façon analogue à la préparation VIII, on obtient les composés suivants :

**PREPARATION XCV**

**Acide 2-[4-[[[5-[[(2-chlorophényl)sulfonyl]cyclopropylamino]-1-oxopentyl] méthylamino]méthyl]phényl]-4,5-dihydro-1*H*-imidazole-1-carboxylique, 1,1-diméthyléthyl ester**

(solide amorphe, rendement = 84%)
F = 50°C

**PREPARATION XCVI**

**Acide 2-[4-[[[5-[cyclopropyl[(2,3-dichlorophényl)sulfonyl]amino]-1-oxopentyl] méthylamino]méthyl]phényl]-4,5-dihydro-1*H*-imidazole-1-carboxylique, 1,1-diméthyléthyl ester**

(huile incolore; rendement = 65%)
RMN $^1$H (300 MHz, DMSO) δ : 7,97 (m, 2H) ; 7,40 (m, 1H) ; 7,25 (m, 2H) ; 7,18 (m, 2H) ; 4,55 (d, 2H) ; 3,84 (m, 4H) ; 3,40 (m, 2H) ; 2,85 (d, 3H) ; 2,46 (m, 3H) ; 1,60 (m, 4H) ; 1,17 (s, 9H) ; 0,59 (m, 2H) ; 0,42 (m, 2H).

**PREPARATION XCVII**

**Acide 2-[4-[[[S-[cyclopropyl[(2,6-dichlorophényl)sulfonyl]amino]-1-oxopentyl] méthylamino]méthyl]phényl]-4,5-dihydro-1*H*-imidazole-1-carboxylique, 1,1-diméthyléthyl ester**

(solide amorphe, rendement = 59%)
F = 50°C

**PREPARATION XCVIII**

**Acide 2-[4-[[[5-[[(2,6-dichlorophényl)sulfonyl](1-méthyléthyl)amino]-1-oxopentyl] méthylamino]méthyl]phényl]-4,5-dibydro-1*H*-imidazole-1-carboxylique, 1,1-diméthyléthyl ester**

(solide blanc, rendement = 69%)
F = 50°C

**PREPARATION IC**

**Acide 2-[4-[[[5-[(2-amino-2-oxoéthyl)[(2,4-dichloro-3-méthylphényl)sulfonyl]amino]-1-oxopentyl]méthylamino]méthyl]phényl]-4,5-dihydro-1*H*-imidazole-1-carboxylique, 1,1-diméthyléthyl ester**

(solide blanc, rendement = 69%)
F = 70°C

**PREPARATION C**

**Acide 2-[4-[[[5-[(3-amino-3-oxopropyl)[(2,4-dichloro-3-méthylphényl)sulfonyl]amino]-1-oxopentyl]méthylamino]méthyl]phényl]-4,5-dihydro-1*H*-imidazole-1-carboxylique, 1,1-diméthyléthyl ester**

(solide blanc, rendement = 76%)
F = 80°C

**PREPARATION CI**

**Acide 2-[4-[[[3-[[(2,3-dichlorophényl)sulfonyl](1-méthyléthyl)amino]-1-oxopropyl]méthylamino]méthyl]phényl]-4,5-dihydro-1*H*-imidazole-1-carboxylique, 1,1-diméthyléthyl ester**

(solide blanc, rendement = 55%)
F = 60°C

**PREPARATION CII**

**Acide 2-[4-[[[3-[[(2,6-dichlorophényl)sulfonyl](1-méthyléthyl)amino]-1-oxopropyl] méthylamino]méthyl]phényl]-4,5-dihydro-1*H*-imidazole-1-carboxylique, 1,1-diméthyléthyl ester**

(solide blanc, rendement = 54%)
F=55°C

**PREPARATION CIII**

**Acide 2-[4-[[[[2-[[(2-chlorophényl)sulfonyl]cyclopropylamino]éthoxylacétyl] méthylamino]méthyl]phényl]-4,5-dihydro-1*H*-imidazole-1-carboxylique, 1,1-diméthyléthyl ester**

(solide amorphe, rendement = 96%)
F = 50°C

**PREPARATION CIV**

**Acide 2-[4-[[[[2-[cyclopropyl[(2,3-dichlorophényl)sulfonyl]amino]éthoxy]acétyl] méthylamino]méthyl]phényl]-4,5-dihydro-1*H*-imidazole-1-carboxylique, 1,1-diméthyléthyl ester**

(solide blanc, rendement = 56%)
F = 62°C

**PREPARATION CV**

**Acide 2-[4-[[[[2-[cyclopropyl[(2,6-dichlorophényl)sulfonyl]amino]éthoxy]acétyl] méthylamino]méthyl]phényl]-4,5-dihydro-1*H*-imidazole-1-carboxylique, 1,1-diméthyléthyl ester**

(solide amorphe, rendement = 64%)
F = 55°C

**PREPARATION CVI**

**Acide 2-[4-[[[[2-[[(2,4-dichloro-3-méthylphényl)sulfonyl](2-phényléthyl)amino] éthoxy]acétyl]méthylamino] méthyl]phényl]-4,5-dihydro-1*H*-imidazole-1-carboxylique, 1,1-diméthyléthyl ester**

(huile incolore, rendement = 63%)
RMN $^{1}$H (250 MHz, DMSO) δ : 7,87 (m, 1H) ; 7,60 (m, 1H) ; 7,30 (m, 3H) ; 7,20 (m, 6H) ; 4,52 (s, 2H) ; 4,19 (d, 2H) ; 3,84 (m, 4H) ; 3,60 (m, 6H) ; 2,83 (s, 3H) ; 2,77 (t, 2H) ; 2,48 (d, 3H) ; 1,17 (s, 9H).

**PREPARATION CVII**

**Acide 2-[4-[[[3-[[(2,6-dichlorophényl)sulfonyl]méthylamino]-1-oxopropyl] méthylamino]méthyl]phényl]-4,5-dibydro-1*H*-imidazole-1-carboxylique,1,1-diméthyléthyl ester**

(solide blanc, rendement = 60%)
F = 55°C

**PREPARATION CVIII**

**Acide 2-[4-[[[3-[cyclopropyl[(2,3-dichlorophényl)sulfonyl]amino]-1-oxopropyl] méthylamino]méthyl]phényl]-4,5-dihydro-1*H*-imidazole-1-carboxylique,1,1-diméthyléthyl ester**

(solide blanc, rendement = 64%)
F = 60˚C

**PREPARATION CIX**

**Acide 2-[4-[[[3-[cyclopropyl[(2,6-dichlorophényl)sulfonyl]amino]-1-oxopropyl] méthylanlino]méthyl]phényl]-4,5-dihydro-1*H*-imidazole-1-carboxylique,1,1-diméthyléthyl ester**

(solide blanc, rendement = 63%)
F = 64˚C

**PREPARATION CX**

**Acide 2-[4-[[[3-([(2,3-dichlorophényl)sulfonyl](2,2,2-trifluoroéthyl)amino]-1-oxopropyl] méthylamino]méthyl] phényl]-4,5-dihydro-1*H*-imidazole-1-carboxylique, 1,1-diméthyléthyl ester**

(solide blanc, rendement = 33%)
F = 55˚C

**PREPARATION CXI**

**Acide 2-[4-[[[3-[[(2,6-dichlorophényl)sulfonyl](2,2,2-trifluoroéthyl)amino]-1-oxopropyl]méthylamino]méthyl] phényl]-4,5-dihydro-1*H*-imidazole-1-carboxylique, 1,1-diméthyléthyl ester**

(solide blanc, rendement = 36%)
F = 50˚C

**PREPARATION CXII**

**Acide 4,5-dihydro-2-[4-[[méthyl[(2E)-4-[méthyl[[2-(trifluorométhyl)phényl] sulfonyl]amino]-1-oxo-2-butènyl] amino]méthyl]phényl]-1*H*-imidazole-1-carboxylique, 1,1-diméthyléthyl ester**

(huile incolore, rendement = 62%)
RMN$^1$H (250 MHz, DMSO) δ: 8,04 (m, 2H) ; 7,88 (m, 2H) ; 7,43 (m, 2H) ; 7,17 (m, 2H) ; 6,60 (m, 2H) ; 4,62 (d, 2H) ; 4,05 (dd, 2H) ; 3,83 (m, 4H) ; 2,86 (m, 6H) ; 1,17 (m, 9H).

**PREPARATION CXIII**

**Acide 2-[4-[[[(2E)-4-[[(2,4-dichloro-3-méthylphényl)sulfonyl]amino]-1-oxo-2-butènyl]méthylamino]méthyl] phényl]-4,5-dihydro-1*H*-imidazole-1-carboxylique,1,1-diméthyléthyl ester**

(huile incolore, rendement = 50%)
RMN $^1$H (300 MHz, DMSO) δ: 8,31 (s, 1H) ; 7,82 (t, 1H) ; 7,62 (dd, 1H) ; 7,44 (dd, 2H) ; 7,22 (dd, 2H) ; 6,50 (m, 2H) ; 4,55 (d, 2H) ; 3,84 (m, 4H) ; 3,70 (m, 2H) ; 2,82 (d, 3H) ; 2,47 (m, 3H) ; 1,18 (s, 9H).

En opérant de façon analogue à l'exemple 1, on obtient les composés suivants :

**Exemple 15**

**5-[[(2-chlorophényl)sulfonyl]cyclopropylamino]-N-[[4-(4,5-dihydro-1*H*-imidazol-2-yl)phényl]méthyl]-N-méthyl-pentanamide, trifluoroacétate**

(solide blanc, rendement = 84%)
F = 55˚C

### Exemple 16

**5-[cyclopropyl[(2,3-dichlorophényl)sulfonyl]amino]-N-[[4-(4,5-dihydro-1*H*-imidazol-2-yl)phényl]méthyl]-N-méthyl-pentanamide, trifluoroacétate**

(solide écru, rendement = 99%)
F = 70˚C

### Exemple 17

**5-[cyclopropyl[(2,6-dichlorophényl)sulfonyl]amino]-N-[[4-(4,5-dihydro-1*H*-imidazol-2-yl)phényl]méthyl]-N-méthyl-pentanamide, trifluoroacétate**

(solide blanc, rendement = 86%)
F = 60˚C

### Exemple 18

**5-[[(2,6-dichlorophényl)sulfonyl](1-méthyléthyl)amino]-N,[[4-(4,5-dihydro,1*H*-imidazol-2-yl)phényl]méthyl]-N-méthyl-pentanamide, trifluoroacétate**

(solide blanc, rendement = 98%)
F = 60˚C

### Exemple 19

**5-[(2-amino-2-oxoéthyl)[(2,4-dichloro-3-méthylphényl)sulfonyl]amino]-N-[[4-(**4,5-**dihydro-1*H*-imidazol-2-yl)phényl]méthyl]-N-méthyl-pentanamide, trifluoroacétate**
(solide blanc, rendement = 99%)
F = 90˚C

### Exemple 20

**5-[(3-amino-3-oxopropyl)[(2,4-dichloro-3-méthylphényl)sulfonyl]amino]-N-[[4-(4,5-dihydro-1*H*-imidazol-2-yl)phényl]méthyl]-N-méthyl-pentanamide, trifluoroacétate**

(solide blanc, rendement = 95%)
F = 85˚C

### Exemple 21

**3-[[(2,3-dichlorophényl)sulfonyl](1-méthyléthyl)amino]-N-[[4-(4,5-dihydro-1*H*-imidazol-2-yl)phényl]méthyl]-N-méthyl-propanamide, trifluoroacétate**

(solide blanc, rendement = 73%)
F = 92˚C

### Exemple 22

**3-[[(2,6-dichlorophényl)sulfonyl](1-méthyléthyl)amino]-N-[[4-(4,5-dihydro-1*H*-imidazol-2-yl)phényl]méthyl]-N-méthyl-propanamide, trifluoroacétate**

(solide blanc, rendement = 73%)
F =75˚C

**Exemple 23**

**2-[2-[[(2-chlorophényl)sulfonyl]cyclopropylamino]éthoxy]-N-[[4-(4,5-dihydro-1*H*-imidazol-2-yl)phényl]méthyl]-N-méthyl-acétamide, trifluoroacétate**

(solide blanc, rendement = 71%)
F = 52°C

**Exemple 24**

**2-[2-[cyclopropyl[(2,3-dichlorophényl)sulfonyl]amino]éthoxy]-N-[[4-(4,5-dihydro-1*H*-imidazol-2-yl)phényl]méthyl]-N-méthyl-acétamide, trifluoroacétate**

(solide blanc, rendement = 97%)
F = 65°C

**Exemple 25**

**2-[2-[cyclopropyl[(2,6-dichlorophényl)sulfonyl]amino]éthoxy]-N-[[4-(4,5-dihydro-1*H*-imidazol-2-yl)phényl]méthyl]-N-méthyl-acétamide, trifluoroacétate**

(solide blanc, rendement = 99%)
F = 60°C

**Exemple 26**

**2-[2-[[(2,4-dichloro-3-méthylphényl)sulfonyl](2-phényléthyl)amino]éthoxy]-N-[[4-(4,5-dihydro-1*H*-imidazol-2-yl)phényl]méthyl]-N-méthyl-acétamide, trifluoroacétate**

(solide blanc, rendement = 81%)
F = 85°C

**Exemple 27**

**3-[[(2,6-dichlorophényl)sulfonyl]méthylamino]-N-[[4-(4,5-dihydro-1*H*-imidazol-2-yl)phényl]méthyl]-N-méthyl-propanamide, trifluoroacétate**

(solide blanc, rendement = 73%)
F = 75°C

**Exemple 28**

**3-[cyclopropyl[(2,3-dichlorophényl)sulfonyl]amino]-N-[[4-(4,5-dihydro-1*H*-imidazol-2-yl)phényl]méthyl]-N-méthyl-propanamide, trifluoroacétate**

(solide blanc, rendement = 86%)
F = 80°C

**Exemple 29**

**3-[cyclopropyl[(2,6-dichlorophényl)sulfonyl]amino]-N-[[4-(4,5-dihydro-1*H*-imidazol-2-yl)phényl]méthyl]-N-méthyl-propanamide, trifluoroacétate**

(solide blanc, rendement = 84%)
F = 65°C

## Exemple 30

**3-[(2,2,2-trifluoroéthyl)[(2,3-dichlorophényl)sulfonyl]amino]-N-[[4-(4,5-dihydro-1*H*-imidazol-2-yl)phényl]méthyl]-N-méthyl-propanamide, trifluoroacétate**

(solide blanc, rendement = 90%)
F = 88°C

## Exemple 31

**3-[(2,2,2-trifluoroéthyl)[(2,6-dichlorophényl)sulfonyl]amino]-N-[[4-(4,5-dihydro-1*H*-imidazol-2-yl)phényl]méthyl]-N-méthyl-propanamide, trifluoroacétate**

(solide blanc, rendement = 80%)
F = 80°C

## Exemple 32

**N-[[4-(4,5-dihydro-1*H*-imidazol-2yl)phényl]méthyl]-N-méthyl-4-[méthyl[[2, (trifluorométhyl)phényl]sulfonyl] amino]-(2E)-2-butènamide, trifluoroacétate**

(solide blanc, rendement = 99%)
F = 50°C

## Exemple 33

**4-[[(2,4-dichloro-3-méthylphényl)sulfonyl]amino]-N-[[4-(4,5-dihydro-1*H*-imidazol-2-yl)phényl]méthyl]-N-méthyl-(2E)-2-butènamide, trifluoroacétate**

(solide blanc, rendement = 62%).
F = 99°C

## PREPARATION CXIV

**Acide [(4-cyanophényl)méthyl]méthylcarbamique, 1,1-diméthyléthyl ester**

On prépare une solution de 1,94 g (13 mM) de [(4-cyanophényl)méthyl]-méthanamine dans 100 ml de dichlorométhane et on ajoute 1,78 g (14,6 mM) de 4-diméthylaminopyridine, puis 3,19 g (14,6 mM) de dicarbonate de di-t-butyle. Le mélange réactionnel est agité à température ambiante pendant 2 heures. La phase organique est lavée à l'eau puis avec une solution d'acide citrique, puis à l'eau, puis séchée sur sulfate de magnésium et concentrée sous pression réduite. Le produit brut est purifié par chromatographie sur gel de silice en éluant avec un mélange toluène/isopropanol (9/1, v/v). On obtient ainsi 2,91 g du produit attendu sous forme d'une huile incolore (rendement = 82%).
RMN $^1$H (250 MHz, DMSO) $\delta$: 7,82 (d, 2H) ; 7,39 (d, 2H) ; 4,45 (s, 2H) ; 2,79 (s, 3H) ; 1,38 (s, 9H).

## PREPARATION CXV

**Acide [[4-(4,5-dihydro-5,5-diméthyl-1*H*-imidazol-2-yl)phényl]méthyl] méthylcarbamique, 1,1-diméthyléthyl ester**

En opérant de façon analogue à la préparation II, au départ du composé obtenu selon la préparation CXIV et de 1,2-diamino-2-méthylpropane, on obtient le produit attendu sous forme d'huile incolore avec un rendement de 82%.
RMN$^1$H (300 MHz, DMSO) $\delta$ : 7,78 (d, 2H) ; 7,25 (d, 2H) ; 4,39 (s, 2H) ; 3,36 (s, 2H) ; 2,76 (s, 3H) ; 1,39 (s, 9H) ; 1,23 (s, 6H).

## PREPARATION CXVI

**4-(4,5-dihydro-5,5-diméthyl-1*H*-imidazol-2-yl)-N-méthyl-benzèneméthanamine, dichlorhydrate**

On prépare une solution de 1,585 g (5 mM) du composé obtenu selon la préparation CXV dans 80 ml d'acétate d'éthyle et on ajoute lentement 24 ml d'une solution de chlorure d'hydrogène dans l'acétate d'éthyle. Le mélange est agité à température ambiante pendant 20 heures. Le précipité est séparé par filtration puis lavé avec de l'acétate d'éthyle puis de l'éther éthylique, puis séché en milieu anhydre. On obtient ainsi 1,33 g du produit attendu (rendement = 92%).
RMN $^1$H (250 MHz, DMSO) δ : 7,73 (d, 2H) ; 7,33 (d, 2H) ; 3,65 (s, 2H) ; 3,35 (s, 2H) ; 2,24 (s, 3H) ; 1,22 (s, 6H).

En opérant de façon analogue à la préparation VIII, au départ du composé obtenu selon la préparation CXVI, on obtient le composé suivant:

## Exemple 34

**5-[[(2,4-dichloro-3-méthylphényl)sulfonyl]méthylamino]-N-[[4-(5,5-diméthyl-1,5-dihydro-1*H*-imidazol-2-yl) phényl]méthyl]-N-méthyl-pentanamide**

(solide blanc, rendement = 38%)
F = 104°C

## Exemple 35

**5-[[(2,4-dichloro-3-méthylphényl)sulfonyl]méthylamino]-N-[[4-(5,5-diméthyl-4,5-dihydro-1*H*-imidazol-2-yl) phényl]méthyl]-N-méthyl-pentanamide, chlorhydrate**

On prépare une solution de 62,6 mg (0,113 mM) du composé obtenu selon l'exemple 34 dans 10 ml de méthanol et on ajoute 225 µl d'une solution d'acide chlorhydrique N. On mélange sous agitation pendant 5 min puis on concentre sous pression réduite. Le résidu est repris dans 5 ml d'eau pure et lyophilisé. On obtient ainsi 48 mg du composé attendu sous forme d'un solide blanc.
(rendement = 72%)
F = 104°C

En opérant de façon analogue à la préparation V, on obtient les composés suivants :

## PREPARATION CXVII

**2,4-dichloro-3,N-diméthyl-N-(2-hydroxyéthyl)benzènesulfonamide**

(non isolé)

## PREPARATION CXVIII

**2,3-dichloro-N-(2-hydroxy-1-méthyléthyl)-N-méthylbenzènesulfonamide**

(huile incolore, rendement = 71%)
RMN$^1$H (300 MHz, DMSO) δ : 8,05 (d, 1H) ; 8,01 (d, 1H) ; 7,55 (t, 1H) ; 4,76 (t, 1H) ; 3,79 (m, 1H) ; 3,40 (m, 2H) ; 2,81 (s, 3H) ; 1,04 (d, 3H).

## PREPARATION CXIX

**2,3-dichloro-N-(2-hydroxyéthyl)-N-méthylbenzènesulfonamide**

(huile incolore, rendement = 99%)
RMN$^1$H (300 MHz, DMSO) δ: 7,93 (m, 2H) ; 7,55 (t, 1H) ; 3,49 (m, 2H) ; 3,27 (t, 2H) ; 2,90 (s, 3H).

**PREPARATION CXX**

**2,6-dichloro-N-(2-hydroxyéthyl)-N-méthylbenzènesulfonamide**

(huile jaune, rendement = 99%)
RMN $^1$H (300 MHz, DMSO) δ : 7,67 (d, 2H) ; 7,57 (m, 1H) ; 4,79 (t, 1H) ; 3,54 (q, 2H) ; 3,29 (t, 2H) ; 2,92 (s, 3H).

**PREPARATION CXXI**

**2-chloro-N-(2-hydroxyéthyl)-N-méthylbenzènesulfonamide**

(huile, rendement = 99%)
RMN $^1$H (300 MHz, DMSO) δ: 7,95 (d, 1H) ; 7,66 (m, 2H) ; 7,60 (m, 1H) ; 4,79 (t, 1H) ; 3,53 (q, 2H) ; 3,25 (t, 2H) ; 2,88 (s, 3H).

**PREPARATION CXXII**

**Acide [2-[[(2,4-dichloro-3-méthylphényl)sulfonyl]méthylamino]éthoxy]acétique, 1,1-diméthyléthyl ester**

On prépare une solution de 100 mg (0,335 mM) de composé obtenu selon la préparation CXVII dans 4 ml de toluène et on ajoute 30 mg (0,111 mM) de chlorure de tétrabutylammonium, puis 4 ml d'une solution de soude à 35%. Le milieu réactionnel est refroidi à 10°C puis on ajoute 98 mg (0,5 mM) de bromoacétate de *t*-butyle. Le mélange est agité à température ambiante pendant 30 min, puis hydrolysé sur de l'eau glacée. Le mélange obtenu est extrait au toluène et la phase organique obtenue est séchée sur sulfate de magnésium et concentrée sous pression réduite. On obtient ainsi le produit attendu sous forme d'une huile incolore (rendement = 87%).
RMN $^1$H (300 MHz, DMSO) δ : 7,84 (d, 1H) ; 7,63 (d, 1H) ; 3,93 (s, 2H) ; 3,59 (t, 2H) ; 3,39 (t, 2H) ; 2,91 (s, 3H) ; 2,49 (s, 3H) ; 1,41 (s, 9H).
En opérant de façon analogue à la préparation CXXII, on obtient les composés suivants :

**PREPARATION CXXIII**

**Acide [2-[[(2,3-dichlorophényl)sulfonyl]méthylamino]propoxy]acétique, 1,1-diméthyléthyl ester**

(huile incolore, rendement = 88%)
RMN $^1$H (250 MHz, DMSO) δ : 8,05 (d, 1H) ; 8,02 (d, 1H) ; 7,92 (t, 1H) ; 4,01 (m, 1H) ; 3,82 (s, 2H) ; 3,47 (m, 2H) ; 2,85 (s, 3H) ; 1,42 (s, 9H) ; 1,03 (d, 3H).

**PREPARATION CXXIV**

**Acide [2-[[(2,3-dichlorophényl)sulfonyl]méthylamino]éthoxy]acétique, 1,1-diméthyléthyl ester**

(huile incolore, rendement = 96%)
RMN $^1$H (300 MHz, DMSO) δ : 7,94 (m, 2H) ; 7,56 (t, 1H) ; 3,93 (s, 2H) ; 3,60 (t, 2H) ; 3,40 (t, 2H) ; 2,94 (s, 3H) ; 1,42 (s, 9H).

**PREPARATION CXXV**

**Acide [2-[[(2,6-dichlorophényl)sulfonyl]méthylamino]éthoxy]acétique, 1,1-diméthyléthyl ester**

(huile jaune, rendement = 99%).
RMN $^1$H (250 MHz, DMSO) δ: 7,67 (d, 2H) ; 7,55 (m, 1H) ; 3,93 (s, 2H) ; 3,62 (t, 2H) ; 3,43 (d, 2H) ; 2,94 (s, 3H) ; 1,41 (s, 9H).

**PREPARATION CXXVI**

**Acide [2-[[(2-chlorophényl)sulfonyl]méthylamino]éthoxy]acétique, 1,1-diméthyléthyl ester**

(huile, rendement = 99%).
RMN$^1$H (300 MHz, DMSO) δ : 7,98 (d, 1H) ; 7,67 (m, 2H) ; 7,54 (m, 1H) ; 3,94 (s, 2H) ; 3,60 (t, 2H) ; 3,39 (t, 2H) ; 2,85

(s, 3H) ; 1,42 (s, 9H).

En opérant de façon analogue à la préparation VII, on obtient les composés suivants :

**PREPARATION CXXVII**

**Acide [2-[[(2,4-dichloro-3-méthylphényl)sulfonyl]méthylamino]éthoxy]acétique**

(huile incolore, rendement = 100%)
RMN $^1$H (300 MHz, DMSO) δ: 12,5 (s large, 1H) ; 7,86 (d, 1H) ; 7,64 (d, 1H) ; 4,02 (s, 2H) ; 3,61 (t, 2H) ; 3,40 (t, 2H) ; 2,91 (s, 3H) ; 2,51 (s, 3H).

**PREPARATION CXXVIII**

**Acide [2-[[(2,3-dichlorophényl)sulfonyl]méthylamino]propoxy]acétique**

(huile incolore, rendement = 81%).
RMN$^1$H (300 MHz, DMSO) δ :12,6 (s large, 1H) ; 8,06 (d, 1H) ; 7,92 (d, 1H) ; 7,53 (t, 1H) ; 3,98 (m, 1H) ; 3,82 (s, 2H) ; 3,50 (m, 2H) ; 2,85 (s, 3H) ; 1,05 (d, 3H).

En opérant de façon analogue à la préparation LXXVIII, on obtient les composés suivants :

**PREPARATION CXXIX**

**Acide [2-[[(2,3-dichlorophényl)sulfonyl]méthylamino]éthoxy]acétique**

(solide blanc écru, rendement = 100%).
F = 80°C

**PREPARATION CXXX**

**Acide [2-[[(2,6-dichlorophényl)sulfonyl]méthylamino]éthoxy]acétique**

**(huile jaune, rendement = 100%).**
RMN$^1$H (250 MHz, DMSO) δ: 12,6 (s large, 1H) ; 7,67 (d, 2H) ; 7,55 (m, 1H) ; 3,97 (s, 2H) ; 3,63 (t, 2H) ; 3,44 (t, 2H) ; 2,94 (s, 3H).

**PREPARATION CXXXI**

**Acide [2-[[(2-chlorophényl)sulfonyl]méthylamino]éthoxy]acétique**

(huile incolore, rendement = 99%).
RMN $^1$H (300 MHz, DMSO) δ : 12,5 (s large, 1H) ; 7,95 (d, 1H) ; 7,66 (m, 2H) ; 7,55 (m, 1H) ; 3,97 (s, 2H) ; 3,61 (t, 2H) ; 3,39 (t, 2H) ; 2,90 (s, 3H).

**PREPARATION CXXXII**

**Acide méthyl[[4-(1,4,5,6-tétrahydro-2-pyrimidinyl)phényl]méthyl]carbamique, 1,1-diméthyléthyl ester**

En opérant de façon analogue à la préparation CXV, au départ de 1,3-propanediamine, on obtient le produit attendu sous forme d'une huile incolore avec un rendement de 30%. RMN$^1$H (250 MHz, DMSO) δ: 7,75 (d, 2H) ; 7,37 (d, 2H) ; 4,44 (s, 2H) ; 3,47 (t, 4H) ; 2,79 (s, 3H) ; 1,90 (m, 2H) ; 1,40 (s, 9H).

**PREPARATION CXXXIII**

**N-méthyl-4-(1,4,5,6-tétrahydro-2-pyrimidinyl)-benzèneméthanamine, dichlorhydrate**

En opérant de façon analogue à la préparation CXVI, on obtient le produit attendu sous forme d'une huile jaune (rendement = 100%).
RMN $^1$H (250 MHz, DMSO) δ: 10,23 (s, 2H) ; 9,73 (s large, 2H) ; 7,83 (m, 4H) ; 4,20 (t, 2H) ; 3,50 (s, 4H) ; 2,51 (m, 3H) ;

1,95 (m, 2H).

**PREPARATION CXXXIV**

**Acide [[4-(4,5-dihydro-1-méthyl-1*H*-imidazol-2-yl)phényl]méthyl] méthylcarbamique,** 1,1-diméthyléthyl ester

En opérant de façon analogue à la préparation CXV, au départ de 1,2-propanediamine, on obtient le produit attendu sous forme d'une huile jaune (rendement = 74%).
RMN$^1$H (250 MHz, DMSO) δ: 7,49 (d, 2H) ; 7,26 (d, 2H) ; 4,4 (s, 2H) ; 3,68 (t, 2H) ; 3,34 (t, 2H) ; 2,78 (s, 3H) ; 2,70 (s, 3H) ; 1,40 (s, 9H).

**PREPARATION CXXXV**

**N-méthyl-4-(4,5-dihydro-1-méthyl-1*H*-imidazol-2-yl)benzèneméthanamine, dichlorhydrate**

En opérant de façon analogue à la préparation CXVI, on obtient le produit attendu sous forme d'une huile beige (rendement = 100%).
RMN$^1$H (250 MHz, DMSO) δ: 10,44 (s, 1H) ; 9,13 (s, 2H) ; 7,74 (m, 4H) ; 4,26 (s, 2H) ; 4,08 (m, 2H) ; 3,92 (m, 2H) ; 3,05 (s, 3H) ; 2,61 (s, 3H).

**PREPARATION CXXXVI**

**4-cyano-2-fluoro-N-méthylbenzèneméthanamine**

On refroidit 1 ml d'une solution de méthanamine à 16,5% dans l'éthanol par un bain de glace et on ajoute goutte à goutte une solution de-120 mg (0,56 mM) de 4-(bromométhyl)-3-fluoro-benzonitrile dans 1 ml de dichlorométhane. Le milieu réactionnel est ensuite agité pendant 4 heures à température ambiante puis est concentré sous pression réduite. Le résidu est repris avec de l'acétate d'éthyle et lavé avec une solution de bicarbonate de sodium. La phase organique est séchée sur sulfate de magnésium puis concentrée sous pression réduite. Le produit brut est purifié par chromatographie sur gel de silice en éluant à l'aide d'un mélange dichlorométhane/méthanol (98/0,05 ; v/v). On obtient ainsi l'amine attendue sous forme d'une huile incolore (rendement = 49%).
RMN$^1$H (300 MHz, DMSO) δ: 7,78 (d, 1H) ; 7,64 (m, 2H) ; 3,73 (s, 2H) ; 2,33 (s, 1H) ; 2,26 (s, 3H).
En opérant de façon analogue aux préparations I à IV, on obtient successivement les composés suivants :

**PREPARATION CXXXII**

**Acide [(2-fluoro-4-cyanophényl)méthyl]méthylcarbamique, phénylméthyl ester**

(huile jaune, rendement = 99%).
RMN$^1$H (300 MHz, DMSO) δ : 7,84 (d, 1H) ; 7,67 (m, 1H) ; 7,36 (m, 8H) ; 5,16 (d, 2H) ; 4,57 (s, 2H) ; 2,91 (s, 3H).

**PREPARATION CXXXVIII**

**Acide [[2-fluoro-4-(4,5-dihydro-1*H*-imidazol-2-yl)phényl]méthyl] méthylcarbamique, phénylméthyl ester**

(solide blanc, rendement = 70%).
F = 90°C

**PREPARATION CXXXIX**

**Acide 2-[3-fluoro-4-[[méthyl[(phénylméthoxy)carbonyl]amino]méthyl]phényl]-4,5-dihydro-1*H*-imidazole-1-carboxylique, 1,1-diméthyléthyl ester**

(solide blanc, rendement = 99%).
F = 131°C

**PREPARATION CXL**

**Acide 2-[3-fluoro-4-[(méthylamino)méthyl]phényl]-4,5-dihydro-1*H*-imidazole-1-carboxylique, 1,1-diméthyléthyl ester**

(huile jaune, rendement = 81 %).
RMN$^1$H (300 MHz, DMSO) δ: 7,44 (t, 1H) ; 7,23 (m, 3H) ; 3,84 (m, 4H) ; 3,69 (s, 2H) ; 2,25 (s, 3H) ; 1,21 (s, 9H).

En opérant de façon analogue à la préparation VIII, on obtient les composés suivants :

**PREPARATION CXLI**

**Acide 2-[4-[[[[2-[[(2,4-dichloro-3-méthylphényl)sulfonyl]méthylamino]éthoxy] acétyl]méthylamino]méthyl] phényl]-4,5-dihydro-1*H*-imidazole-1-carboxylique, 1,1-diméthyléthyl ester**

(huile incolore, rendement = 81%).
RMN$^1$H (250 MHz, DMSO) δ: 7,87 (t, 1H) ; 7,62 (m, 1H) ; 7,44 (m, 2H) ; 7,24 (d, 2H) ; 4,51 (s, 2H) ; 4,19 (d, 2H) ; 3,83 (m, 4H) ; 3,62 (m, 2H) ; 3,43 (m, 2H) ; 2,85 (m, 6H) ; 1,18 (s, 9H).

**PREPARATION CXLII**

**Acide 2-[4-[[[[2-[[(2,3-dichlorophényl)sulfonyl]méthylamino]propoxy]acétyl] méthylamino]méthyl]phényl]-4,5-dihydro-1*H*-imidazole-1-carboxylique, 1,1-diméthyléthyl ester**

(huile incolore, rendement = 52%).
RMN $^1$H (250 MHz, DMSO) δ: 8,08 (m, 1H) ; 7,89 (m, 1H) ; 7,50 (m, 3H) ; 7,30 (m, 2H) ; 4,49 (d, 2H) ; 4,05 (d, 2H) ; 3,98 (m, 1H) ; 3,84 (m, 4H) ; 3,49 (m, 2H) ; 2,83 (m, 6H) ; 1,18 (s, 1H) ; 1,04 (t, 3H).

**PREPARATION CXLIII**

**Acide 2-[4-[[[[2-[[(2,3-dichlorophényl)sulfonyl]méthylamino]éthoxy]acétyl] méthylamino]méthyl]phényl]-4,5-dihydro-1*H*-imidazole-1-carboxylique, 1,1-diméthyléthyl ester**

(huile incolore, rendement = 73%).
RMN$^1$H (300 MHz, DMSO) δ: 7,95 (m, 2H) ; 7,50 (m, 3H) ; 7,24 (d, 2H) ; 4,52 (s, 2H) ; 4,18 (d, 2H) ; 3,83 (m, 4H) ; 3,64 (m, 2H) ; 3,46 (m, 2H) ; 2,91 (m, 6H) ; 1,18 (s, 9H).

**PREPARATION CXLIV**

**Acide 2-[4-[[[[2-[[(2,6-dichlorophényl)sulfonyl]méthylamino]éthoxy]acétyl]méthyl amino]méthyl]phényl]-4,5-dihydro-1*H*-imidazole-1-carboxylique, 1,1-diméthyléthyl ester**

(huile incolore, rendement = 58%).
RMN $^1$H (300 MHz, DMSO) δ: 7,67 (m, 2H) ; 7,55 (m, 1H) ; 7,45 (m, 2H) ; 7,25 (d, 2H) ; 4,51 (s, 2H) ; 4,18 (d, 2H) ; 3,83 (m, 4H) ; 3,66 (m, 2H) ; 3,45 (m, 2H) ; 2,84 (m, 6H) ; 1,18 (s, 9H).

**Exemple 36**

**2-[2-[[(2,3-dichlorophényl)sulfonyl]méthylamino]éthoxy]-N-méthyl-N-[[4-(1,4,5,6-tétrahydro-2-pyrimidinyl) phényl]méthyl]acétamide**

(huile incolore, rendement = 25%).
RMN $^1$H (300 MHz, DMSO) δ: 7,95 (m, 2H) ; 7,50 (m, 3H) ; 7,24 (d, 2H) ; 4,52 (s, 2H) ; 4,18 (d, 2H) ; 3,83 (m, 4H) ; 3,64 (m, 2H) ; 3,46 (m, 2H) ; 2,91 (m, 6H) ; 1,18 (s, 9H).

**Exemple 37**

**2-[2-[[(2,4-dichloro-3-méthylphényl)sulfonyl]méthylamino]éthoxy]-N-[[4-(4,5-dihydro-1-méthyl-1*H*-imidazol-2-yl)phényl]méthyl]-N-méthyl-acétamide**

(huile jaune, rendement = 25%).
RMN$^1$H (300 MHz, DMSO) δ: 7,84 (t, 1H) ; 7,63 (m, 1H) ; 7,49 (m, 2H) ; 7,28 (m, 2H) ; 4,53 (s, 2H) ; 4,20 (d, 2H) ; 3,65 (m, 4H) ; 3,42 (m, 4H) ; 2,78 (m, 9H) ; 2,51 (s, 3H).

**Exemple 38**

**2-[2-[[(2,3-dichlorophényl)sulfonyl]méthylamino]éthoxy]-N-[[4-(4,5-dihydro-1-méthyl-1*H*-imidazol-2-yl)phényl]méthyl]-N-méthyl-acétamide**

(huile, rendement = 20%).
RMN $^1$H (250 MHz, DMSO) δ : 7,98 (m, 2H) ; 7,55 (m, 3H) ; 7,34 (m, 2H) ; 4,54 (s, 2H) ; 4,20 (d, 2H) ; 3,60 (m, 8H) ; 2,80 (m, 9H).

**PREPARATION CXLV**

**Acide 2-[4-[[[[2-[[(2-chlorophényl)sulfonyl]méthylamino]éthoxy]acétyl] méthylamino]méthyl]phényl]-4,5-dihydro-1*H*-imidazole-1-carboxylique, 1,1-diméthyléthyl ester**

(huile incolore, rendement = 46%).
RMN$^1$H (300 MHz, DMSO) δ: 7,97 (t, 1H) ; 7,65 (m, 2H) ; 7,56 (m, 1H) ; 7,47 (m, 2H) ; 7,24 (d, 2H) ; 4,52 (s, 2H) ; 4,20 (d, 2H) ; 3,83 (m, 4H ; 3,63 (m, 2H) ; 3,40 (m, 2H) ; 2,84 (m, 6H) ; 1,18 (s, 9H).

**PREPARATION CXLVI**

**Acide 2-[4-[[[[2-[[(2,3-dichlorophényl)sulfonyl]méthylamino]éthoxy]acétyl] méthylamino]méthyl]-3-fluorophényl]-4,5-dihydro-1*H*-imidazole-1-carboxylique, 1,1-diméthyléthyl ester**

(huile incolore, rendement = 46%).
RMN$^1$H (250 MHz, DMSO) δ: 7,94 (m, 2H) ; 7,55 (dd, 1H) ; 7,26 (m, 3H) ; 4,56 (s, 2H) ; 4,20 (d, 2H) ; 3,85 (m, 4H) ; 3,61 (m, 2H) ; 3,45 (m, 2H) ; 2,85 (m, 6H) ; 1,20 (s, 9H).

En opérant de façon analogue à l'exemple 1, on obtient les composés suivants :

**Exemple 39**

**2-[2-[[(2,4-dichloro-3-méthylphényl)sulfonyl]méthylamino]éthoxy]-N-[[4-(4,5-dihydro-1*H*-imidazol-2-yl)phényl]méthyl]-N-méthyl-acétamide, trifluoroacétate**

(solide blanc, rendement = 98%).
F = 75°C

**Exemple 40**

**2-[2-[[(2,3-dichlorophényl)sulfonyl]méthylamino]éthoxy]-N-[[4-(4,5-dihydro-1*H*-imidazol-2-yl)phényl]méthyl]-N-méthyl-acétamide, trifluoroacétate**

(solide blanc, rendement = 96%).
F = 60°C

**Exemple 41**

**2-[2-[[(2,6-dichlorophényl)sulfonyl]méthylamino]éthoxy]-N-[[4-(4,5-dihydro-1*H*-imidazol-2-yl)phényl]méthyl]-N-méthyl-acétamide, trifluoroacétate**

(solide blanc, rendement = 96%).
F = 60°C

**Exemple 42**

**2-[2-[[(2-chlorophényl)sulfonyl]méthylamino]éthoxy]-N-[[4-(4,5-dihydro-1*H*-imidazol-2-yl)phényl]méthyl]-N-méthyl-acétamide, trifluoroacétate**

(solide blanc, rendement = 99%).
F = 60°C

**Exemple 43**

**2-[2-[[(2,3-dichlorophényl)sulfonyl]méthylamino]éthoxy]-N-[[2-fluoro-4-(4,5-dihydro-1*H*-imidazol-2-yl)phényl]méthyl]-N-méthyl-acétamide, trifluoroacétate**

(solide blanc, rendement = 87%).
F = 80°C

En opérant de façon analogue à la préparation CXVI au départ du composé obtenu selon la Préparation CXLII, on obtient le composé suivant :

**Exemple 44**

**2-[2-[[(2,3-dichlorophényl)sulfonyl]méthylamino]propoxy]-N-[[4-(4,5-dihydro-1*H*-imidazol-2-yl)phényl]méthyl]-N-méthyl-acétamide, chlorhydrate**

(solide blanc, rendement = 85%).
F = 104°C

**Exemple 45**

**2-[2-[[(2,3-dichlorophényl)sulfonyl]méthylamino]éthoxy]-N-méthyl-N-[[4-(1,4,5,6-tétrahydro-2-pyrimidinyl)phényl]méthyl]acétamide, trifluoroacétate**

On prépare une solution de 40 mg (0,075 mM) du composé obtenu selon l'exemple 36 dans 10 ml de méthanol et on ajoute 5,9 µl d'acide trifluoroacétique. Le mélange est agité 15 min à température ambiante puis concentré sous pression réduite. Le résidu est repris dans 3 ml d'eau pure et la solution obtenue est filtrée. Le filtrat est lyophilisé. On obtient ainsi 35 mg du composé attendu sous forme d'une poudre blanche (rendement = 73%).
RMN $^{1}H$ (250 MHz, DMSO) δ : 10,05 (s large, 2H) ; 7,98 (m, 2H) ; 7,73 (m, 2H) ; 7,56 (m, 4H) ; 4,59 (s, 2H) ; 4,20 (d, 2H) ; 3,65 (m, 2H) ; 3,46 (m, 6H) ; 2,87 (m, 6H) ; 1,98 (m, 2H).

**Exemple 46**

**2-[2-[[(2,4-dichloro-3-méthylphényl)sulfonyl]méthylamino]éthoxy]-N-[[4-(4,5-dihydro-1-méthyl-1*H*-imidazol-2-yl)phényl]méthyl]-N-méthyl-acétamide, chlorhydrate**

On dissout 90 mg (0,166 mM) du composé obtenu selon l'exemple 37 dans 3 ml de méthanol et on ajoute 1 ml d'une solution saturée de chlorure d'hydrogène dans l'éther éthylique. Le mélange est maintenu sous agitation à température ambiante pendant 1 heure ; on ajoute 10 ml d'éther éthylique puis on concentre sous pression réduite. Le résidu est repris dans 6 ml d'eau pure, la solution obtenue est filtrée puis lyophilisée. On obtient ainsi 95 mg du produit attendu sous forme d'un solide blanc cristallisé (rendement = 98%).
F = 60°C

## Exemple 47

**2-[2-[[(2,3-dichlorophényl)sulfonyl]méthylamino]éthoxy]-N-[[4-(4,5-dihydro-1-méthyl-1*H*-imidazol-2-yl)phényl]méthyl]-N-méthyl-acétamide, chlorhydrate**

En opérant de façon analogue à l'exemple 46, au départ du composé obtenu selon l'exemple 38, on obtient le composé attendu sous forme d'une huile incolore (rendement = 53%).
RMN $^1$H (250 MHz, DMSO) δ: 10,20 (s, 1H) ; 7,98 (m, 2H) ; 7,65 (m, 2H) ; 7,53 (m, 3H) ; 4,60 (s, 2H) ; 4,20 (d, 2H) ; 3,95 (m, 4H) ; 3,70 (m, 4H) ; 3,06 (s, 3H) ; 2,90 (m, 6H).

## PREPARATION CXLVII

**Acide (2-hydroxyéthyl)méthylcarbamique, 1,1-diméthyléthyl ester**

En opérant de façon analogue à la préparation CXIV, au départ de 2-(méthylamino)éthanol, on obtient le composé attendu sous forme d'une huile incolore (rendement = 87%).
RMN$^1$H (300 MHz, DMSO) δ: 4,65 (t, 1H) ; 3,45 (q, 2H) ; 3,19 (t, 2H) ; 2,81 (s, 3H) ; 1,38 (s, 9H).

## PREPARATION CXLVIII

**Acide [2-[[(1,1-diméthyléthoxy)carbonyl]méthylamino]éthoxy]acétique, 1,1-diméthyléthyl ester**

En opérant de façon analogue à la préparation CXXII, au départ du composé obtenu selon la préparation CXLVII, on obtient le produit attendu sous forme d'une huile jaune (rendement = 99%).
RMN$^1$H (250 MHz, DMSO) δ: 3,96 (s, 2H) ; 3,52 (t, 2H) ; 3,31 (t, 2H) ; 2,81 (s, 3H) ; 1,42 (s, 9H) ; 1,38 (s, 9H).

## PREPARATION CIL

**Acide [2-(méthylamino)éthoxy]acétique, trifluoroacétate**

En opérant de façon analogue à la préparation LXXVIII, au départ du composé obtenu selon la préparation CXLVIII, on obtient le produit attendu sous forme d'une huile jaune (rendement = 99%).
RMN $^1$H (250 MHz, DMSO) δ: 8,50 (s large, 1H) ; 4,09 (s, 2H) ; 3,70 (m, 2H) ; 3,1 (m, 2H) ; 2,60 (m, 3H).

## PREPARATION CL

**Acide [2-[méthyl[(phénylméthoxy)carbonyl]amino]éthoxylacétique**

On prépare une solution de 25 g (0,101 mM) du composé obtenu selon la préparation CIL, dans 400 ml de dichlorométhane et on ajoute à 0˚C 35,2 ml (0,25 mM) de triéthylamine puis, goutte à goutte, 15,5 ml de chloroformiate de benzyle. Le mélange réactionnel est agité pendant 5 heures à température ambiante. Le mélange réactionnel est hydrolysé sur 200 ml d'eau glacée et 50 ml d'acide chlorhydrique N. La phase organique séparée est lavée à l'eau puis séchée sur sulfate de magnésium et concentrée sous pression réduite. Le produit brut huileux est purifié par chromatographie sur gel de silice en éluant avec un mélange toluène/isopropanol/ammoniaque (9/1/0,1 ; v/v/v). on obtient ainsi 13,7 g du produit attendu sous forme d'une huile incolore (rendement = 51%).
RMN$^1$H (250 MHz, DMSO) δ: 7,33 (m, 5H) ; 5,06 (s, 2H) ; 4,10 (s, 2H) ; 3,58 (t, 2H) ; 3,49 (m, 2H) ; 2,90 (d, 3H).

## PREPARATION CLI

**Acide 2-[4-(2,8-diméthyl-3,9-dioxo-11-phényl-5,10-dioxa-2,8-diazaundec-1-yl) phényl]-4,5-dihydro-1*H*-imidazole-1-carboxylique,1,1-diméthyléthyl ester**

En opérant de façon analogue à la préparation VIII, au départ de l'acide obtenu selon la préparation CL, on obtient le produit attendu sous forme d'une huile jaune (rendement = 62%). RMN $^1$H (250 MHz, DMSO) δ: 7,42-7,22 (m, 9H) ; 5,05 (s, 2H) ; 4,51 (s, 2H) ; 4,21 (s, 2H) ; 3,84 (m, 4H) ; 3,58 (m, 2H) ; 3,45 (m, 2H) ; 2,88 (m, 6H) ; 1,17 (s, 9H).

## PREPARATION CLII

**Acide 4,5-dihydro-2-[4-[[méthyl[[2-(méthylamino)éthoxy]acétyl] amino] méthyl]phényl]-1*H*-imidazole-1-carboxylique, 1,1-diméthyléthyl ester**

En opérant de façon analogue à la préparation IV, au départ du composé obtenu selon la préparation CLI, on obtient le produit attendu sous forme d'une huile jaune (rendement = 91%). RMN$^1$H (250 MHz, DMSO) δ: 7,41 (d, 2H) ; 7,26 (d, 2H) ; 4,53 (d, 2H) ; 4,20 (d, 2H) ; 3,84 (m, 4H) ; 3,50 (m, 2H) ; 2,86 (s, 3H) ; 2,63 (m, 2H) ; 2,26 (d, 3H) ; 1,18 (s, 9H).

En opérant de façon analogue à la préparation V, au départ du composé obtenu selon la préparation CLII, on obtient les composés suivants :

## PREPARATION CLIII

**Acide 4,5-dihydro-2-[4-[[méthyl[[2-[méthyl[[2-(trifluorométhyl)phényl]sulfonyl] amino]éthoxy]acétyl]amino] méthyl]phényl]-1*H*-imidazole-1-carbozylique, 1,1-diméthyléthyl ester**

RMN$^1$H (300 MHz, DMSO) δ: 8,02 (m, 2H) ; 7,87 (m, 2H) ; 7,44 (m,2H) ; 7,23 (d, 2H) ; 4,53 (s, 2H) ; 4,23 (d, 2H) ; 3,83 (m, 4H) ; 3,65 (m, 2H) ; 3,48 (m, 2H) ; 2,88 (m, 6H) ; 1,17 (s, 9H).

## PREPARATION CLIV

**Acide 4,5-dihydro-2-[4-[[[[2-[[(4-méthoxy-2,6-diméthylphényl)sulfonyl]méthyl amino]éthoxy]acétyl]méthylamino]méthyl]phényl]-1*H*-imidazole-1-carboxylique, 1,1-diméthyléthyl ester**

(huile incolore, rendement = 74%).
RMN $^1$H (300 MHz, DMSO) δ: 7,42 (m, 2H) ; 7,22 (d, 2H) ; 6,80 (d, 2H) ; 4,50 (s, 2H) ; 4,15 (d, 2H) ; 3,84 (m, 4H) ; 3,79 (s, 3H) ; 3,57 (m, 2H) ; 3,22 (m, 2H) ; 2,76 (m, 6H) ; 2,53 (s, 6H); 1,17 (s, 9H).

## PREPARATION CLV

**Acide 2-[[[[2-[[(2,4-dichlorophényl)sulfonyl]méthylamino]éthoxy]acétyl] méthylamino]méthyl]phényl]-4,5-dihydro-1*H*-imidazole-1-carboxylique, 1,1-diméthyléthyl ester**

(huile incolore, rendement = 70%).
RMN $^1$H (250 MHz, DMSO) δ: 7,89 (m, 2H) ; 7,60 (m, 1H) ; 7,43 (m, 2H) ; 7,23 (d, 2H) ; 4,51 (s, 2H) ; 4,20 (d, 2H) ; 3,84 (m, 4H) ; 3,60 (m, 2H) ; 3,42 (m, 2H) ; 2,83 (m, 6H) ; 1,18 (s, 9H).

## PREPARATION CLVI

**Acide 2-[4-[[[[2-[[(2,4-dichloro-5-méthylphényl)sulfonyl]méthylamino]éthoxy] acétyl]méthylamino]méthyl] phényl]-4,5-dihydro-1*H*-imidazole-1-carboxylique,1,1-diméthyléthyl ester**

(huile incolore, rendement = 43%).
RMN $^1$H (300 MHz, DMSO) δ: 7,94 (m, 1H) ; 7,82 (m, 1H) ; 7,42 (m, 2H) ; 7,23 (d, 2H) ; 4,52 (s, 2H) ; 4,20 (d, 2H) ; 3,83 (m, 4H) ; 3,60 (m, 2H) ; 3,42 (m, 2H) ; 2,85 (m, 6H) ; 2,38 (s, 3H) ; 1,18 (s, 9H).

## PREPARATION CLVII

**Acide 4,5-dihydro-2-[4-[[méthyl[[2-[méthyl[(2-nitrophényl)sulfonyl] amino]éthoxy]acétyl]amino]méthyl]phényl]-1*H*-imidazole-1-carboxylique, 1,1-diméthyléthyl ester**

(mousse blanche, rendement = 50%).
F = 60°C

**PREPARATION CLVIII**

**Acide 2-[4-[[[[2-[[[2,6-dichloro-4-(trifluorométhyl)phényl]sulfonyl] méthylamino] éthoxy]acétyl]méthylamino] méthyl]phényl]-4,5-dihydro-1*H*-imidazole-1-carboxylique, 1,1-diméthyléthyl ester**

(huile incolore, rendement = 55%).
RMN $^1H$ (300 MHz, DMSO) δ: 8,08 (d, 2H) ; 7,42 (m, 2H) ; 7,22 (d, 2H) ; 4,50 (s,2H) ; 4,18 (d, 2H) ; 3,83 (m, 4H) ; 3,64 (m, 2H) ; 3,50 (m, 2H) ; 2,80 (m, 6H) ; 1,18 (s, 9H).

**PREPARATION CLIX**

**Acide 2-[4-[[[[2-[[(2-chloro-3-méthylphényl)sulfonyl]méthylamino]éthoxy]acétyl] méthylamino]méthyl]phényl]-4,5-dihydro-1*H*-imidazole-1-carboxylique, 1,1-diméthyléthyl ester**

(solide blanc, rendement = 63%).
F = 50°C

**PREPARATION CLX**

**Acide 2-[4-[[[[2-[[(2-cyanophényl)sulfonyl]méthylamino]éthoxy]acétyl]méthyl amino]méthyl]phényl]-4,5-dihydro-1*H*-imidazole-1-carboxylique,1,1-diméthyléthyl ester**

(solide blanc, rendement = 56%).
F = 60°C

**PREPARATION CLXI**

**Acide 4,5-dihydro-2-[4-[[méthyl[[2-[méthyl[(2,3,4-(trichlorophényl)sulfonyl] amino]éthoxy]acétyl]amino]méthyl]phényl]-1*H*-imidazole-1-carboxylique, 1,1-diméthyléthyl ester**

(solide blanc, rendement = 59%).
F = 60°C

**PREPARATION CLXII**

**Acide 2-[4-[[[[2-[[(3-chloro-2-méthylphényl)sulfonyl]méthylamino]éthoxy]acétyl] méthylamino]méthyl]phényl]-4,5-dihydro-1*H*-imidazole-1-carboxylique, 1,1-diméthyléthyl ester**

(huile incolore, rendement = 57%).
RMN $^1H$ (250 MHz, DMSO) δ: 7,78 (m, 2H) ; 7,41 (m, 3H) ; 7,23 (d, 2H) ; 4,52 (s, 2H) ; 4,20 (d, 2H) ; 3,82 (m, 4H) ; 3,59 (m, 2H) ; 3,39 (m, 2H) ; 2,83 (m, 6H) ; 2,59 (d, 3H) ; 1,18 (s, 9H).

**PREPARATION CLXIII**

**Acide 2-[4-[[[[2-[[(2-chloro-4-cyanophényl)sulfonyl]méthylamino]éthoxy]acétyl] méthylamino]méthyl]phényl]-4,5-dihydro-1*H*-imidazole-1-carboxylique, 1,1-diméthyléthyl ester**

(solide blanc, rendement = 51%).
F = 60°C

**PREPARATION CLXIV**

**Acide 4,5-dihydro-2-[4-[[méthyl[[2-[méthyl[[2-nitro-4-(trifluorométhyl)phényl] sulfonyl]amino]éthoxy]acétyl] amino]méthyl]phényl]-1*H*-imidazole-1-carboxylique, 1,1-diméthyléthyl ester**

(huile incolore, rendement = 47%)
RMN $^1H$ (300 MHz, DMSO) δ: 8,59 (s, 1H) ; 8,27 (m, 2H) ; 7,42 (m, 2H) ; 7,23 (d, 2H) ; 4,51 (s, 2H) ; 4,20 (d, 2H) ; 3,83 (m, 4H) ; 3,64 (m, 2H) ; 3,47 (m, 2H) ; 2,90 (m, 6H) ; 1,18 (s, 9H).

## PREPARATION CLXV

**Acide 2-[4-[[[[2-[[(2,6-difluorophényl)sulfonyl]méthylamino]éthoxy]acétyl] méthylamino]méthyl]phényl]-4,5-dihydro-1*H*-imidazole-1-carboxylique, 1,1-diméthyléthyl ester**

(huile incolore, rendement = 49%).
RMN $^1$H (250 MHz, DMSO) δ: 7,74 (m, 1H) ; 7,43 (m, 2H) ; 7,27 (m, 4H) ; 4,51 (s, 2H) ; 4,16 (d, 2H) ; 3,82 (m, 4H) ; 3,60 (m, 2H) ; 3,56 (m, 2H) ; 2,82 (m, 6H) ; 1,18 (s, 9H).

## PREPARATION CLXVI

**Acide 4,5-dihydro-2-[4-[[méthyl[[2-[méthyl[[4-(trifluorométhoxy)phényl]sulfonyl] amino]ethoxy]acétyl]amino] méthyl]phényl]-1*H*-imidazole-1-carboxylique, 1,1-diméthyléthyl ester**

(huile incolore, rendement = 63%).
RMN$^1$H (250 MHz, DMSO) δ : 7,93 (m, 2H) ; 7,60 (d, 2H) ; 7,42 (m, 2H) ; 7,23 (d, 2H) ; 4,51 (s, 2H) ; 4,19 (d, 2H) ; 3,84 (m, 4H) ; 3,59 (m, 2H) ; 3,22 (m, 2H) ; 2,78 (m, 6H) ; 1,18 (s, 9H).

## PREPARATION CLXVII

**Acide 2-[4-[[[[2-[[(2,5-dichlorothien-3-yl)sulfonyl]méthylamino]éthoxy]acétyl] méthylamino]méthyl]phényl]-4,5-dihydro-1*H*-imidazole-1-carboxylique, 1,1-diméthyléthyl ester**

(huile incolore, rendement = 30%).
RMN$^1$H (250 MHz, DMSO) δ: 7,41 (m, 3H) ; 7,23 (d, 2H) ; 4,52 (s, 2H) ; 4,21 (d, 2H) ; 3,84 (m, 4H) ; 3,62 (m, 2H) ; 3,37 (m, 2H) ; 2,84 (m, 6H) ; 1,18 (s, 9H).

## PREPARATION CLXVIII

**Acide 4,5-dihydro-2-[4-[[méthyl[[2-[méthyl[(3-méthylphényl)sulfonyl] amino]éthoxy]acétyl]amino]méthyl]phényl]-1*H*-imidazole-1-carboxylique, 1,1-diméthyléthyl ester**

(huile incolore, rendement = 33%).
RMN$^1$H (300 MHz, DMSO) δ : 7,60 (m, 2H) ; 7,56 (m, 2H) ; 7,50 (m, 2H) ; 7,22 (d, 2H) ; 4,52 (s, 2H) ; 4,20 (d, 2H) ; 3,84 (m, 4H) ; 3,57 (m, 2H) ; 3,17 (m, 2H) ; 2,76 (m, 6H) ; 2,40 (s, 3H) ; 1,18 (s, 9H).

## PREPARATION CLXIX

**Acide 4,5-dihydro-2-[4-[[méthyl[[2-[méthyl(1-naphtalénylsulfonyl)amino] éthoxy]acétyl]amino]méthyl]phényl]-1*H*-imidazole-1-carboxylique, 1,1-diméthyléthyl ester**

(huile incolore, rendement = 33%).
RMN $^1$H (300 MHz, DMSO) δ: 8,60 (m, 1H) ; 8,20 (m, 1H) ; 8,08 (m, 2H) ; 7,68 (m, 3H) ; 7,46 (m, 2H) ; 7,23 (d, 2H) ; 4,50 (d, 2H) ; 4,13 (d, 2H) ; 3,84 (m, 4H) ; 3,59 (m, 2H) ; 3,41 (m, 2H) ; 2,86 (m, 6H) ; 1,17 (s, 9H).

## PREPARATION CLXX

**Acide 4,5-dihydro-2-[4-[[méthyl[[2-[méthyl[[3-(trifluorométhyl)phényl]sulfonyl] amino]éthoxy]acétyl]amino] méthyl]phényl]-1*H*-imidazole-1-carboxylique, 1,1-diméthyléthyl ester**

(huile incolore, rendement = 66%).
RMN $^1$H (300 MHz, DMSO) δ: 8,12 (m, 3H) ; 7,85 (m, 1H) ; 7,44 (m, 2H) ; 7,23 (d, 2H) ; 4,50 (s, 2H) ; 4,15 (d, 2H) ; 3,82 (m, 4H) ; 3,60 (m, 2H) ; 3,26 (m, 2H) ; 2,81 (m, 6H) ; 1,18 (s, 9H).

**PREPARATION CLXXI**

**Acide 2-[4-[[[[2-[[(4-chloro-3-méthylphényl)sulfonyl]méthylamino]éthoxy]acétyl] méthylamino]méthyl]phényl]-4,5-dihydro-1*H*-imidazole-1-carboxylique, 1,1-diméthyléthyl ester**

(solide blanc, rendement = 59%).
F = 60°C

**PREPARATION CLXXII**

**Acide 2-[4-[[[[2-[[(2,4-difluorophényl)sulfonyl]méthylamino]éthoxy]acétyl] méthylamino]méthyl]phényl]-4,5-dihydro-1*H*-imidazole-1-carboxylique,1,1-diméthyléthyl ester**

(huile incolore, rendement = 64%).
RMN $^1$H (250 MHz, DMSO) δ: 7,87 (m, 1H) ; 7,43 (m, 3H) ; 7,20 (m, 3H) ; 4,51 (s, 2H) ; 4,17 (d, 2H) ; 3,84 (m, 4H) ; 3,61 (m, 2H) ; 3,34 (m, 2H) ; 2,84 (m, 6H) ; 1,18 (s, 9H).

**PREPARATION CLXXIII**

**Acide 2-[4-[[[[2-[[(3-chlorophényl)sulfonyl]méthylamino]éthoxy]acétyl] méthylamino]méthyl]phényl]-4,5-dihydro-1*H*-imidazole-1-carboxylique, 1,1-diméthyléthyl ester**

(huile incolore, rendement = 51%).
RMN $^1$H (300 MHz, DMSO) δ: 7,78 (m, 3H) ; 7,66 (m, 1H) ; 7,45 (m, 2H) ; 7,23 (d, 2H) ; 4,51 (s, 2H) ; 4,19 (d, 2H) ; 3,82 (m, 4H) ; 3,52 (m, 2H) ; 3,37 (m, 2H) ; 2,78 (m, 6H) ; 1,18(s, 9H).
En opérant de façon analogue à l'exemple 1, on obtient les composés suivants :

**Exemple 48**

**N-[[4-(4,5-dihydro-1*H*-imidazol-2-yl)phényl]méthyl]-N-méthyl-2-[2-[méthyl[[2-(trifluorométhyl)phényl]sulfonyl] amino]éthoxy]acétamide, trifluoroacétate**

(solide fin blanc, rendement = 96%).
F = 60°C

**Exemple 49**

**N-[[4-(4,5-dihydro-1*H*-imidazol-2-yl)phényl]méthyl]-2-[2-[2-(4-méthoxy-2,6-diméthylphényl)sulfonyl]méthylamino]éthoxy]-N-méthyl-acétamide, trifluoroacétate**

(solide pâteux, rendement = 99%).
RMN $^1$H (250 MHz, DMSO) δ : 10,48 (s large, 2H) ; 7,89 (d, 2H) ; 7,48 (d, 2H) ; 6,79 (s, 2H) ; 4,59 (s, 2H) ; 4,15 (d, 2H) ; 4,01 (s, 4H) ; 3,79 (s, 3H) ; 3,59 (m, 2H) ; 3,24 (m, 2H) ; 2,71 (m, 6H) ; 2,53 (s, 6H).

**Exemple 50**

**2-[2-[[(2,4-dichlorophényl)sulfonyl]méthylamino]éthoxy]-N-[[4-(4,5-dihydro-1*H*-imidazol-2-yl)phényl]méthyl]-N-méthyl-acétamide, trifluoroacétate**

(solide blanc, rendement = 99%).
F = 60°C

**Exemple 51**

**2-[2-[[(2,4-dichloro-5-méthylphényl)sulfonyl]méthylamino]éthoxy]-N-[[4-(4,5-dihydro-1*H*-imidazol-2-yl)phényl]méthyl]-N-méthyl-acétamide, trifluoroacétate**

(solide pâteux, rendement = 99%).

RMN $^1H$ (250 MHz, DMSO) δ : 10,5 (d, 2H) ; 7,87 (m, 4H) ; 7,50 (d, 2H) ; 4,60 (s, 2H) ; 4,10 (d, 2H) ; 4,01 (s, 4H) ; 3,62 (m, 2H) ; 3,45 (m, 2H) ; 2,85 (m, 6H) ; 2,38 (s, 3H).

**Exemple 52**

**N-[[4-(4,5-dihydro-1*H*-imidazol-2-yl)phényl]méthyl]-N-méthyl-2-[2-[méthyl[(2-nitrophényl)sulfonyl]amino] éthoxy]acétamide, trifluoroacétate**

(pâte jaune, rendement = 99%).
RMN $^1H$ (300 MHz, DMSO) δ: 10,49 (s, 2H) ; 7,89 (m, 6H) ; 7,49 (d, 2H) ; 4,61 (s, 2H) ; 4,22 (d, 2H) ; 4,01 (s, 4H) ; 3,66 (m, 2H) ; 3,41 (m, 2H) ; 2,90 (m, 6H).

**Exemple 53**

**2-[2-[[[2,6-dichloro-4-(trifluorométhyl)phényl]sulfonyl]méthylamino]éthoxy]-N-[[4-(4,5-dihydro-1*H*-imidazol-2-yl)phényl]méthyl]-N-méthyl-acétamide, trifluoroacétate**

(solide blanc, rendement = 99%).
F = 60°C

**Exemple 54**

**2-[2-[[(2-chloro-3-méthylphényl)sulfonyl]méthylamino]éthoxy]-N-[[4-(4,5-dihydro-1*H*-imidazol-2-yl)phényl] méthyl]-N-méthyl-acétamide, trifluoroacétate**

(solide blanc, rendement = 67%).
F = 63°C

**Exemple 55**

**2-[2-[[(2-cyanophényl)sulfonyl]méthylamino]éthoxy]-N-[[4-(4,5-dihydro-1*H*-imidazol-2-yl)phényl]méthyl]-N-méthyl-acétamide, trifluoroacétate**

(solide blanc, rendement = 94%).
F = 60°C

**Exemple 56**

**N-[[4-(4,5-dihydro-1*H*-imidazol-2-yl)phényl]méthyl]-N-méthyl-2-[2-[méthyl[(2,3,4-trichlorophényl)sulfonyl] amino]éthoxy]acétamide, trifluoroacétate**

(solide blanc, rendement = 99%).
F = 70°C

**Exemple 57**

**2-[2-[[(3-chloro-2-méthylphényl)sulfonyl]méthylamino]éthoxy]-N-[[4-(4,5-dihydro-1*H*-imidazol-2-yl)phényl] méthyl]-N-méthyl-acétamide, trifluoroacétate**

(solide fin blanc, rendement = 74%).
F = 60°C

**Exemple 58**

**2-[2-[[(2-chloro-4-cyanophényl)sulfonyl]méthylamino]éthoxy]-N-[[4-(4,5-dihydro-1*H*-imidazol-2-yl)phényl]méthyl]-N-méthyl-acétamide, trifluoroacétate**

(solide blanc, rendement = 97%).

F = 64°C

**Exemple 59**

**N-[[4-(4,5-dihydro-1*H*-imidazol-2-yl)phényl]méthyl]-N-méthyl-2-[2-[méthyl[[2-nitro-4-(trifluorométhyl)phényl] sulfonyl]amino]éthoxy]acétamide, trifluoroacétate**

(solide blanc, rendement = 85%).
RMN $^1$H (250 MHz, DMSO) δ: 10,47 (s, 2H) ; 8,59 (s, 1H) ; 8,23 (m, 2H) ; 7,92 (m, 2H) ; 7,48 (d, 2H) ; 4,60 (s, 2H) ; 4,20 (d, 2H) ; 4,01 (s, 4H) ; 3,65 (m, 2H) ; 3,47 (m, 2H) ; 2,85 (m, 6H).

**Exemple 60**

**2-[2-[[(2,6-difluorophényl)sulfonyl]méthylamino]éthoxy]-N-[[4-(4,5-dihydro-1*H*-imidazol-2-yl)phényl]méthyl]-N-méthyl-acétamide, trifluoroacétate**

(mousse, rendement = 99%).
RMN $^1$H (250 MHz, DMSO) δ : 10,47 (s, 2H) ; 7,92 (m, 2H) ; 7,76 (m, 1H) ; 7,47 (d, 2H) ; 7,27 (t, 2H) ; 4,60 (s, 2H) ; 4,20 (d, 2H) ; 4,01 (s, 4H) ; 3,65 (m, 2H) ; 3,37 (m, 2H) ; 2,89 (m, 6H).

**Exemple 61**

**N-[[4-(4,5-dihydro-1*H*-imidazol-2-yl)phényl]méthyl]-N-méthyl-2-[2-[méthyl[[4-(trifluorométhoxy)phényl]sulfonyl]amino]éthoxy]acétamide, trifluoroacétate**

(huile incolore, rendement = 67%).
RMN $^1$H (250 MHz, DMSO) δ : 10,51 (s, 2H) ; 7,91 (t, 4H) ; 7,59 (d, 2H) ; 7,48 (d, 2H) ; 4,60 (s, 2H) ; 4,20 (d, 2H) ; 4,01 (s, 4H) ; 3,61 (m, 2H) ; 3,21 (m, 2H) ; 2,75 (m, 6H).

**Exemple 62**

**2-[2-[[(2,5-dichlorothién-3-yl)sulfonyl]méthylamino]éthoxy]-N-[[4-(4,5-dihydro-1*H*-imidazol-2-yl)phényl]méthyl]-N-méthyl-acétamide, trifluoroacétate**

(solide blanc, rendement = 99%).
F = 60°C

**Exemple 63**

**N-[[4-(4,5-dihydro-1*H*-imidazol-2-yl)phényl]méthyl]-N-méthyl-2-[2-[méthyl[(3-méthylphényl)sulfonyl]amino] éthoxy]acétamide, trifluoroacétate**

(huile incolore, rendement = 68%).
RMN $^1$H (250 MHz, DMSO) δ : 10,48 (s, 2H) ; 7,88 (m, 2H) ; 7,61 (m, 2H) ; 7,51 (m, 4H) ; 4,63 (s, 2H) ; 4,20 (d, 2H) ; 4,01 (s, 4H) ; 3,62 (m, 2H) ; 3,09 (m, 2H) ; 2,76 (m, 6H) ; 2,40 (s, 3H).

**Exemple 64**

**N-[[4-(4,5-dihydro-1*H*-imidazol-2-yl)phényl]méthyl]-N-méthyl-2-[2-[méthyl(1-naphtalénylsulfonyl)amino] éthoxy]acétamide, trifluoroacétate**

(solide blanc, rendement = 99%).
F = 66°C

**Exemple 65**

**N-[[4-(4,5-dihydro-1*H*-imidazol-2-yl)phényl]méthyl]-N-méthyl-2-[2-[méthyl[[3-(trifluorométhyl)phényl]sulfonyl] amino]éthoxy]acétamide, trifluoroacétate**

(solide blanc, rendement = 99%).
F = 60˚C

**Exemple 66**

**2-[2-[[(4-chloro-3-méthylphényl)sulfonyl]méthylamino]éthoxy]-N-[[4-(4,5-dihydro-1*H*-imidazol-2-yl)phényl] méthyl]-N-méthyl-acétamide, trifluoroacétate**

(solide blanc, rendement = 59%).
F = 60˚C

**Exemple 67**

**2-[2-[[(2,4-difluorophényl)sulfonyl]méthylamino]éthoxy]-N-[[4-(4,5-dihydro-1*H*-imidazol-2-yl)phényl]méthyl]-N-méthyl-acétamide, trifluoroacétate**

(huile incolore, rendement = 99%).
RMN $^1$H (250 MHz, DMSO) δ : 10,51 (s, 2H) ; 7,88 (m, 3H) ; 7,53 (m, 3H) ; 7,29 (m, 1H) ; 4,60 (s, 2H) ; 4,20 (d, 2H) ; 4,01 (s, 4H) ; 3,60 (m, 2H) ; 3,26 (m, 2H) ; 2,85 (m, 6H).

**Exemple 68**

**2-[2-[[(3-chlorophényl)sulfonyl]méthylamino]éthoxy]-N-[[4-(4,5-dihydro-1*H*-imidazol-2-yl)phényl]méthyl]-N-méthyl-acétamide, trifluoroacétate**

(solide blanc, rendement = 99%).
F = 60˚C

**PREPARATION CLXXIV**

**5-bromo-N-[(4-cyanophényl)méthyl]-N-méthylpentanamide**

On prépare une solution de 30,14 g (0,206 M) de 4-[(méthylamino)méthyl]-benzonitrile dans 500 ml de dichlorométhane et on ajoute 22,9 g (0,227 M) de triéthylamine. Le mélange est refroidi à l'aide d'un bain de glace et on ajoute goutte à goutte une solution de 41,1 g (0,206 M) de chlorure de 5-bromopentanoyle dans 200 ml de dichlorométhane. Le mélange réactionnel est maintenu sous agitation à température ambiante pendant 5 heures puis hydrolysé sur 300 ml d'acide chlorhydrique 1N froid. La phase organique est séparée, lavée à l'eau, puis avec une solution de bicarbonate de sodium, puis à l'eau et séchée sur sulfate de magnésium. Après concentration sous pression réduite, on obtient le produit attendu sous forme d'une huile jaune (rendement = 97%).
RMN $^1$H (300 MHz, DMSO) δ : 7,80 (d, 2H) ; 7,39 (d, 2H) ; 4,62 (d, 2H) ; 3,50 (m, 2H) ; 2,81 et 2,50 (s, 3H) ; 2,41 (m, 2H) ; 1,80 (m, 2H) ; 1,64 (m, 2H).

**PREPARATION CLXXV**

**N-[(4-cyanophényl)methyl]-N-méthyl-5-[méthyl(phénylméthyl)amino]-pentanamide**

On prépare une solution de 62,2 g (0,201 M) du composé obtenu selon la préparation CLXXIV dans 500 ml d'acétonitrile et on ajoute à température ambiante 24,4 g (0,201 M) de N-méthyl-benzèneméthanamine, puis 27,8 g (0,201 M) de carbonate de potassium. Le mélange réactionnel est agité à 50˚C pendant 8 heures, puis pendant une nuit à température ambiante. Le solvant est ensuite éliminé sous pression réduite et le résidu d'évaporation est repris par 300 ml de dichlorométhane. Cette phase organique est lavée 2 fois à l'eau, puis séchée sur sulfate de magnésium et concentrée sous pression réduite. Le produit brut est purifié par chromatographie sur gel de silice en éluant à l'aide d'un mélange dichlorométhane/méthanol (95/5 ; v/v). On obtient ainsi 51,7 g du composé attendu sous forme d'une

huile jaune (rendement = 74%).
RMN $^1$H (250 MHz, DMSO) δ : 7,78 (m, 2H) ; 7,35 (m, 2H) ; 7,26 (m, 5H) ; 4,60 (d, 2H) ; 3,40 (d, 2H) ; 2,75 (d, 3H) ; 2,32 (m, 4H) ; 2,05 (d, 3H) ; 1,50 (m, 4H).

## PREPARATION CLXXVI

**Acide [5-[[(4-cyanophényl)méthyl]méthylamino]-5-oxopentyl]méthylcarbamique, phénylméthyl ester**

On dissout 51,67g (0,148 M) du composé obtenu selon la préparation CLXXV dans 400 ml de dichlorométhane et on ajoute progressivement une solution de 42 ml (0,296 M) de chloroformiate de benzyle dans 200 ml de dichlorométhane. Le mélange réactionnel est maintenu sous agitation pendant 16 heures à température ambiante, puis hydrolysé sur 300 ml d'acide chlorhydrique 0,5 N froid. La phase organique est séparée puis lavée à l'eau jusqu'à neutralité, séchée sur sulfate de magnésium et concentrée sous pression réduite. Ce produit brut est purifié par chromatographie sur gel de silice en éluant avec un mélange toluène/isopropanol (95/5 ; v/v). On obtient ainsi 51,6 g du produit attendu sous forme d'une huile jaune (rendement = 89%).
RMN $^1$H (250 MHz, DMSO) δ : 7,79 (m, 2H) ; 7,36 (m, 7H) ; 5,04 (d, 2H) ; 4,60 (d, 2H) ; 3,22 (m, 2H) ; 2,84 (m, 6H) ; 2,35 (m, 2H) ; 1,47 (m, 4H).
En opérant de façon analogue aux préparations II à IV, au départ du composé obtenu selon la préparation CLXXVI, on obtient les composés suivants :

## PREPARATION CLXXVII

**Acide [5-[[[4-(4,5-dihydro-1*H*-imidazol-2-yl)phényl]méthyl]méthylamino]-5-oxopentyl]méthylcarbamique, 1,1-diméthyléthyl ester**

(huile jaune, rendement = 94%).
RMN $^1$H (250 MHz, DMSO) δ : 7,78 (m, 2H) ; 7,30 (m, 5H) ; 7,22 (d, 2H) ; 6,83 (s large, 1H) ; 5,04 (d, 2H) ; 4,50 (d, 2H) ; 3,64 (m, 4H) ; 3,26 (m, 2H) ; 2,82 (m, 6H) ; 2,38 (m, 2H) ; 1,48 (m, 4H).

## PREPARATION CLXXVIII

**Acide 4,5-dihydro-2-[4-[[méthyl[5-[méthyl[(phénylméthoxy)carbonyl]amino]-1-oxopentyl]amino]méthyl]phényl]-1*H*-imidazole-1-carboxylique, 1,1-diméthyléthyl ester**

(huile incolore, rendement = 95%).

## PREPARATION CLXXIX

**Acide 4,5-dihydro-2-[4-[[méthyl[5-(méthylamino)-1-oxopentyl]amino] méthyl] phényl]-1*H*-imidazole-1-carboxylique, 1,1-diméthyléthyl ester**

(huile jaune, rendement = 90%).
RMN $^1$H (250 MHz, DMSO) δ : 7,43 (m, 2H); 7,20 (m, 2H) ; 4,56 (d, 2H) ; 3,84 (m, 4H) ; 2,87 (d, 3H) ; 2,42 (m, 4H) ; 2,29 (d, 3H) ; 1,49 (m, 4H) ; 1,18 (s, 9H).
En opérant de façon analogue à la préparation V, on obtient les composés suivants :

## PREPARATION CLXXX

**Acide 4,5-dihydro-2-[4-[[méthyl[5-[méthyl[[2-(trifluorométhyl)phényl]sulfonyl]amino]-1-oxopentyl]amino]méthyl]phényl]-1*H*-imidazole-1-carboxylique, 1,1-diméthyléthyl ester**

(huile incolore, rendement = 41%).
RMN $^1$H (250 MHz, DMSO) δ : 7,91 (m, 4H) ; 7,43 (dd, 2H) ; 7,21 (dd, 2H) ; 4,56 (d, 2H) ; 3,85 (m, 4H) ; 3,21 (m, 2H) ; 2,84 (m, 6H) ; 2,36 (m, 2H) ; 1,55 (m, 4H) ; 1,14 (s, 9H).

PREPARATION CLXXXI

**Acide 2-[4-[[[5-[[(2,4-dichloro-5-méthylphényl)sulfonyl]méthylamino]-1-oxopentyl] méthylamino]méthyl]phényl]-4,5-dihydro-1*H*-imidazole-1-carboxylique, 1,1-diméthyléthyl ester.**

(mousse blanche, rendement = 53%).
RMN $^1$H (250 MHz, DMSO) δ : 7,92 (d, 1H) ; 7,83 (d, 1H) ; 7,43 (dd, 2H) ; 7,20 (dd, 2H) ; 4,54 (d, 2H) ; 3,85 (m, 4H) ; 3,17 (m, 2H) ; 2,85 (m, 6H) ; 2,39 (s, 3H) ; 2,35 (m, 2H) ; 1,52 (m, 4H) ; 1,17 (s, 9H).

PREPARATION CLXXXII

**Acide 4,5-dihydro-2-[4-[[méthyl[5-[méthyl[(3-méthylphényl)sulfonyl]amino]-1-oxopentyl]amino]méthyl]phényl]-1*H*-imidazole-1-carboxylique, 1,1-diméthyléthyl ester**

(huile incolore, rendement = 51%).
RMN $^1$H (250 MHz, DMSO) δ : 7,52 (m, 3H) ; 7,42 (dd, 2H) ; 7,21 dd, 2H) ; 4,56 (d, 2H) ; 3,85 (m, 4H) ; 2,90 (m, 2H) ; 2,83 (d, 3H) ; 2,62 (d, 3H) ; 2,40 (s, 3H) ; 2,35 (m, 2H) ; 1,52 (m, 4H) ; 1,18 (s, 9H).

PREPARATION CLXXXIII

**Acide 4,5-dihydro-2-[4-[[[5-[[(4-méthoxy-2,6-diméthylphényl)sulfonyl]méthylamino]-1-oxopentyl]méthylamino]méthyl]phényl]-1*H*-imidazole-1-carboxylique, 1,1-diméthyléthyl ester**

(huile incolore, rendement = 20%).
RMN $^1$H (250 MHz, DMSO) δ: 7,42 (m, 2H) ; 7,19 (m, 2H) ; 6,80 (m, 2H) ; 4,53 (d, 2H) ; 3,85 (m, 4H) ; 3,78 (s, 3H) ; 3,02 (m, 2H) ; 2,82 (d, 3H) ; 2,58 (m, 6H) ; 2,26 (m, 2H) ; 1,49 (m, 4H) ; 1,17 (s, 9H).

PREPARATION CLXXXIV

**Acide 4,5-dihydro-2-[4-[[méthyl[5-[méthyl[(2,3,4-trichlorophényl)sulfonyl]amino]-1-oxopentyl]amino]méthyl]phényl]-1*H*-imidazole-1-carboxylique, 1,1-diméthyléthyl ester**

(solide blanc, rendement = 62%)
F = 60°C

PREPARATION CLXXXV

**Acide 4,5-dihydro-2-[4-[[méthyl[5-[méthyl[(2-nitrophényl)sulfonyl]amino]-1-oxopentyl]amino]méthyl]phényl]-1*H*-imidazole-1-carboxylique, 1,1-diméthyléthyl ester**

(solide blanc, rendement = 39%).
F = 60°C

PREPARATION CLXXXVI

**Acide 2-[4-[[[5-[[(2,4-dichlorophényl)sulfonyl]méthylamino]-1-oxopentyl] méthylamino]méthyl]phényl]-4,5-dihydro-1*H*-imidazole-1-carboxylique, 1,1-diméthyléthyl ester.**

(huile incolore, rendement = 46%).
RMN $^1$H (250 MHz, DMSO) δ : 7,94 (m, 2H) ; 7,63 (m, 1H) ; 7,43 (dd, 2H) ; 7,20 (dd, 2H) ; 4,55 (d, 2H) ; 3,84 (m, 4H) ; 3,17 (m, 2H) ; 2,83 (m, 6H) ; 2,37 (m, 2H) ; 1,49 (m, 4H) ; 1,17 (s, 9H).

PREPARATION CLXXXVII

**Acide 2-[4-[[[5-[[(2-chloro-3-méthylphényl)sulfonyl]méthylamino]-1-oxopentyl]méthylamino]méthyl]phényl]-4,5-dihydro-1*H*-imidazole-1-carboxylique, 1,1-diméthyléthyl ester.**

(huile incolore, rendement = 65%).

RMN $^1H$ (250 MHz, DMSO) δ : 7,82 (m, 1H) ; 7,64 (m, 1H) ; 7,43 (m, 3H) ; 7,20 (dd, 2H) ; 4,54 (d, 2H) ; 3,86 (m, 4H) ; 3,18 (m, 2H) ; 2,84 (m, 6H) ; 2,48 (s, 3H) ; 2,35 (m, 2H) ; 1,51 (m, 4H) ; 1,18 (s, 9H).

En opérant de façon analogue à l'exemple 1, on obtient les composés suivants :

**Exemple 69**

**N-[[4-(4,5-dihydro-1*H*-imidazol-2-yl)phényl]méthyl]-N-méthyl-5-[méthyl[[2-(trifluorométhyl)phényl]sulfonyl] amino]pentanamide, trifluoroacétate**

(solide amorphe, rendement = 99%).
RMN $^1H$ (300 MHz, DMSO) δ : 10,50 (d, 2H) ; 8,02 (m, 1H) ; 7,92 (m, 5H) ; 7,46 (d, 2H) ; 4,66 (d, 2H) ; 4,01 (s, 4H) ; 3,22 (, 2H) ; 2,90 (m, 6H) ; 2,40 (m, 2H) ; 1,55 (m, 4H).

**Exemple 70**

**5-[[(2,4-dichloro-5-méthylphényl)sulfonyl]méthylamino]-N-[[4-(4,5-dihydro-1*H*-imidazol-2-yl)phényl]méthyl]-N-méthyl-pentanamide, trifluoroacétate**

(solide blanc, rendement = 99%).
F= 50°C

**Exemple 71**

**N-[[4-(4,5-dihydro-1*H*-imidazol-2-yl)phényl]méthyl-N-méthyl-5-[méthyl[(3-méthylphényl)sulfonyl]amino]pentanamide, trifluoroacétate**

(solide blanc, rendement = 99%).
RMN $^1H$ (300 MHz, DMSO) δ : 10,48 (d, 2H) ; 7,90 (dd, 2H) ; 7,52 (m, 6H) ; 4,66 (d, 2H) ; 4,01 (s, 4H) ; 2,93 (m, 5H) ; 2,62 (d, 3H) ; 2,41 (m, 5H) ; 1,53 (m, 4H).

**Exemple 72**

**N-[[4-(4,5-dihydro-1*H*-imidazol-2-yl)phényl]méthyl]-5-[[(4-méthoxy-2,6-diméthylphényl)sulfonyl]méthylamino]-N-méthyl-pentanamide, trifluoroacétate**

**(solide blanc, rendement = 66%).**
F = 60°C

**Exemple 73**

**N-[[4-(4,5-dihydro-1*H*-imidazol-2-yl)phényl]méthyl-N-méthyl-5-[méthyl[(2,3,4-trichlorophényl)sulfonyl]amino] pentanamide, trifluoroacétate**

(solide blanc, rendement = 84%).
F = 70°C

**Exemple 74**

**N-[[4-(4,5-dihydro-1*H*-imidazol-2-yl)phényl]méthyl-N-méthyl-5-[méthyl[(2-nitrophényl)sulfonyl]amino]pentanamide, trifluoroacétate**

(solide blanc, rendement = 79%).
F = 60°C

**Exemple 75**

**5-[[(2,4-dichlorophényl)sulfonyl]méthylamino]-N-[[4-(4,5-dihydro-1*H*-imidazol-2-yl)phényl]méthyl]-N-méthyl-pentanamide, trifluoroacétate**

(solide blanc, rendement = 99%).
F = 60°C

**Exemple 76**

**5-[[(2-chloro-3-méthylphényl)sulfonyl]méthylamino]-N-[[4-(4,5-dihydro-1*H*-imidazol-2-yl)phényl]méthyl]-N-méthyl-pentanamide, trifluoroacétate**

(solide blanc, rendement = 93%).
F = 72°C

**PREPARATION CLXXXVIII**

**N-[(4-cyanophényl)méthyl]-5-[[(2,4-dichloro-3-méthylphényl)sulfonyl]méthylamino]-N-méthyl-pentanamide**

En opérant de façon analogue à la préparation VIII, au départ de 4-[(méthylamino)méthyl]benzonitrile, on obtient le composé attendu sous forme d'une huile incolore (rendement = 84%).
RMN $^1$H (250 MHz, DMSO) δ : 7,83 (m, 3H) ; 7,65 (m, 1H) ; 7,39 (d, 2H) ; 4,60 (d, 2H) ; 3,22 (m, 2H) ; 2,85 (m, 6H) ; 2,50 (s, 3H) ; 2,38 (m, 2H) ; 1,53 (m, 4H).

**Exemple 77**

**N-[[4-[amino(hydroxyimino)méthyl]phényl]méthyl]-5-[[(2,4-dichloro-3-méthyl) phényl]sulfonyl]méthylamino]-N-méthyl-pentanamide**

On prépare une solution de 2,51 g (5,2 mM) du composé obtenu selon la préparation CLXXXVIII dans 56 ml de diméthylsulfoxyde et on ajoute progressivement, à température ambiante, 1,90 g (27 mM) de chlorhydrate d'hydroxylamine et 7,5 ml (54 mM) de triéthylamine. Le mélange est maintenu sous agitation à température ambiante pendant 30 heures puis versé sur 250 ml d'eau glacée. Le mélange est extrait par l'acétate d'éthyle (2 fois) et les phases organiques rassemblées sont lavées à l'eau puis séchées sur sulfate de magnésium et concentrées sous pression réduite. On obtient ainsi 2,47 g de produit attendu sous forme de cristaux blancs (rendement = 92%).
F = 74°C

**PREPARATION CLXXXIX**

**N-[[4-[[(acétyloxy)imino]aminométhyl]phényl]méthyl]-5-[[(2,4-dichloro-3-méthylphényl)sulfonyl]méthylamino]-N-méthyl-pentanamide**

On prépare une solution de 1 g (1,95 mM) du composé obtenu selon l'exemple 77 dans 50 ml de dichlorométhane et on ajoute 0,59 g (5,82 mM) d'anhydride acétique. Le mélange réactionnel est agité à température ambiante pendant 20 heures puis concentré sous pression réduite. Le résidu est repris dans 100 ml d'acétate d'éthyle et la solution obtenue est agitée pendant 15 min avec une solution aqueuse à 5% de carbonate de sodium. La phase aqueuse est éliminée et la phase organique est lavée à l'eau, puis séchée sur sulfate de magnésium et concentrée sous pression réduite. On obtient ainsi 1 g du produit attendu sous forme d'une huile incolore (rendement = 92%).
RMN $^1$H (250 MHz, DMSO) δ : 7,82 (t, 1H) ; 7,65 (m, 3H) ; 7,26 (d, 2H) ; 6,76 (s large, 2H) ; 4,56 (d, 2H) ; 3,20 (m, 2H) ; 2,80 (m, 6H) ; 2,50 (s, 3H) ; 2,36 (m, 2H) ; 2,12 (s, 3H) ; 1,52 (m, 4H).

**Exemple 78**

**N-[[4-(aminoiminométhyl)phényl]méthyl]-5-[[(2,4-dichloro-3-méthylphényl) sulfonyl]méthylamino]-N-méthyl-pentanamide, trifluoroacétate**

On dissout 1g (1,79 mM) du composé obtenu selon la préparation CLXXXIX dans 50 ml de méthanol et on

ajoute 200 mg de charbon palladié à 5%. Le mélange est agité sous atmosphère d'hydrogène, à pression atmosphérique et à température ambiante pendant 6 heures. Le mélange est ensuite filtré et le filtrat est concentré sous pression réduite. Le produit brut est repris à chaud avec 10 ml de dioxanne. Le produit cristallisé par refroidissement de la solution est séparé par filtration et séché sous vide. Le composé obtenu est ensuite purifié par chromatographie sur gel de silice RP 18 en utilisant comme phase mobile un mélange acétonitrile/eau/acide trifluoroacétique (50/49/1 ; v/v/v). Les fractions pures sont concentrées sous pression réduite, reprises avec de l'éthanol, à nouveau concentrées sous pression réduite puis reprises en solution dans l'eau et lyophilisées. On obtient ainsi le produit attendu avec un rendement de 20%.
RMN $^1H$ (300 MHz, DMSO) $\delta$ : 9,28 (m, 2H) ; 9,10 (m, 2H) ; 7,79 (m, 3H) ; 7,64 (m, 1H) ; 7,42 (d, 2H) ; 4,63 (d, 2H) ; 3,20 (m, 2H) ; 2,85 (m, 6H) ; 2,30 (m, 2H) ; 1,51 (m, 4H).

## Exemple 79

**N-[[4-(4,5-dihydro-1*H*-imidazol-2-yl)phényl]méthyl]-2-[2-[[(4-méthoxy-2,6-diméthylphényl)sulfonyl]méthylamino]éthoxy]-N-méthyl-acétamide, fumarate**

On prépare une solution de 1 g du composé obtenu selon l'exemple 49 dans 20 ml de méthanol et on ajoute 2 g de résine IRA 400 basique. Le mélange est agité pendant 10 min puis la résine est séparée par filtration et le filtrat est concentré sous pression réduite. On obtient ainsi 0,68 g d'huile que l'on reprend dans 2 ml de méthanol. On ajoute ensuite 157 mg d'acide fumarique et on agite le mélange jusqu'à dissolution. On ajoute ensuite 20 ml d'éther éthylique. Il se forme une huile que l'on sépare. Cette huile est reprise dans 10 ml d'eau, la solution obtenue est filtrée puis lyophilisée. On obtient ainsi 0,78 g du composé attendu sous forme d'un solide fin blanc (rendement = 76%).
F = 88°C

## Exemple 80

**N-[[4-(4,5-dihydro-1-méthyl-1*H*-imidazol-2-yl)phényl]méthyl]-N-méthyl-4-[méthyl[[2-(trifluorométhyl)phényl] sulfonyl] amino]-(2E)-2-butènamide**

En opérant de façon analogue à la preparation VIII, au départ de l'acide obtenu selon la préparation XCIV et de l'amine obtenue selon la préparation CXVI, on obtient le produit attendu sous forme d'une huile incolore (rendement = 48 %).
RMN $^1H$ (300 MHz, DMSO) $\delta$ : 8,04 (m, 2H) ; 7,90 (m, 2H) ; 7,52 (t, 2H) ; 7,23 (m, 2H) ; 6,60 (m, 2H) ; 4,66 et 4,59 (2s, 2H) ; 4,09 (d, 1H) ; 4,02 (m, 1H) ; 3,70 (t, 2H) ; 3,36 (t, 2H) ; 3,06 et 3,03 (2s, 3H) ; 2,99 et 2,72 (2s, 3H) ; 2,68 (s, 3H).

## Exemple 81

**N-[[4-(4,5-dihydro-1-méthyl-1*H*-imidazol-2-yl)phényl]méthyl]-N-méthyl-4-[méthyl[[2-(trifluorométhyl)phényl] sulfonyl]amino]-(2E)-2-butènamide, fumarate**

En opérant de façon analogue à l'exemple 79, au départ du composé obtenu selon l'exemple 80, on obtient le produit attendu sous forme d'un solide blanc (rendement = 89 %).
F = 76-78 °C

## PREPARATION XCC

**Acide 2-[4-[[[(2E)-4-[[(2,3-dichlorophényl)sulfonyl]méthylamino]-1-oxo-2-buténoyl]méthylamino]méthyl]-3-fluorophényl]-4,5-dihydro-1*H*-imidazole-1-carboxylique, 1,1-diméthyléthyl ester**

En opérant de façon analogue à la préparation VIII, au départ de l'acide obtenu selon la préparation XXXVII et de l'amine obtenue selon la préparation CXL, on obtient le produit attendu sous forme d'une huile jaune (rendement = 47 %).
RMN $^1H$ (300 MHz, DMSO) $\delta$ : 7,99 (m, 2H) ; 7,58 (m, 1H) ; 7,26 (m, 3H) ; 6,59 (m, 2H) ; 4,65 (d, 2H) ; 4,03 (m, 2H) ; 3,85 (m, 4H) ; 3,17 et 2,86 (2s, 3H) ; 3,00 (s, 3H) ; 1,20 (s, 9H).

## Exemple 82

**4-[[(2,3-dichlorophényl)sulfonyl]méthylamino]-N-[[4-(4,5-dihydro-1*H*-imidazol-2-yl)-2-fluorophényl]méthyl]-N-méthyl-(2E)-2-butènamide, trifluoroacétate**

En opérant de façon analogue à l'exemple 1, au départ du composé obtenu selon la préparation XCC, on obtient le produit attendu sous forme d'un solide blanc écru (rendement = 79 %).
F=80°C

## PREPARATION XCCI

**Acide 2-[4-[[[(2E)-4-[[(2-chlorophényl)sulfonyl]méthylamino]-1-oxo-2-buténydl]méthylamino]méthyl]-3-fluorophényl]-4,5-dihydro-1*H*-imidazole-1-carboxylique, 1,1-diméthyléthyl ester**

En opérant de façon analogue à la préparation XCC, au départ de l'acide obtenu selon la préparation XXXIII, on obtient le produit attendu sous forme d'une huile incolore (rendement = 69 %).
RMN $^1$H (250 MHz, DMSO) δ : 8,00 (m, 1H) ; 7,66 (m, 2H) ; 7,56 (m, 1H) ; 7,26 (m, 3H) ; 6,59 (m, 2H) ; 4,65 (d, 2H) ; 4,05 (d, 1H) ; 3,98 (d, 1H) ; 3,86 (m, 4H) ; 3,00 et 2,72 (2s, 3H) ; 2,83 (d, 3H) ; 1,20 (s, 9H).

## Exemple 83

**4-[[(2-chlorophényl)sulfonyl]méthylamino]-N-[[4-(4,5-dihydro-1*H*-imidazol-2-yl)-2-fluorophényl]méthyl]-N-méthyl-(2E)-2-butènamide, trifluoroacétate**

En opérant de façon analogue à l'exemple 1, au départ du composé obtenu selon la préparation XCCI, on obtient le produit attendu sous forme d'un solide blanc écru (rendement = 62 %).
F=75°C

## PREPARATION XCCII

**4-méthoxy-N,2,6-triméthylbenzènesulfonamide**

En opérant de façon analogue à la préparation V, au départ de chlorure de 4-méthoxy-2,6-diméthylbenzènesulfonyle, on obtient le composé attendu sous forme d'un solide blanc (rendement = 96 %).
F = 131° C

## PREPARATION XCCIII

**Acide 3-[[(4-méthoxy-2,6-diméthylphényl)sulfonyl]méthylamino]propanoïque, éthyl ester**

En opérant de façon analogue à la préparation VI, au départ du composé obtenu selon la préparation XCCII et de 3-bromopropanoate d'éthyle, on obtient le produit attendu sous forme d'une huile jaune (rendement = 97 %).
RMN $^1$H (250 MHz, $CDCl_3$) δ : 6,63 (s, 2H) ; 4,09 (q, 2H) ; 3,81 (s, 3H) ; 3,45 (t, 2H) ; 2,74 (s, 3H) ; 2,62 (m, 2H) ; 2,60 (s, 6H) ; 1,23 (t, 3H).

## PREPARATION XCCIV

**Acide 3-[[(4-méthoxy-2,6-diméthylphényl)sulfonyl]méthylamino]propanoique**

En opérant de façon analogue à la préparation VII, au départ du composé obtenu selon la préparation XCCIII, on obtient l'acide attendu sous forme d'un solide blanc (rendement = 97 %).
F=103°C

**PREPARATION XCCV**

**Acide 4,5-dihydro-2-[4-[[[3-[[(4-méthoxy-2,6-diméthylphényl)sulfonyl]méthylamino]-1-oxopropyl]méthylamino]méthyl]phényl]-1*H*-imidazole-1-carboxylique 1,1-diméthyléthyl ester**

En opérant de façon analogue à la préparation VIII, au départ de l'acide obtenu selon la préparation XCCIV et de l'amine obtenue selon la préparation IV, on obtient le produit attendu sous forme d'une huile incolore (rendement = 61 %).
RMN $^1$H (250 MHz, $CDCl_3$) δ : 7,52 (m, 2H) ; 7,27 (m, 2H) ; 6,66 (d, 2H) ; 4,57 (d, 2H) ; 4,00 (m, 4H) ; 3,85 (d, 3H) ; 3,60 (m, 2H) ; 2,90 (d, 3H) ; 2,78 (d, 3H) ; 2,77 (m, 2H) ; 2,62 (d, 6H) ; 1,28 (d, 9H).

**Exemple 84**

**N-[[4-(4,5-dihydro-1*H*-imidazol-2-yl)phényl]méthyl]-3-[[(4-méthoxy-2,6-diméthylphényl)sulfonyl]méthylamino]-N-méthyl-propanamide, trifluoroacétate**

En opérant de façon analogue à l'exemple 1, au départ du composé obtenu selon la préparation XCCV, on obtient le produit attendu sous forme d'un solide blanc (rendement = 99 %).
F=59°C

En opérant de façon analogue aux préparations CXXXVI, I, II, III et IV, au départ du 4-(bromométhyl)-3-chlorobenzonitrile, on obtient respectivement les 5 composés suivants :

**PREPARATION XCCVI**

**4-cyano-2-chloro-N-méthylbenzèneméthanamine**

(Huile incolore, rendement = 89 %).
RMN $^1$H (300 MHz, DMSO) δ : 7,99 (d, 1H) ; 7,83 (dd, 1H) ; 7,71 (d, 1H) ; 3,76 (s, 2H) ; 2,29 (s, 3H).

**PREPARATION XCCVII**

**Acide [(2-chloro-4-cyanophényl)méthyl]méthylcarbamique, phénylméthyl ester**

(Huile incolore, rendement = 99%).
RMN $^1$H (250 MHz, DMSO) δ : 8,00 (s, 1H) ; 7,80 (m, 1H) ; 7,25 (m, 6H) ; 5,10 (d, 2H) ; 4,59 (s, 2H) ; 2,94 (s, 3H).

**PREPARATION XCCVIII**

**Acide [[2-chloro-4-(4,5-dihydro-1*H*-imidazol-2-yl)phényl]méthyl]méthylcarbamique, phénylméthyl ester**

(Huile incolore, rendement = 99%).

**PREPARATION ICC**

**Acide 2-[3-chloro-4-[[méthyl[(phénylméthoxy)carbonyl]amino]méthyl]phényl]-4,5-dihydro-1*H*-imidazole-1-carboxylique, 1,1-diméthyléthyl ester**

(Huile jaune, rendement = 94%).
RMN $^1$H (250 MHz, DMSO) δ : 7,51 (d, 1H) ; 7,41 (m, 7H) ; 5,10 (d, 2H) ; 4,57 (s, 2H) ; 3,85 (m, 4H) ; 2,89 (s, 3H) ; 1,14 (s, 9H).

**PREPARATION CC**

**Acide 2-[3-chloro-4-[(méthylamino)méthyl]phényl]-4,5-dihydro-1*H*-imidazole-1-carboxylique,1,1-diméthyléthyl ester**

(Huile incolore, rendement = 11%).
RMN $^1$H (250 MHz, DMSO) δ : 7,51 (d, 1H) ; 7,43 (d, 1H) ; 7,40 (dd, 1H) ; 3,85 (m, 4H) ; 3,74 (s, 2H) ; 2,31 (s, 3H) ;

1,19 (s, 9H).

En opérant de façon analogue aux préparations VI et VII, au départ de la sulfonamide obtenue selon la préparation XCCII et de 5-bromopentanoate d'éthyle, on obtient les composés suivants :

**PREPARATION CCI**

**Acide 5-[[(4-méthoxy-2,6-diméthylphényl)sulfonyl]méthylamino]pentanoïque, éthyl ester**

(Huile incolore, rendement = 58 %).
RMN $^1H$ (300 MHz, $CDCl_3$) δ : 6,63 (s, 2H) ; 4,11 (q, 2H) ; 3,81 (s, 3H) ; 3,10 (t, 2H) ; 2,70 (s, 3H) ; 2,61 (s, 6H) ; 2,24 (t, 2H) ; 1,55 (m, 4H) ; 1,25 (t, 3H).

**PREPARATION CCII**

**Acide 5-[[(4-méthoxy-2,6-diméthylphényl)sulfonyl]méthylamino]pentanoïque**

(Solide pâteux blanc, rendement = 99 %).
F = 80-84 °C

**PREPARATION CCIII**

**Acide 2-[3-chloro-4-[[[5-[[(4-méthoxy-2,6-diméthylphényl)sulfonyl]méthylamino]-1-oxopentyl]méthylamino]méthyl]phényl]-4,5-dihydro-1*H*-imidazole-1-carboxylique, 1,1-diméthyléthyl ester**

En opérant de façon analogue à la préparation VIII, au départ de l'acide obtenu selon la préparation CCII et de l'amine obtenue selon la préparation CC, on obtient le produit attendu sous forme d'une huile que l'on traite sans purification complémentaire.

**Exemple 85**

**N-[[2-chloro-4-(4,5-dihydro-1*H*-imidazol-2-yl)phényl]méthyl]-5-[[(4-méthoxy-2,6-diméthylphényl)sulfonyl]méthylamino]-N-méthyl-pentanamide**

On ajoute 234 mg (3,7 mM) du composé obtenu selon la préparation CCIII à un mélange de 1,5 ml d'acide trifluoroacétique dans 2,5 ml de DCM, on agite ce milieu réactionnel pendant une heure à température ambiante puis on concentre ce mélange sous pression réduite. Le résidu est ensuite purifié par chromatographie sur gel de silice en éluant à l'aide d'un mélange DC/méthanol/ammoniaque (120/10/0,02). On obtient ainsi 186 mg de produit attendu sous forme d'une huile incolore (rendement : 94 %).
RMN $^1H$ (300 MHz, DMSO) δ : 7,90 (d, 1H) ; 7,75 (dd, 1H) ; 7,18 (s large, 1H) ; 7,15 (t, 1H) ; 6,79 (d, 2H) ; 4,60 (d, 2H) ; 3,79 (s, 3H) ; 3,61 (s, 4H) ; 3,01 (t, 2H) ; 2,94 et 2,82 (2s, 3H) ; 2,63 et 2,59 (2s, 3H) ; 2,51 (s, 6H) ; 2,37 et 2,17 (2t, 2H) ; 1,48 (m, 4H).

**Exemple 86**

**N-[[2-chloro-4-(4,5-dihydro-1*H*-imidazol-2-yl)phényl]méthyl]-5-[[(4-méthoxy-2,6-diméthylphényl)sulfonyl]méthylamino]-N-méthyl-pentanamide, fumarate**

En opérant de façon analogue à l'exemple 79, au depart du compose obtenu selon l'exemple 85, on obtient le sel attendu sous forme d'un solide blanc (rendement = 98 %).
F = 60 °C

**PREPARATION CCIV**

**Acide 2-[2-[méthyl[[2-(trifluorométhyl)phényl]sulfonyl]amino]éthoxy]acétique, 1,1-diméthyléthyl ester**

En opérant de façon analogue à la préparation VI, au départ du composé obtenu selon la préparation LXXXIX et de (2-iodoéthoxy)acétate de t-butyle, on obtient le produit attendu sous forme d'une huile incolore (rendement = 72 %).
RMN $^1H$ (250 MHz, DMSO) δ : 8,02 (m, 2H) ; 7,88 (m, 2H) ; 3,97 (s, 2H) ; 3,63 (t, 2H) ; 3,45 (t, 2H) ; 2,95 (s, 3H) ; 1,40

(s, 9H).

## PREPARATION CCV

### Acide 2-[2-[méthyl[[2-(trifluorométhyl)phényl]sulfonyl]amino]éthoxy]acétique

En opérant de façon analogue à la préparation VII, au départ du composé obtenu selon la préparation CCIV, on obtient le produit attendu sous forme d'une huile incolore (rendement = 94 %).
RMN $^1H$ (250 MHz, DMSO) δ : 8,03 (m, 2H) ; 7,86 (m, 2H) ; 4,01 (s, 2H) ; 3,65 (t, 2H) ; 3,45 (t, 2H) ; 2,95 (s, 3H).

## Exemple 87

### N-[[4-(4,5-dihydro-1-méthyl-1*H*-imidazol-2-yl)phényl]méthyl]-N-méthyl-2-[2-[méthyl[[2-(trifluorométhyl)phényl]sulfonyl]amino]éthoxy]acétamide

En opérant de façon analogue à la préparation VIII, au depart de l'acide obtenu selon la préparation CCV et de l'amine obtenue selon la préparation CXVI, on obtient le produit attendu sous forme d'une huile jaune (rendement = 62 %).
RMN $^1H$ (300 MHz, DMSO) δ : 8,04 (m, 2H) ; 7,87 (m, 2H) ; 7,50 (m, 2H) ; 7,23 (d, 2H) ; 4,54 (s, 2H) ; 4,25 (d, 2H) ; 3,67 (m, 4H) ; 3,40 (m, 4H) ; 2,95 (d, 3H) ; 2,93 (d, 3H) ; 2,80 (s, 3H).

## Exemple 88

### N-[[4-(4,5-dihydro-1-méthyl-1*H*-imidazol-2-yl)phényl]méthyl]-N-méthyl-2-[2-[méthyl[[2-(trifluorométhyl)phényl]sulfonyl]amino]éthoxy]acétamide, fumarate

En opérant de façon analogue à l'exemple 79, au départ du composé obtenu selon l'exemple 87, on obtient le produit attendu sous forme d'un solide blanc (rendement = 100 %).
F = 62 °C

## PREPARATION CCVI

### Acide 2-[3-chloro-4-[[[[2-[[(2-chlorophényl)sulfonyl]méthylamino]éthoxy]acétyl]méthylamino]méthyl]phényl]-4,5-dihydro-1*H*-imidazole-1-carboxylique, 1,1-diméthyléthyl ester

En opérant de façon analogue à la préparation VIII, au départ de l'acide obtenu selon la préparation CXXXI et de l'amine obtenue selon la préparation CC, on obtient le produit attendu sous forme d'une huile incolore (rendement = 41 %).
RMN $^1H$ (250 MHz, DMSO) δ : 7,99 (m, 1H) ; 7,62 (m, 2H) ; 7,52 (m, 3H) ; 7,23 (m, 1H) ; 4,58 (s, 2H) ; 4,28 et 4,12 (2s, 2H) ; 3,85 (s, 4H) ; 3,58 (m, 2H) ; 3,42 (m, 2H) ; 2,91 et 2,86 (2s, 3H) ; 2,90 et 2,83 (2s, 3H) ; 1,20 (s, 9H).

## Exemple 89

### N-[[2-chloro-4-(4,5-dihydro-1*H*-imidazol-2-yl)phényl]méthyl]-2-[2-[[(2-chlorophényl)sulfonyl]méthylamino]éthoxy]-N-méthyl-acétamide, trifluoroacétate

En opérant de façon analogue à l'exemple 1, au départ du produit obtenu selon la préparation CCVI, on obtient le produit attendu sous forme d'un solide rose (rendement = 99 %).
RMN $^1H$ (250 MHz, DMSO) δ : 8,08 (m, 1H) ; 7,99 (m, 2H) ; 7,68 (m, 2H) ; 7,57 (m, 1H) ; 7,53 (d, 1H) ; 4,67 (d, 2H) ; 4,32 et 4,12 (2s, 2H) ; 4,02 (s, 4H) ; 3,67 et 3,54 (2t, 2H) ; 3,46 et 3,26 (2t, 2H) ; 2,98 (s, 3H) ; 2,90 (s, 3H).

## PREPARATION CCVII

### 4-méthoxy-2,6,N-triméthyl-N-(2-hydroxyéthyl)benzènesulfonamide

En opérant de façon analogue à la préparation V, au départ du chlorure de 2,6-diméthyl-4-méthoxybenzènesulfonyle et de 2-(N-méthylamino)éthanol, on obtient la sulfonamide attendue sous forme d'une huile incolore (rendement = 95 %).

RMN $^1H$ (300 MHz, DMSO) δ: 6,80 (s, 2H) ; 4,70 (t, 1H) ; 3,80 (s, 3H) ; 3,47 (t, 2H) ; 3,09 (t, 2H) ; 2,69 (s, 3H) ; 2,53 (s, 6H).
En opérant de façon analogue aux préparations CXXII et LXXVIII, au départ du composé obtenu selon la préparation CCVII, on obtient les 2 composés suivants :

## PREPARATION CCVIII

**Acide [2-[[(4-méthoxy-2,6-diméhylphényl)sufonyl]méthylamino]éthoxy]acétique, 1,1-diméthyléthyl ester**

(Huile incolore ; rendement = 94 %)
RMN 1H (250 MHz, DMSO) δ : 6,80 (s, 2H) ; 3,89 (s, 2H) ; 3,79 (s, 3H) ; 3,55 (t, 2H) ; 3,21 (t, 2H) ; 2,70 (s, 3H) ; 2,50 (s, 6H) ; 1,41 (s, 9H).

## PREPARATION CCIX

**Acide [2-[[(4-méthoxy-2,6-diméthylphényl)sulfonyl]méthylamino]éthoxy]acétique**

(Solide blanc; rendement = 68 %)
F = 85 °C

## PREPARATION CCX

**Acide 2-[3-chloro-4-[[[[2-[[(4-méthoxy-2,6-diméthylphényl)sulfonyl]méthylamino]éthoxy] acétyl]méthylamino] méthyl]phényl]-4,5-dihydro-1*H*-imidazole-1-carboxylique, 1,1-diméthyléthyl ester**

En opérant de façon analogue à la préparation VIII, au départ des composés obtenus selon les préparations CCIX et CC, on obtient le produit attendu sous forme d'une huile incolore (rendement = 57 %).
RMN $^1H$ (300 MHz, DMSO) δ : 7,55 (d, 1H) ; 7,41 (dd, 1H) ; 7,21 (m, 1H) ; 6,80 (d, 2H) ; 4,57 (s, 2H) ; 4,22 et 4,08 (2s, 2H) ; 3,85 (m, 4H) ; 3,80 (d, 3H) ; 3,59 et 3,52 (2t, 2H) ; 3,27 et 3,16 (2t, 2H) ; 2,88 et 2,79 (2s, 3H) ; 2,71 et 2,66 (2s, 3H) ; 2,53 (s, 6H) ; 1,20 (s, 9H).

## Exemple 90

**N-[[2-chloro-4-(4,5-dihydro-1*H*-imidazol-2-yl)phényl]méthyl]-2-[2-[[(4-méthoxy-2,6-diméthylphényl)sulfonyl] méthylamino]éthoxy]-N-méthyl-acétamide, trifluoroacétate**

En opérant de façon analogue à l'exemple 1, au départ du composé obtenu selon la préparation CCX, on obtient le produit attendu sous forme d'un solide rose (rendement = 94 %).
F = 65 °C

## PREPARATION CCXI

**Acide [(4-cyanophényl)méthyl][méthyl]carbamique,1,1-diméthyléthyl ester**

En opérant de façon analogue à la préparation III, au départ de [(4-cyanophényl)méthyl] méthanamine, on obtient le produit attendu sous forme d'une huile jaune (rendement = 92 %). RMN $^1H$ (300 MHz, DMSO) δ : 7,83 (d, 2H) ; 7,40 (d, 2H) ; 4,46 (s, 2H) ; 2,79 (s, 3H) ; 1,38 (m, 9H).

## PREPARATION CCXII

**Acide [[4-[4,5-dihydro-1-(1-méthyléthyl)-1*H*-imidazol-2-yl]phényl]méthyl] méthylcarbamique,1,1-diméthyléthyl ester**

En opérant de façon analogue à la préparation II, au départ du composé obtenu selon la préparation CCXI et de N-(1-méthyléthyl)-éthylènediamine, on obtient le produit attendu sous forme d'une huile épaisse jaune (rendement = 99 %).
RMN $^1H$ (300 MHz, DMSO) δ : 7,47 (d, 2H) ; 7,30 (d, 2H) ; 4,41 (s, 2H) ; 3,75 (m, 3H) ; 3,48 (t, 2H) ; 2,79 (s, 3H) ; 1,38 (s, 9H) ; 1,03 (d, 6H).

**PREPARATION CCXIII**

**1-(1-méthyléthyl)-2-[4-(méthylaminométhyl)phényl]-4,5-dihydro-1*H*-imidazole**

En opérant de façon analogue à l'exemple 1, au départ du composé obtenu selon la préparation CCXII, on obtient le produit attendu sous forme d'une pâte jaune (rendement = 99 %).
RMN $^1$H (250 MHz, DMSO) δ : 10,5 (s, 1H) ; 9,15 (s large, 2H) ; 7,70 (s, 4H) ; 4,27 (t, 2H) ; 4,01 (m, 4H) ; 3,85 (m, 1H) ; 2,61 (t, 3H) ; 1,25 (d, 6H).

**Exemple 91**

**N-[[4-(4,5-dihydro-1-(1-méthyléthyl)-1*H*-imidazol-2-yl)phényl]méthyl]-2-[2-[[(4-méthoxy-2,6-diméthylphényl) sulfonyl]méthylamino]éthoxy]-N-méthyl-acétamide**

En opérant de façon analogue à la préparation VIII, au départ de l'acide obtenu selon la préparation CCIX et de l'amine obtenue selon la préparation CCXIII, on obtient le produit attendu sous forme d'une huile incolore (rendement = 36 %).
RMN $^1$H (250 MHz, DMSO) δ : 7,40 (m, 2H) ; 7,24 (m, 2H) ; 6,80 (s, 2H) ; 4,51 (s, 2H) ; 4,18 et 4,12 (2s, 2H) ; 3,80 (s, 3H) ; 3,66 (m, 5H) ; 3,30 (m, 4H) ; 2,80 et 2,77 (2s, 3H) ; 2,70 et 2,66 (2s, 3H) ; 2,53 (s, 6H) ; 0,96 (d, 6H).

**Exemple 92**

**N-[[4-(4,5-dihydro-1-(1-méthyléthyl)-1*H*-imidazol-2-yl)phényl]méthyl]-2-[2-[[(4-méthoxy-2,6-diméthylphényl) sulfonyl]méthylamino]éthoxy]-N-méthyl-acétamide, fumarate**

En opérant de façon analogue à l'exemple 79, au départ du composé obtenu selon l'exemple 91, on obtient le produit attendu sous forme d'un solide crème (rendement = 99 %).
F=106˚C

En opérant de façon analogue aux préparations CCXI à CCXIII, on obtient les 3 composés suivants :

**PREPARATION CCXIV**

**Acide [(2-chloro-4-cyanophényl)méthyl]méthylcarbamique, 1,1-diméthyléthyl ester**

(Solide blanc ; rendement = 72 %).
F = 86-87 ˚C

**PREPARATION CCXV**

**Acide [[2-chloro-4-(4,5-dihydro-1-méthyl-1*H*-imidazol-2-yl)phényl]méthyl] méthylcarbamique,1,1-diméthyléthyl ester**

(Huile jaune ; rendement = 66 %).
RMN $^1$H (300 MHz, DMSO) δ : 7,56 (s, 1H) ; 7,51 (d, 1H) ; 7,22 (d, 1H) ; 4,49 (s, 2H) ; 3,73 (t, 2H) ; 3,38 (t, 2H) ; 2,84 (s, 3H) ; 2,71 (s, 3H) ; 1,40 (d, 9H).

**PREPARATION CCXVI**

**1-méthyl-2-[3-chloro-4-(méthylaminométhyl)phényl]-4,5-dihydro-1*H*-imidazole**

(Solide beige ; rendement = 92 %)
F = 94-98 ˚C

**Exemple 93**

**N-[[2-chloro-4-(4,5-dihydro-1-méthyl-1*H*-imidazol-2-yl)phényl]méthyl]-2-[2-[[(4-méthoxy-2,6-diméthylphényl) sulfonyl]méthylamino]éthoxy]-N-méthyl-acétamide**

En opérant de façon analogue à la préparation VIII, au départ des composés obtenus selon les préparations CCIX et CCXVI, on obtient le produit attendu sous forme d'une pâte blanche (rendement = 53 %).
RMN $^1$H (300 MHz, DMSO) δ : 7,60 (s, 1H) ; 7,50 (m, 1H) ; 7,25 (d, 1H) ; 6,80 (d, 2H) ; 4,57 (s, 2H) ; 4,27 et 4,08 (2s, 2H) ; 3,78 (s, 3H) ; 3,70 (t, 2H) ; 3,60 (t, 2H) ; 3,40 (t, 2H) ; 3,25 (t, 2H) ; 2,92 et 2,81 (2s, 3H) ; 2,74 (s, 3H) ; 2,71 et 2,64 (2s, 3H) ; 2,51 (d, 6H).

**Exemple 94**

**N-[[2-chloro-4-(4,5-dihydro-1-méthyl-1*H*-imidazol-2-yl)phényl]méthyl]-2-[2-[[(4-méthoxy-2,6-diméthylphényl) sulfonyl]méthylamino]éthoxy]-N-méthyl-acétamide, fumarate**

En opérant de façon analogue à l'exemple 79, au départ du composé obtenu selon l'exemple 93, on obtient le produit attendu sous forme d'un solide blanc (rendement = 99%).
F = 65-68 °C

**Exemple 95**

**N-[[2-chloro-4-(4,5-dihydro-1-méthyl-1*H*-imidazol-2-yl)phényl]méthyl]-N-méthyl-2-[2-[méthyl[[2-(trifluorométhyl)phényl]sulfonyl]amino]éthoxy]acétamide**

En opérant de façon analogue à la préparation VIII, au départ des composés obtenus selon les préparations CCV et CCXVI, on obtient le produit attendu sous forme d'une huile blanche (rendement = 41 %).
RMN $^1$H (300 MHz, DMSO) δ : 8,01 (m, 2H) ; 7,86 (m, 2H) ; 7,64 (s, 1H) ; 7,49 (m, 1H) ; 7,30 (m, 1H) ; 4,60 (s, 2H) ; 4,33 et 4,16 (2s, 2H) ; 3,70 (m, 4H) ; 3,49 (m, 4H) ; 2,97 et 2,89 (2s, 3H) ; 2,96 et 2,84 (2s, 3H) ; 2,79 et 2,76 (2s, 3H).

**Exemple 96**

**N-[[2-chloro-4-(4,5-dihydro-1-méthyl-1*H*-imidazol-2-yl)phényl]méthyl]-N-méthyl-2-[2-[méthyl[[2-(trifluorométhyl)phényl]sulfonyl]amino]éthoxy]acétamide, fumarate**

En opérant de façon analogue à l'exemple 79, au départ du composé obtenu selon l'exemple 95, on obtient le produit attendu sous forme d'un solide blanc (rendement = 99 %). F = 55 °C.
En opérant de façon analogue aux préparations CCVII à CCIX, on obtient les 3 composés suivants :

**PREPARATION CCXVII**

**2-cyano-N-méthyl-N-(2-hydroxyéthyl)benzènesulfonamide**

(Huile blanche, rendement = 90 %).
RMN $^1$H (300 MHz, DMSO) δ : 7,93 (d, 1H) ; 7,88 (d, 1H) ; 7,85 (m, 2H) ; 4,80 (s, 1H) ; 3,50 (q, 2H) ; 3,24 (t, 2H) ; 2,87 (s, 3H).

**PREPARATION CCXVIII**

**Acide [2-[[(2-cyanophényl)sulfonyl]méthylamino]éthoxy]acétique, 1,1-diméthyléthyl ester**

(Huile jaune ; rendement = 87 %)
RMN $^1$H (300 MHz, DMSO) δ : 8,13 (d, 1H) ; 8,02 (d, 1H) ; 7,90 (m, 2H) ; 3,90 (s, 2H) ; 3,59 (t, 2H) ; 3,37 (t, 2H) ; 2,90 (s, 3H) ; 1,41 (s, 9H).

**PREPARATION CCXIX**

**Acide [2-[[(2-cyanophényl)sulfonyl]méthylamino]éthoxy]acétique**

(Solide blanc; rendement = 99 %)
RMN $^1$H (300 MHz, DMSO) δ : 8,13 (d, 1H) ; 8,02 (d, 1H) ; 7,80 (m, 2H) ; 3,93 (s, 2H) ; 3,60 (t, 2H) ; 3,77 (t, 2H) ; 2,90 (s, 3H).

**Exemple 97**

**2-[2-[[(2-cyanophényl)sulfonyl]méthylamino]éthoxy]-N-[[4-(4,5-dihydro-1-méthyl-1*H*-imidazol-2-yl)phényl] méthyl]-N-méthyl-acétamide**

En opérant de façon analogue à la préparation VIII, au départ des composés obtenus selon les préparations CXVI et CCXIX, on obtient le produit attendu sous forme d'une huile jaune (rendement = 57%).
RMN $^1$H (300 MHz, DMSO) δ : 8,11 (m, 2H) ; 7,88 (m, 2H) ; 7,50 (m, 2H) ; 7,28 (m, 2H) ; 4,51 (s, 2H) ; 4,20 et 4,13 (2s, 2H) ; 3,60 (m, 4H) ; 3,40 (m, 4H) ; 2,90 et 2,87 (2s, 3H) ; 2,85 et 2,78 (2s, 3H) ; 2,70 (s, 3H).

**Exemple 98**

**2-[2-[[(2-cyanophényl)sulfonyl]méthylamino]éthoxy]-N-[[4-(4,5-dihydro-1-méthyl-1*H*-imidazol-2-yl) phényl]méthyl]-N-méthyl-acétamide, fumarate**

En opérant de façon analogue à l'exemple 79, au départ du composé obtenu selon l'exemple 97, on obtient le produit attendu sous forme d'un solide blanc (rendement = 90 %).
F=59°C

En opérant de façon analogue aux préparations CCVII à CCIX, on obtient les 3 composés suivants :

**PREPARATION CCXX**

**2-chloro-4-méthoxy-N-méthyl-N-(2-hydroxyéthyl)benzènesulfonamide**

(Huile incolore, rendement = 99 %)
RMN $^1$H (250 MHz, DMSO) δ : 7,90 (d, 1H) ; 7,24 (d, 1H) ; 7,06 (dd, 1H) ; 4,76 (t, 1H) ; 3,86 (s, 3H) ; 3,52 (q, 2H) ; 3,19 (t, 2H) ; 2,84 (s, 3H).

**PREPARATION CCXXI**

**Acide [2-[[(2-chloro-4-méthoxyphényl)sulfonyl]méthylamino]éthoxy]acétique, 1,1-diméthyléthyl ester**

(Huile incolore ; rendement = 96 %)
RMN $^1$H (300 MHz, DMSO) δ : 7,90 (d, 1H) ; 7,24 (d, 1H) ; 7,06 (dd, 1H) ; 4,03 (s, 2H) ; 3,86 (s, 3H) ; 3,58 (t, 2H) ; 3,34 (t, 2H) ; 2,85 (s, 3H) ; 1,42 (s, 9H).

**PREPARATION CCXXII**

**Acide [2-[[(2-chloro-4-méthoxyphényl)sulfonyl]méthylamino]éthoxy]acétique**

(Huile incolore ; rendement = 99 %)
RMN $^1$H (300 MHz, DMSO) δ : 7,91 (d, 1H) ; 7,24 (d, 1H) ; 7,07 (dd, 1H) ; 3,99 (s, 2H) ; 3,86 (s, 3H) ; 3,60 (t, 2H) ; 3,34 (t, 2H) ; 2,84 (s, 3H).

En opérant de façon analogue aux exemples 97 et 98, au départ de d'acide obtenu selon la préparation CCXXII, on obtient les 2 composés suivants :

**Exemple 99**

**2-[2-[[(2-chloro-4-méthoxyphényl)sulfonyl]méthylamino]éthoxy]-N-[[4-(4,5-dihydro-1-méthyl-1*H*-imidazo1-2-yl)phényl]méthyl]-N-méthyl-acétamide**

(Huile incolore ; rendement = 27 %)
RMN $^1$H (300 MHz, DMSO) δ : 7,91 (d, 1H) ; 7,48 (m, 2H) ; 7,24 (m, 3H) ; 7,10 (dd, 1H) ; 4,52 (s, 2H) ; 4,23 et 4,18 (2s, 2H) ; 3,85 (s, 3H) ; 3,68 (m, 4H) ; 3,39 (m, 4H) ; 2,87 et 2,81 (2s, 3H) ; 2,84 et 2,80 (2s, 3H) ; 2,69 (s, 3H).

**Exemple 100**

**2-[2-[[(2-chloro-4-méthoxyphényl)sulfonyl]méthylamino]éthoxy]-N-[[4-(4,5-dihydro-1-méthyl-1*H*-imidazol-2-yl)phényl]méthyl]-N-méthyl-acétamide, fumarate**

(Solide blanc ; rendement = 87 %)
F=50°C

**PREPARATION CCXXIII**

**N-(2-cyanoéthyl)-N-méthyl-2-(trifluorométhyl)benzènesulfonamide**

En opérant de façon analogue à la préparation V, au départ du chlorure de 2-(trifluorométhyl)benzènesulfonyle et de 3-(méthylamino)propionitrile, on obtient le produit attendu sous forme d'une huile jaune (rendement = 98 %).
RMN$^1$H (250 MHz, DMSO) δ : 8,02 (m, 2H) ; 7,90 (m, 2H) ; 3,55 (t, 2H) ; 2,29 (s, 3H) ; 2,86 (t, 2H).

**PREPARATION CCXXIV**

**Acide 3-[méthyl[[(2-(trifluorométhyl)phényl]sulfonyl]amino]propanoïque**

On mélange 140 mg (0,48 mM) du composé obtenu selon la préparation CCXIII avec 3 ml d'acide chlorhydrique 10N et on agite ce mélange à doux reflux pendant 3 heures. Le mélange réactionnel est ensuite refroidi, dilué avec de l'eau, partiellement neutralisé jusqu'à pH 2 avec de la soude puis extrait par 25 ml d'acétate d'éthyle. La phase organique est séchée sur sulfate de magnésium puis concentrée sous pression réduite. On obtient ainsi l'acide attendu sous forme d'une huile incolore (rendement = 64 %).
RMN$^1$H (250 MHz, DMSO) δ : 8,01 (m, 2H) ; 7,91 (m, 2H) ; 3,46 (t, 2H) ; 2,89 (s, 3H) ; 2,50 (t, 2H),
**[0401]** En opérant de façon analogue à la préparation VIII et à l'exemple 1, au départ de l'acide obtenu selon la préparation CCXXIV, on obtient les 2 composés suivants :

**PREPARATION CCXXV**

**Acide 4,5-dihydro-2-[4-[[méthyl[3-[méthyl[[2-(trifluorométhyl)phényl)sulfonyl]amino]-1-oxopropyl]amino]méthyl]phényl]-1*H*-imidazole-1-carboxylique, 1,1-diméthyléthyl ester**

(Solide blanc ; rendement = 74 %).
F=50°C

**Exemple 101**

**N-[[4-(4,5-dihydro-1*H*-imidazol-2-yl)phényl]méthyl-N-méthyl-3-[méthyl[[2-(trifluorométhyl)phényl]sulfonyl] amino]propanamide, trifluoroacétate**

(Solide blanc, rendement = 81%).
F = 56°C

## PREPARATION CCXXVI

**N-[(4-cyanophényl)méthyl]-N-méthyl-2-[2-[méthyl[[2-(trifluorométhyl)phényl] sulfonyl]amino]éthoxy] acétamide**

En opérant de façon analogue à la préparation VIII, au départ de l'acide obtenu selon la préparation CCV et de 4-(méthylaminométhyl)benzonitrile, on obtient le produit attendu sous forme d'une huile jaune (rendement = 62 %).
RMN$^1$H (250 MHz, DMSO) δ : 8,04 (m, 2H) ; 7,87 (m, 4H) ; 7,43 (d, 2H) ; 4,59 (s, 2H) ; 4,28 et 4,17 (2s, 2H) ; 3,67 (m, 2H) ; 3,46 et 3,40 (2t, 2H) ; 2,95 et 2,90 (2s, 3H) ; 2,89 et 2,78 (2s, 3H).

## Exemple 102

**N-([4-(1-éthyl-4,5-dihydro-1*H*-imidazol-2-yl)phényl]méthyl]-N-méthyl-2-[2-[méthyl[[2-(trifluorométhyl)phényl] sulfonyl]amino]éthoxy]acétamide**

En opérant de façon analogue à la préparation II, au départ du composé obtenu selon la préparation CCXXVI et de N-éthyléthylènediamine, on obtient le produit attendu sous forme d'une huile incolore (rendement = 19 %).
RMN $^1$H (250 MHz, DMSO) δ : 8,01 (m, 2H) ; 7,87 (m, 2H) ; 7,45 (m, 2H) ; 7,28 (d, 2H) ; 4,53 (s, 2H) ; 4,27 et 4,20 (2s, 2H) ; 3,70 (m, 4H) ; 3,40 (m, 4H) ; 3,01 (q, 2H) ; 2,98 et 2,95 (2s, 3H) ; 2,93 et 2,79 (2s, 3H) ; 0,99 (t, 3H).

## Exemple 103

**N-[[4-[1-(1-méthyléthyl)-4,5-dihydro-1*H*-imidazol-2-yl]phényl]méthyl]-N-méthyl-2-[2-[méthyl[[2-(trifluorométhyl)phényl]sulfonyl]amino]éthoxy]acétamide**

En opérant de façon analogue à l'exemple 102, au départ de N-(1-méthyléthyl)éthylènediamine, on obtient le produit attendu sous forme d'une huile (rendement = 54 %).
RMN $^1$H (250 MHz, DMSO) δ 8,04 (m, 2H) ; 7,89 (m, 2H) ; 7,45 (m, 2H) ; 7,28 (d, 2H) ; 4,53 (s, 2H) ; 4,27 et 4,20 (2s, 2H) ; 3,67 (m, 5H) ; 3,48 (m, 2H) ; 3,30 (m, 2H) ; 2,95 et 2,93 (2s, 3H) ; 2,88 et 2,80 (2s, 3H) ; 0,99 (d, 6H).

## PREPARATION CCXXVII

**N-[(4-cyanophényl)méthyl]-N-méthyl-2-[2-[méthyl[(4-méthoxy-2,6-diméthylphényl)sulfonyl) amino]éthoxy] acétamide**

En opérant de façon analogue à la préparation CCXXVI, au départ de l'acide obtenu selon la préparation CCIX, on obtient le produit attendu sous forme d'une huile incolore (rendement = 95 %).
RMN$^1$H (250 MHz, DMSO) δ : 7,81 (d, 2H) ; 7,37 (d, 2H) ; 6,80 (d, 2H) ; 4,57 (s, 2H) ; 4,20 et 4,10 (2s, 2H) ; 3,79 (s, 3H) ; 3,45 (m, 2H) ; 3,24 et 3,15 (2t, 2H) ; 2,85 et 2,76 (2s, 3H) ; 2,71 et 2,65 (2s, 3H) ; 2,53 (s, 6H).

## PREPARATION CCXXVIII

**N-[[4-(aminothioxométhyl)phényl]-N-méthyl]-N-méthyl-2-[2-[méthyl[(4-méthoxy-2,6-diméthylphényl)sulfonyl] amino]éthoxy]acétamide**

On prépare une solution de 1,655 g (5mM) du composé obtenu selon la préparation CCXXVII dans 50 ml de pyridine et on ajoute 2,09 ml (25 mM) de triéthylamine. On introduit ensuite dans cette solution du sulfure d'hydrogène gazeux que l'on fait barboter pendant 10 minutes. La solution jaune au départ prend une couleur verte. Le mélange réactionel est ensuite agité pendant 6 heures à température ambiante puis dilué avec 200 ml d'acétate d'éthyle. La solution obtenue est concentrée sous pression réduite. Le résidu est repris dans 200 ml de toluène et la solution est séchée sur sulfate de magnésium et concentrée sous pression réduite. On obtient ainsi 1,5 g du produit attendu sous forme d'une pâte jaune (rendement = 84 %).
RMN$^1$H (300 MHz, DMSO) δ : 9,82 (m large, 1H) ; 9,45 (m large, 1H) ; 7,87 (m, 2H) ; 7,20 (m, 2H) ; 6,79 (d, 2H) ; 4,51 (s, 2H) ; 4,18 et 4,12 (2s, 2H) ; 3,78 (s, 3H) ; 3,56 (m, 2H) ; 3,24 (m, 2H) ; 2,82 et 2,76 (2s, 3H) ; 2,71 et 2,68 (s, 3H) ; 2,53 (s, 6H).

## PREPARATION CCXXIX

**N-[[4-[imino(méthylthio)méthyl]phényl]méthyl]-N-méthyl-2-[2-[méthyl[(4-méthoxy-2,6-diméthylphényl)sulfonyl]amino]éthoxy]acétamide**

On dissout 1,5 g (3,04 mM) du composé obtenu selon la préparation CCXXVIII dans 5 ml d'acétone et on ajoute 3 ml d'iodure de méthyle. Le mélange est chauffé à reflux pendant 1 heure puis concentré sous pression réduite. Le résidu est repris dans 50 ml de DCM et la solution est lavée par une solution de bicarbonate de sodium. La phase organique est séchée puis concentrée sous pression réduite. On obtient ainsi le produit attendu, utilisé sans autre purification (rendement = 98 %).
RMN $^1H$ (300 MHz, DMSO) $\delta$ : 10,25 (m, 1H) ; 7,78 (m, 2H) ; 7,27 (m, 2H) ; 6,79 (d, 2H) ; 4,53 (s, 2H) ; 4,18 et 4,12 (2s, 2H) ; 3,79 (s, 3H) ; 3,54 (m, 2H) ; 3,24 et 3,18 (2t, 2H) ; 2,85 et 2,77 (2s, 3H) ; 2,71 et 2,67 (2s, 3H) ; 2,58 (s, 6H) ; 2,40 (s, 3H).

## Exemple 104

**N-[[4-(1*H*-1,2,4-triazol-5-yl)phényl]méthyl]-N-méthyl-2-[2-[méthyl[(4-méthoxy-2,6-diméthylphényl)sulfonyl]amino]éthoxy]acétamide**

On prépare une solution de 1,5 g (2,96 mM) du composé obtenu selon la préparation CCXXIX dans 16,5 ml d'éthanol, puis on ajoute 324 mg (5,4 mM) de formylhydrazine, 279 mg (2,63 mM) de triéthylamine et 35 µl d'acide sulfurique. Le mélange réactionnel est chauffé à reflux pendant 2 heures puis concentré sous pression réduite. Le résidu est repris en solution dans le DCM, lavé deux fois à l'eau, séché sur sulfate de magnésium et concentré sous pression réduite. Le résidu est purifié une première fois sur colonne de silice en éluant à l'aide d'un mélange toluène/isopropanol (9/1 ;v/v), puis une seconde fois par chromatographie en phase inverse sur silice greffée RP18, en éluant avec un mélange acétonitrile/eau (4/6 ; v/v). Le produit pur est obtenu par lyophilisation des fractions pures. On obtient ainsi 340 mg du produit attendu sous forme d'un solide floconneux vert (rendement = 23 %).
F=60˚C

## PREPARATION CCXXX

**N-méthyl-2,4-dinitrobenzènesulfonamide**

En opérant de façon analogue à la préparation V, au départ de chlorure de 2,4-dinitro-benzènesulfonyle, on obtient le produit attendu sous forme d'un solide jaune (rendement = 76%).
F=155˚C

## PREPARATION CCXXXI

**Acide 4-[[(2,4-dinitrophényl)sulfonyl]méthylamino]-2-butènoique,1,1-diméthyléthyl ester**

En opérant de façon analogue à la préparation VI, au départ du composé obtenu selon la préparation CCXXX et de l'ester *t*-butylique de l'acide 4-bromo-2-butènoïque, on obtient le produit attendu sous forme d'un solide jaune (rendement = 82 %).
RMN $^1H$ (300 MHz, $CDCl_3$) $\delta$ : 8,50 (dd, 1H) ; 8,48 (m, 1H) ; 8,25 (d, 1H) ; 6,69 (dt, 1H) ; 5,92 (dt, 1H) ; 4,04 (dd, 2H) ; 2,93 (s, 3H) ; 1,48 (s, 9H),

## PREPARATION CCXXXII

**Acide 4-[[(2,4-dinitrophényl)sulfonyl]méthylamino]-2-butènoïque**

En opérant de façon analogue à la préparation LXXVIII, au départ de l'ester obtenu selon la préparation CCXXXI, on obtient le produit attendu sous forme d'un solide jaune pâle (rendement = 99 %).
F=174˚C

**PREPARATION CCXXXIII**

**Acide 2-[4-[[[(2E)-4-[[(2,4-dinitrophényl)sulfonyl]méthylamino]-1-oxo-2-butènyl]méthylamino]méthyl]phényl]-4,5-dihydro-1*H*-imidazole-1-carboxylique, 1,1-diméthyléthyl ester**

En opérant de façon analogue à la préparation VIII, au départ de l'acide obtenu selon la préparation CCXXXII et de l'amine obtenue selon la préparation IV, on obtient le produit attendu sous forme d'un solide jaune (rendement = 88 %).
F = 78˚C

**PREPARATION CCXXXIV**

**Acide 4,5-dihydro-2-[4-[[méthyl[(2E)-4-(méthylamino)-1-oxo-2-butènyl]amino]méthyl]phényl]-1*H*-imidazole-1-carboxylique, 1,1-diméthyléthyl ester**

On dissout 1 g (1,62 mM) du composé obtenu selon la préparation CCXXXIII dans 18 ml de DCM et on ajoute 672 mg (4,9 mM) de carbonate de potassium et 167 µl (1,62 mM) de thiophénol. Le mélange est agité pendant 1,5 heure à température ambiante, dilué avec 25 ml de DCM. Cette phase organique est lavée à l'eau 3 fois, séchée sur sulfate de magnésium et concentrée sous pression réduite. Le résidu est purifié par chromatographie sur gel de silice en éluant à l'aide d'un mélange DCM/méthanol (9/1 ; v/v). On obtient ainsi 560 mg du produit attendu sous forme d'une huile jaune (rendement = 89 %).
RMN $^1$H (250 MHz, $CDCl_3$) $\delta$ : 7,49 (dd, 2H) ; 7,22 (dd, 2H) ; 6,96 (m, 1H) ; 6,47 (dd, 1H) ; 4,65 (d, 2H) ; 3,97 (m, 4H) ; 3,40 (m, 2H) ; 2,98 et 2,96 (2s, 3H) ; 2,49 et 2,42 (2s, 3H) ; 1,29 et 1,25 (2s, 9H).

En opérant de façon analogue à la préparation V, au départ de l'amine obtenue selon la préparation CCXXXIV et de différents chlorures de benzènesulfonyle, on obtient les composés suivants :

**PREPARATION CCXXXV**

**Acide 2-[4-[[[(2E)-4-[[(2-chloro-4-fluorophényl)sulfonyl]méthylamino]-1-oxo-2-butènyl]méthylamino]méthyl] phényl]-4,5-dihydro-1*H*-imidazole-1-carboxylique, 1,1-diméthyléthyl ester**

(Huile incolore, rendement = 59 %)
RMN $^1$H (250 MHz, $CDCl_3$) $\delta$ : 8,13 (m, 1H) ; 7,50 (m, 2H) ; 7,25 (m, 4H) ; 6,79 (m, 1H) ; 6,51 1 (dd, 1H) ; 4,66 et 4,59 (2s, 2H) ; 4,10 et 4,01 (2d, 2H) ; 4,00 (m, 4H) ; 2,99 et 2,95 (2s, 3H) ; 2,86 et 2,75 (2s, 3H) ; 1,30 (d, 9H).

**PREPARATION CCXXXVI**

**Acide 2-[4-[[[(2E)-4-[[[2,6-dichloro-4-(trifluorométhyl)phényl]sulfonyl]méthylamino]-1-oxo-2-butènyl]méthylamino]méthyl]phényl]-4,5-dihydro-1*H*-imidazole-1-carboxylique, 1,1-diméthyléthyl ester**

(Huile incolore ; rendement = 73 %).
RMN $^1$H (250 MHz, $CDCl_3$) $\delta$ : 7,70 (d, 2H) ; 7,50 (m, 2H) ; 7,24 (m, 2H) ; 6,84 (m, 1H) ; 6,55 (dd, 1H) ; 4,66 et 4,59 (2s, 2H) ; 4,18 et 4,07 (2d, 2H) ; 4,00 (m, 4H) ; 3,00 et 2,96 (2s, 3H) ; 2,97 et 2,84 (2s, 3H) ; 1,27 (d, 9H).

**PREPARATION CCXXXVII**

**Acide 2-[4-[[[(2E)-4-[[(2,6-difluorophényl)sulfonyl]méthylamino]-1-oxo-2-butènyl]méthylamino]méthyl]phényl]-4,5-dihydro-1*H*-imidazole-1-carbobylique, 1,1-diméthyléthyl ester**

(Huile incolore ; rendement = 61 %).
RMN $^1$H (250 MHz, $CDCl_3$) $\delta$ : 7,52 (m, 3H) ; 7,27 (m, 2H) ; 7,02 (m, 2H) ; 6,85 (m, 1H) ; 6,53 (dd, 1H) ; 4,66 et 4,59 (2s, 2H) ; 4,09 et 4,01 (2d, 2H) ; 3,98 (m, 4H) ; 2,99 et 2,94 (2s, 3H) ; 2,95 et 2,82 (2s, 3H) ; 1,27 (d, 9H).

**PREPARATION CCXXXVIII**

**Acide 2-[4-[[[(2E)-4-[[(2-nitrophényl)sulfonyl]méthylamino]-1-oxo-2-butènyl]méthylamino]méthyl]phényl]-4,5-dibydro-1*H*-imidazole-1-carboxylique, 1,1-diméthyléthyl ester**

**PREPARATION CCXXXIX**

**Acide 2-[4-[[[(2E)-4-[[(2,4,6-triméthylphényl)sulfonyl]méthylamino]-1-oxo-2-butènyl]méthylamino]méthyl]phényl]-4,5-dihydro-1*H*-imidazole-1-carboxylique, 1,1-diméthyléthyl ester**

(Pâte blanche ; rendement = 69 %).
RMN$^1$H (300 MHz, $CDCl_3$) δ : 7,49 (dd, 2H) ; 7,25 (dd, 2H) ; 6,93 (d,2H); 6,77 (m, 1H) ; 6,53 (dd, 1H) ; 4,65 et 4,57 (2s, 2H) ; 4,06 et 3,85 (2d, 2H) ; 3,94 (m, 4H) ; 2,98 et 2,94 (2s, 3H) ; 2,71 et 2,57 (2s, 3H) ; 2,61 (s, 6H) ; 2,29 et 2,28 (2s, 3H) ; 1,25 (d, 9H).

**PREPARATION CCXL**

**Acide 2-[4-[[[(2E)-4-[[(2,5-dichlorophényl)sulfonyl]méthylamino]-1-oxo-2-butènyl]méthylamino]méthyl]phényl]-4,5-dihydro-1*H*-imidazole-1-carboxylique, 1,1-diméthyléthyl ester**

(Huile incolore ; rendement = 63 %).
RMN $^1$H (300 MHz, $CDCl_3$) δ : 8,05 (m, 1H) ; 7,46 (m, 4H) ; 7,27 (d, 1H) ; 7,17 (d, 1H) ; 6,81 1 (m, 1H) ; 6,50 (dd, 1H) ; 4,66 et 4,59 (2s, 2H) ; 4,12 et 4,04 (2d, 2H) ; 4,01 (m, 4H) ; 2,99 et 2,95 (2s, 3H) ; 2,89 et 2,78 (2s, 3H) ; 1,27 (d, 9H).

**PREPARATION CCXLI**

**Acide 2-[4-[[[(2E)-4-[[(2,4-dichlorophényl)sulfonyl]méthylamino]-1-oxo-2-butènyl]méthylamino]méthyl]phényl]-4,5-dihydro-1*H*-imidazole-1-carboxylique, 1,1-diméthyléthyl ester**

(Huile incolore ; rendement = 48 %).
RMN $^1$H (250 MHz, $CDCl_3$) δ : 8,05 (t, 1H) ; 7,54 (m, 3H) ; 7,37 (m, 1H) ; 7,27 (d, 1H) ; 7,20 (d, 1H) ; 6,83 (m, 1H) ; 6,50 (dd, 1H) ; 4,66 et 4,56 (2s, 2H) ; 4,10 et 3,99 (2d, 2H) ; 3,96 (m, 4H) ; 2,99 et 2,95 (2s, 3H) ; 2,86 et 2,75 (2s, 3H) ; 1,27 (d, 9H).

**PREPARATION CCXLII**

**Acide 2-[4-[[[(2E)-4-[[(3-chloro-2-méthylphényl)sulfonyl]méthylamino]-1-oxo-2-butènyl]méthylamino]méthyl] phényl]-4,5-dihydro-1*H*-imidazole-1-carboxylique, 1,1-diméthyléthyl ester**

(Huile incolore ; rendement = 69 %).
RMN$^1$H (250 MHz, $CDCl_3$) δ : 7,84 (t, 1H) ; 7,50 (m, 3H) ; 7,26 (m, 3H) ; 6,82 (m, 1H) ; 6,54 (dd, 1H) ; 4,66 et 4,58 (2s, 2H) ; 3,97 (m, 6H) ; 3,01 et 2,94 (2s, 3H) ; 2,82 et 2,70 (2s, 3H) ; 2,67 et 2,63 (2s, 3H) ; 1,27 (d, 9H).

**PREPARATION CCXLIII**

**Acide 2-[4-[[[(2E)-4-[[(2-cyanophényl)sulfonyl]méthylamino]-1-oxo-2-butènyl]méthylamino]méthyl]phényl]-4,5-dihydro-1*H*-imidazole-1-carboxylique, 1,1-diméthyléthyl ester**

(Solide jaune ; rendement = 67 %).
F=64˚C

**PREPARATION CCXLIV**

**Acide 2-[4-[[[(2E)-4-[[(2-chloro-4-méthoxyphényl)sulfonyl]méthylamino]-1-oxo-2-butènyl]méthylaminolméthyl]phényl]-4,5-dihydro-1*H*-imidazole-1-carboxylique, 1,1-diméthyléthyl ester**

(Solide jaune ; rendement = 78 %).
F=58˚C

PREPARATION CCXLV

**Acide 2-[4-[[[(2E)-4-[[(2,4-dichloro-5-méthylphényl)sulfonyl]méthylamino]-1-oxo-2-butènyl]méthylamino]méthyl]phényl]-4,5-dihydro-1*H*-imidazole-1-carboxylique, 1,1-diméthyléthyl ester**

(Solide blanc écru; rendement = 75 %).
F=70˚C

PREPARATION CCXLVI

**Acide 2-[4-[[[(2E)-4-[[(2,3,4-trichlorophényl)sulfonyl]méthylamino]-1-oxo-2-butènyl]méthylamino]méthyl]phényl]-4,5-dihydro-1*H*-imidazole-1-carboxylique, 1,1-diméthyléthyl ester**

(Solide blanc écru ; rendement = 78 %).
F = 66 ˚C

PREPARATION CCXLVII

**Acide 2-[4-[[[(2E)-4-[[(2-chloro-4-(trifluorométhoxy)phényl]sulfonyl]méthylamino]-1-oxo-2-butènyl]méthylamino]méthyl]phényl]-4,5-dihydro-1*H*-imidazole-1-carboxylique, 1,1-diméthyléthyl ester**

(Solide blanc; rendement = 81 %).
F=50˚C

PREPARATION CCXLVIII

**Acide 2-[4-[[[(2E)-4-[[[4-méthoxy-2-(trifluorométhyl)phényl]sulfonyl]méthylamino]-1-oxo-2-butènyl]méthylamino]méthyl]phényl]-4,5-dihydro-1*H*-imidazole-1-carboxylique, 1,1-diméthyléthyl ester**

(Solide blanc ; rendement = 62 %).
F=50˚C

PREPARATION CCIL

**Acide 2-[4-[[[(2E)-4-[[(2,6-diméthyl-4-méthoxyphényl)sulfonyl]méthylamino]-1-oxo-2-butènyl]méthylamino] méthyl]phényl]-4,5-dihydro-1*H*-imidazole-1-carboxylique, 1,1-diméthyléthyl ester**

(Solide blanc ; rendement = 75 %).
F=50˚C
En opérant de façon analogue à l'exemple 1, au départ des composés obtenus selon les préparations CCXXXV à CCIL, on obtient les produits suivants :

Exemple 105

**(2E)-4-[[(2-chloro-4-fluorophényl)sulfonyl]méthylamino]-N-[[4-(4,5-dihydro-1*H*-imidazol-2-yl)phényl]méthyl]-N-méthyl-2-butènamide, trifluoroacétate**

(Pâte rose ; rendement = 99%)
RMN $^1$H (250 MHz, DMSO) δ : 10,48 (s, 2H) ; 8,09 (m, 1H) ; 7,91 (m, 2H) ; 7,78 (m, 1H) ; 7,49 (m, 3H) ; 6,61 (m, 2H) ; 4,76 et 4,66 (2s, 2H) ; 4,05 (m, 2H) ; 4,01 (s, 4H) ; 3,09 et 3,03 (2s, 3H) ; 2,96 et 2,90 (2s, 3H).

Exemple 106

**(2E)-4-[[[12,6-dichloro-4-(trifluorométhyl)phényl]sulfonyl]méthylamino]-N-[[4-(4,5-dihydro-1*H*-imidazol-2-yl) phényl]méthyl]-N-méthyl-2-butènamide, trifluoroacétate**

(solide blanc ; rendement = 99%)
F = 62˚C

**Exemple 107**

**(2E)-4-[[(2,6-difluorophényl)sulfonyl]méthylamino]-N-[[4-(4,5-dihydro-1*H*-imidazol-2-yl)phényl]méthyl]-N-méthyl-2-butènamide, trifluoroacétate**

(Pâte rose ; rendement = 99%)
RMN $^1$H (250 MHz, DMSO) δ : 10,49 (s, 2H) ; 7,90 (m, 2H) ; 7,80 (m, 1H) ; 7,47 (d, 2H) ; 7,32 (m, 2H) ; 6,60 (m, 2H) ; 4,76 et 4,66 (2s, 2H) ; 4,04 et 3,94 (2d, 2H) ; 4,01 (s, 4H) ; 3,03 et 2,90 (2s, 3H) ; 2,86 et 2,71 (2s, 3H).

**Exemple 108**

**(2E)-4-[[(2-nitrophényl)sulfonyl]méthylamino]-N-[[4-(4,5-dihydro-1*H*-imidazol-2-yl)phényl]méthyl]-N-méthyl-2-butènamide, trifluoroacétate**

(Pâte rose ; rendement = 94%)
RMN $^1$H (250 MHz, DMSO) δ : 10,54 (s, 2H) ; 7,94 (m, 6H) ; 7,48 (m, 2H) ; 6,60 (m, 2H) ; 4,74 et 4,65 (2s, 2H) ; 4,08 et 3,99 (2d, 2H) ; 4,01 (s, 4H) ; 3,01 et 2,89 (2s, 3H) ; 2,87 et 2,72 (2s, 3H).

**Exemple 109**

**(2E)-4-[[(2,4,6-triméthylphényl)sulfonyl]méthylamino]-N-[[4-(4,5-dihydro-1*H*-imidazol-2-yl)phényl]méthyl]-N-méthyl-2-butènamide, trifluoroacétate**

(Solide blanc ; rendement = 78%)
F=64˚C

**Exemple 110**

**(2E)-4-[[(2,5-dichlorophényl)sulfonyl]méthylamino]-N-[[4-(4,5-dihydro-1*H*-imidazol-2-yl)phényl]méthyl]-N-méthyl-2-butènamide, trifluoroacétate**

(Solide blanc ; rendement = 99%)
F = 65 ˚C

**Exemple 111**

**(2E)-4-[[(2,4-dichlorophényl)sulfonyl]méthylamino]-N-[[4-(4,5-dihydro-1*H*-imidazol-2-yl)phényl]méthyl]-N-méthyl-2-butènamide, trifluoroacétate**

(Solide blanc ; rendement = 99%)
F = 65 ˚C

**Exemple 112**

**(2E)-4-[[(3-chloro-2-méthylphényl)sulfonyl]méthylamino]-N-[[4-(4,5dihydro-1*H*-imidazol-2-yl)phényl]méthyl]-N-méthyl-2-butènamide, trifluoroacétate**

(Solide blanc ; rendement = 99%)
F = 64 ˚C

**Exemple 113**

**(2E)-4-[[(2-cyanophényl)sulfonyl]méthylamino]-N-[[4-(4,5-dihydro-1*H*-imidazol-2-yl)phényl]méthyl]-N-méthyl-2-butènamide, trifluoroacétate**

(Solide blanc ; rendement = 99%)
F=60˚C

**Exemple 114**

**(2E)-4-[[(2-chloro-4-méthoxyphényl)sulfonyl]méthylamino]-N-[[4-(4,5-dihydro-1*H*-imidazol-2-yl)phényl]méthyl]-N-méthyl-2-butènamide, trifluoroacétate**

(Solide blanc ; rendement = 90%)
F = 68 °C

**Exemple 115**

**(2E)-4-[[(2,4-dichloro-5-méthylphényl)sulfonyl]méthylamino]-N-[[4-(4,5-dihydro-1*H*-imidazol-2-yl)phényl]méthyl]-N-méthyl-2-butènamide, trifluoroacétate**

(Solide blanc ; rendement = 99 %)
F = 79 °C

**Exemple 116**

**(2E)-4-[[(2,3,4-trichlorophényl)sulfonyl]méthylamino]-N-[[4-(4,5-dihydro-1*H*-imidazol-2-yl)phényl]méthyl]-N-méthyl-2-butènamide, trifluoroacétate**

(Solide blanc ; rendement = 90 %)
F = 87 °C

**Exemple 117**

**(2E)-4-[[[2-chloro-4-(trifluorométhoxy)phényl]sulfonyl]méthylamino]-N-[[4-(4,5-dihydro-1*H*-imidazol-2-yl)phényl]méthyl]-N-méthyl-2-butènamide, trifluoroacétate**

(Solide blanc ; rendement = 99 %)
F=68°C

**Exemple 118**

**(2E)-4-[[[4-méthoxy-2-(trifluorométhyl)phényl]sulfonyl]méthylamino]-N-[[4-(4,5-dihydro-1*H*-imidazol-2-yl)phényl]métbyl]-N-méthyl-2-butènamide, trifluoroacétate**

(Solide blanc ; rendement = 99 %)
F=50°C

**Exemple 119**

**(2E)-4-[[(4-méthoxy-2,6-diméthylphényl)sulfonyl]méthylamino]-N-[[4-(4,5-dihydro-1*H*-imidazol-2-yl)phényl]méthyl]-N-méthyl-2-butènamide, trifluoroacétate**

(Solide blanc ; rendement = 99 %)
F = 50 °C
En opérant de façon analogue à la préparation V, au départ de l'amine obtenue selon la préparation CLXXIX et de différents chlorures de benzènesulfonyle, on obtient les composés suivants :

**PREPARATION CCL**

**Acide 2-[4-[[[5-[[(2-chloro-4-méthoxyphényl)sulfonyl]méthylamino]-1-oxopentyl] méthylamino]méthyl]phényl]-4,5-dihydro-1*H*-imidazole-1-carboxylique, 1,1-diméthyléthyl ester**

(Solide blanc ; rendement = 32 %)
F=60°C

## PREPARATION CCLI

**Acide 2-[4-[[[5-[[(4-méthoxy-2-méthylphényl)sulfonyl]méthylamino]-1-oxopentyl] méthylamino]méthyl]phényl]-4,5-dihydro-1*H*-imidazole-1-carboxylique, 1,1-diméthyléthyl ester**

(Huile incolore ; rendement = 24 %)
RMN $^1$H (250 MHz, DMSO) δ : 7,73 (t, 1H) ; 7,42 (m, 2H) ; 7,20 (m, 2H) ; 6,93 (m, 2H) ; 4,56 (d, 2H) ; 3,86 (s, 4H) ; 3,81 (s, 3H) ; 3,04 (m, 2H) ; 2,88 et 2,80 (2s, 3H) ; 2,68 et 2,65 (2s, 3H) ; 2,48 (m, 2H) ; 1,50 (m, 4H) ; 1,17 (s, 9H).

## PREPARATION CCLII

**Acide 2-[4-[[[5-[[[2-méthyl-4-(trifluorométhoxy)phényl]sulfonyl]méthylamino]-1-oxopentyl] méthylamino]méthyl]phényl]-4,5-dihydro-1*H*-imidazole-1-carboxylique, 1,1-diméthyléthyl ester**

(Huile incolore ; rendement = 30 %)
RMN $^1$H (300 MHz, DMSO) δ : 7,88 (t, 1H) ; 7,44 (m, 4H) ; 7,20 (m, 2H) ; 4,59 et 4,52 (2s, 2H) ; 3,83 (m, 4H) ; 3,14 (m, 2H) ; 2,89 et 2,81 (2s, 3H) ; 2,76 et 2,74 (2s, 3H) ; 2,55 et 2,51 (2s, 3H) ; 2,50 (m, 2H) ; 1,52 (m, 4H) ; 1,17 (s, 9H).

## PREPARATION CCLIII

**Acide 2-[4-[[[5-[[[4-méthoxy-2-(trifluorométhyl)phényl]sulfonyl]méthylamino]-1-oxopentyl] méthylamino]méthyl]phényl]-4,5-dibydro-1*H*-imidazole-1-carboxylique, 1,1-diméthyléthyl ester**

(Huile incolore ; rendement = 35 %)
RMN $^1$H (300 MHz, DMSO) δ : 7,95 (t, 1H) ; 7,41 (m, 4H) ; 7,22 (m, 2H) ; 4,59 et 4,52 (2s, 2H) ; 3,91. (s, 3H) ; 3,83 (m, 4H) ; 3,16 (m, 2H) ; 2,89 et 2,81 (2s, 3H) ; 2,78 et 2,76 (2s, 3H) ; 2,36 (m, 2H) ; 1,48 (m, 4H) ; 1,17 (s, 9H).

En opérant de façon analogue à l'exemple 1, au départ des composés obtenus selon les préparations CCL à CCLIII, on obtient les produits suivants :

## Exemple 120

**5-[[(2-chloro-4-méthoxyphényl)sulfonyl]méthylamino]-N-[[4-(4,5-dihydro-1*H* imidazol-2-yl)phényl]méthyl]-N-méthyl-pentanamide, trifluoroacétate**

(Huile jaune ; rendement = 97 %)
RMN $^1$H (300 MHz, DMSO) δ : 10,50 (d, 2H) ; 7,90 (m, 3H) ; 7,45 (d, 2H) ; 7,26 (s, 1H) ; 7,11 1 (m, 1H) ; 4,69 et 4,60 (2s, 2H) ; 4,00 (s, 4H) ; 3,86 (s, 3H) ; 3,14 (m, 2H) ; 2,95 et 2,82 (2s, 3H) ; 2,75 et 2,72 (2s, 3H) ; 2,49 et 2,30 (2m, 2H) ; 1,52 (m, 4H).

## Exemple 121

**5-[[(4-méthoxy-2-méthylphényl)sulfonyl]méthylamino]-N-[[4-(4,5-dihydro-1*H* imidazol-2-yl)phényl]méthyl]-N-méthyl-pentanamide, trifluoroacétate**

(Huile incolore ; rendement = 94 %)
RMN $^1$H (300 MHz, DMSO) δ : 10,52 (d, 2H) ; 7,90 (m, 2H) ; 7,70 (m, 1H) ; 7,47 (m, 2H); 6,90 (m, 2H) ; 4,68 et 4,60 (2s, 2H) ; 4,01 (s, 4H) ; 3,81 (s, 3H) ; 3,07 (m, 2H) ; 2,94 et 2,82 (2s, 3H) ; 2,68 et 2,64 (2s, 3H) ; 2,50 (s, 3H) ; 2,49 et 2,39 (2t, 2H) ; 1,52 (m, 4H).

## Exemple 122

**5-[[[2-méthyl-4-(trifluorométhoxy)phényl]sulfonyl]méthylamino]-N-[[4-(4,5-dibydro-1*H*-imidazol-2-yl)phényl] méthyl]-N-méthyl-pentanamide, trifluoroacétate**

(Pâte rouge ; rendement = 99 %)
RMN $^1$H (250 MHz, DMSO) δ : 10,49 (d, 2H) ; 7,89 (m, 3H) ; 7,44 (m, 4H) ; 4,69 et 4,60 (2s, 2H) ; 4,00 (s, 4H) ; 3,14 (m, 2H) ; 2,95 et 2,82 (2s, 3H) ; 2,76 et 2,73 (2s, 3H) ; 2,57 et 2,55 (2s, 3H) ; 2,44 et 2,38 (2m, 2H) ; 1,50 (m, 4H).

**Exemple 123**

**5-[[[4-méthoxy-2-(trifluorométhyl)phényl)sulfonyl]méthylamino]-N-[[4-(4,5-dihydro-1*H*-imidazol-2-yl)phényl]méthyl]-N-méthyl-pentanamide, trifluoroacétate**

(Pâte rose ; rendement = 99 %)
RMN $^1H$ (250 MHz, DMSO) δ : 10,50 (d, 2H) ; 7,90 (m, 3H) ; 7,40 (m, 4H) ; 4,69 et 4,60 (2s, 2H) ; 4,00 (s, 4H) ; 3,92 (s, 3H) ; 3,19 (m, 2H) ; 2,96 et 2,83 (2s, 3H) ; 2,78 et 2,75 (2s, 3H) ; 2,49 et 2,45 (2m, 2H) ; 1,53 (m, 4H).

En opérant de façon analogue à la préparation V, au départ de l'amine obtenue selon la préparation CLII et de différents chlorures de benzènesulfonyle, on obtient les composés suivants :

**PREPARATION CCLIV**

**Acide 2-[4-[[[[2-[[(2-chloro-4-méthoxyphényl)sulfonyl]méthylamino]éthoxy] acétyl]méthylamino]méthyl]phényl]-4,5-dihydro-1*H*-imidazole-1-carboxylique,1,1-diméthyléthyl ester**

(Huile incolore ; rendement = 64 %)
RMN $^1H$ (300 MHz, DMSO) δ : 7,89 (m, 1H) ; 7,42 (m, 2H) ; 7,22 (m, 3H) ; 7,05 (dd, 1H) ; 4,52 (s, 2H) ; 4,22 et 4,18 (2s, 2H) ; 3,82 (m, 4H) ; 3,79 (s, 3H) ; 3,60 (m, 2H) ; 3,39 (m, 2H) ; 2,85 et 2,76 (2s, 3H) ; 2,84 et 2,82 (2s, 3H) ; 1,18 (s, 9H).

**PREPARATION CCLV**

**Acide 2-[4-[[[[2-[[(4-méthoxy-2-méthylphényl)sulfonyl]méthylamino]éthoxy]acétyl] méthylamino]méthyl]phényl]-4,5-dihydro-1*H*-imidazole-1-carboxylique,1,1-diméthyléthyl ester**

(Huile incolore ; rendement = 53 %)
RMN $^1H$ (300 MHz, DMSO) δ : 7,74 (m, 1H) ; 7,44 (m, 2H) ; 7,23 (d, 2H) ; 6,92 (m, 2H) ; 4,51 (s, 2H) ; 4,20 et 4,17 (2s, 2H) ; 3,84 (m, 4H) ; 3,81 (s, 3H) ; 3,59 (m, 2H) ; 3,26 (m, 2H) ; 2,83 et 2,77 (2s, 3H) ; 2,78 et 2,75 (2s, 3H) ; 2,50 (s, 3H) ; 1,17 (s, 9H).

En opérant de façon analogue à l'exemple 1, au départ des composés précédents, on obtient les composés suivants :

**Exemple 124**

**2-[2-[[(2-chloro-4-méthoxyphényl)sulfonyl]méthylamino]éthoxy]-N-[[4-(4,5-dihydro-1*H*-imidazol-2-yl)phényl]méthyl]-N-méthyl-acétamide, trifluoroacétate**

(Solide blanc, rendement = 96 %).
F = 60°C

**Exemple 125**

**2-[2-[[(4-méthoxy-2-méthylphényl)sulfonyl]méthylamino]éthoxy]-N-[[4-(4,5-dihydro-1*H*-imidazol-2-yl)phényl]méthyl]-N-méthyl-acétamide, trifluoroacétate**

(Pâte blanche ; rendement = 94 %).
RMN$^1H$ (300 MHz, DMSO) δ : 10,50 (d, 2H) ; 7,90 (m, 2H) ; 7,73 (m, 1H) ; 7,49 (d, 2H) ; 6,97 (m, 1H) ; 6,90 (m, 1H) ; 4,60 (s, 2H) ; 4,25 et 4,16 (2s, 2H) ; 4,00 (s, 4H) ; 3,81 (s, 3H) ; 3,56 (m, 2H) ; 3,30 et 3,20 (2t, 2H) ; 2,89 et 2,72 (2s, 3H) ; 2,78 (s, 3H) ; 2,50 (s, 3H).

Tableau I

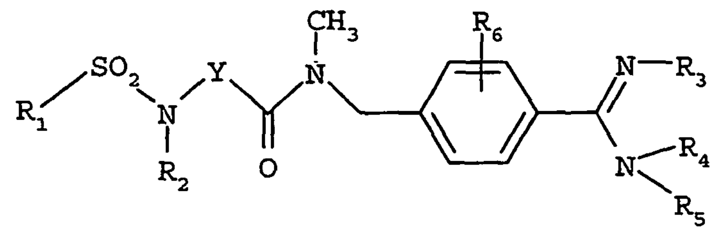

| Ex.* | $R_1$ | $R_2$ | Y | $R_3$ | $R_4$ | $R_5$ | $R_6$ |
|---|---|---|---|---|---|---|---|
| 1 | 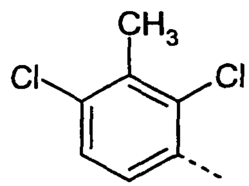 | $CH_3$ | $-(CH_2)_4-$ | $-(CH_2)_2-$ | | H | H |
| 2 | 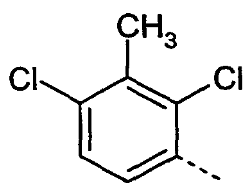 | $CH_3$ | $-(CH_2)_3-$ | $-(CH_2)_2-$ | | H | H |
| 3 | 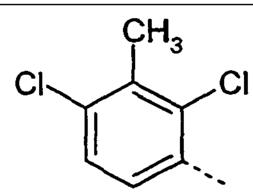 | $CH_3$ | $-(CH_2)_2-$ | $-(CH_2)_2-$ | | H | H |

(suite)

| Ex.* | R1 | R2 | Y | R3 | R4 | R5 | R6 |
|---|---|---|---|---|---|---|---|
| 4 | 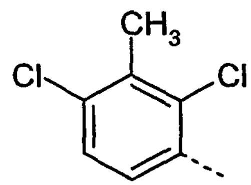 | $CH_3$ | $-CH_2-CH=CH-$ | $-(CH_2)_2-$ | | H | H |
| 5 | 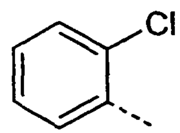 | $CH_3$ | $-(CH_2)_4-$ | $-(CH_2)_2-$ | | H | H |
| 6 | 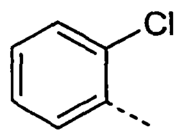 | $CH_3$ | $-CH_2-CH=CH-$ | $-(CH_2)_2-$ | | H | H |
| 7 | 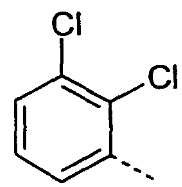 | $CH_3$ | $-(CH_2)_2-$ | $-(CH_2)_2-$ | | H | H |
| 8 | 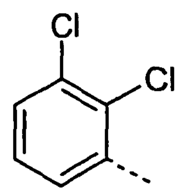 | $CH_3$ | $-(CH_2)_3-$ | $-(CH_2)_2-$ | | H | H |

(suite)

| Ex.* | $R_1$ | $R_2$ | Y | $R_3$ | $R_4$ | $R_5$ | $R_6$ |
|---|---|---|---|---|---|---|---|
| 9 | 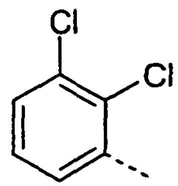 | $CH_3$ | $-(CH_2)_4-$ | $-(CH_2)_2-$ | | H | H |
| 10 | 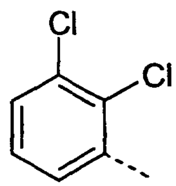 | $CH_3$ | $-CH_2-CH=CH-$ | $-(CH_2)_2-$ | | H | H |
| 11 | 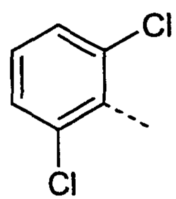 | $CH_3$ | $-(CH_2)_4-$ | $-(CH_2)_2-$ | | H | H |
| 12 | 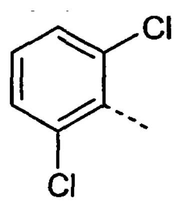 | $CH_3$ | $-CH_2-CH=CH-$ | $-(CH_2)_2-$ | | H | H |
| 13 | 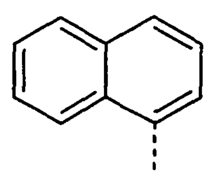 | $CH_3$ | $-(CH_2)_4-$ | $-(CH_2)_2-$ | | H | H |

(suite)

| Ex.* | $R_1$ | $R_2$ | Y | $R_3$ | $R_4$ | $R_5$ | $R_6$ |
|---|---|---|---|---|---|---|---|
| 14 | 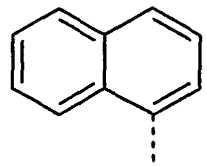 | $CH_3$ | $-CH_2-CH=CH-$ | $-(CH_2)_2-$ | | H | H |
| 15 | 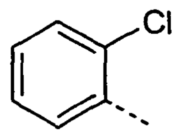 | cPr | $-(CH_2)_4-$ | $-(CH_2)_2-$ | | H | H |
| 16 | 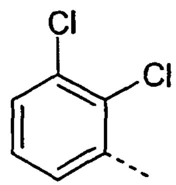 | cPr | $-(CH_2)_4-$ | $-(CH_2)_2-$ | | H | H |
| 17 | 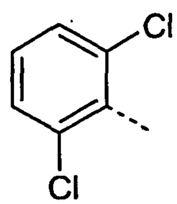 | cPr | $-(CH_2)_4-$ | $-(CH_2)_2-$ | | H | H |
| 18 | 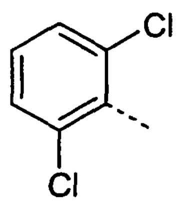 | $CH(CH_3)_2$ | $-(CH_2)_4-$ | $-(CH_2)_2-$ | | H | H |

(suite)

| Ex.* | $R_1$ | $R_2$ | Y | $R_3$ | $R_4$ | $R_5$ | $R_6$ |
|---|---|---|---|---|---|---|---|
| 19 | 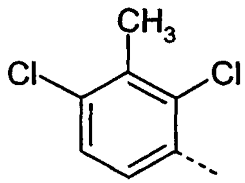 | $CH_2CONH_2$ | $-(CH_2)_4-$ | $-(CH_2)_2-$ | | H | H |
| 20 | 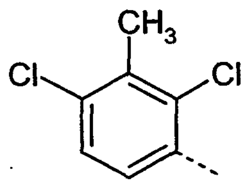 | $(CH_2)_2CONH_2$ | $-(CH_2)_4-$ | $-(CH_2)_2-$ | | H | H |
| 21 | 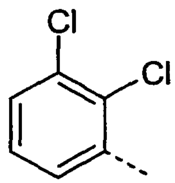 | $CH(CH_3)_2$ | $-(CH_2)_2-$ | $-(CH_2)_2-$ | | H | H |
| 22 | 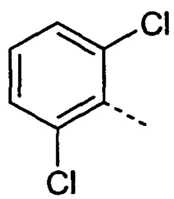 | $CH(CH_3)_2$ | $-(CH_2)_2-$ | $-(CH_2)_2-$ | | H | H |
| 23 | 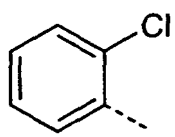 | cPr | $-(CH_2)_2-O-CH_2-$ | $-(CH_2)_2-$ | | H | H |

(suite)

| Ex.* | $R_1$ | $R_2$ | Y | $R_3$ | $R_4$ | $R_5$ | $R_6$ |
|---|---|---|---|---|---|---|---|
| 24 | 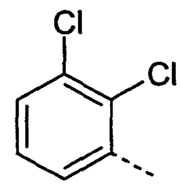 | cPr | $-(CH_2)_2-O-CH_2-$ | $-(CH_2)_2-$ | | H | H |
| 25 | 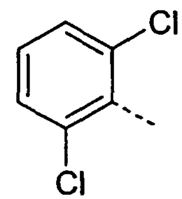 | cPr | $-(CH_2)_2-O-CH_2-$ | $-(CH_2)_2-$ | | H | H |
| 26 | 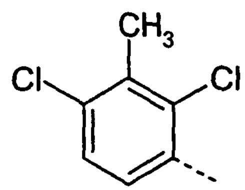 | $(CH_2)_2-Ph$ | $-(CH_2)_2-O-CH_2-$ | $-(CH_2)_2-$ | | H | H |
| 27 | 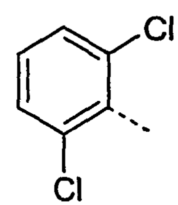 | $CH_3$ | $-(CH_2)_2-$ | $-(CH_2)_2-$ | | H | H |
| 28 | 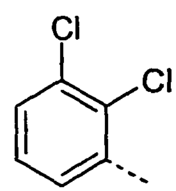 | cPr | $-(CH_2)_2-$ | $-(CH_2)_2-$ | | H | H |

(suite)

| Ex.* | $R_1$ | $R_2$ | Y | $R_3$ | $R_4$ | $R_5$ | $R_6$ |
|---|---|---|---|---|---|---|---|
| 29 | 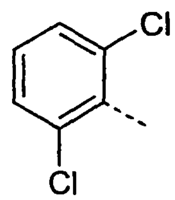 | cPr | $-(CH_2)_2-$ | $-(CH_2)_2-$ | | H | H |
| 30 | 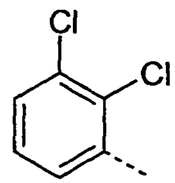 | $CH_2-CF_3$ | $-(CH_2)_2-$ | $-(CH_2)_2-$ | | H | H |
| 31 | 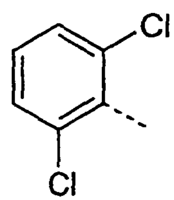 | $CH_2-CF_3$ | $-(CH_2)_2-$ | $-(CH_2)_2-$ | | H | H |
| 32 | 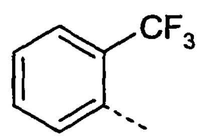 | $CH_3$ | $-CH_2-CH=CH-$ | $-(CH_2)_2-$ | | H | H |
| 33 | 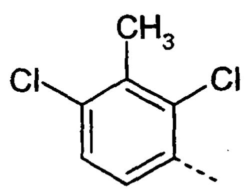 | H | $-CH_2-CH=CH-$ | $-(CH_2)_2-$ | | H | H |

(suite)

| Ex.* | $R_1$ | $R_2$ | Y | $R_3$ | $R_4$ | $R_5$ | $R_6$ |
|---|---|---|---|---|---|---|---|
| 34 bas | 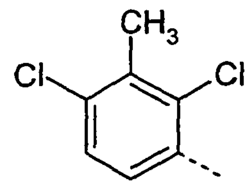 | $CH_3$ | $-(CH_2)_4-$ | $-CH_2-C(CH_3)_2-$ | | H | H |
| 35 | 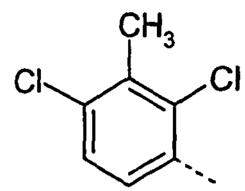 | $CH_3$ | $-(CH_2)_4-$ | $-CH_2-C(CH_3)_2-$ | | H | H |
| 36 bas | 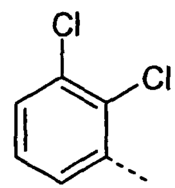 | $CH_3$ | $-(CH_2)_2-O-CH_2-$ | $-(CH_2)_3-$ | | H | H |
| 37 bas | 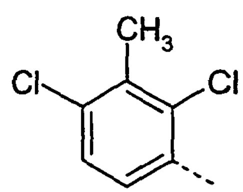 | $C_3$ | $-(CH_2)_2-O-CH_2-$ | $-(CH_2)_2-$ | | $CH_3$ | H |
| 38 bas | 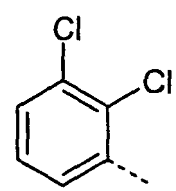 | $CH_3$ | $-(CH_2)_2-O-CH_2-$ | $-(CH_2)_2-$ | | $CH_3$ | H |

(suite)

| Ex.* | $R_1$ | $R_2$ | Y | $R_3$ | $R_4$ | $R_5$ | $R_6$ |
|---|---|---|---|---|---|---|---|
| 39 | 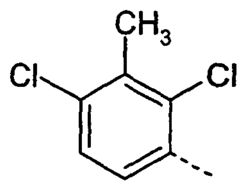 | $CH_3$ | $-(CH_2)_2-O-CH_2-$ | $-(CH_2)_2-$ | | H | H |
| 40 | 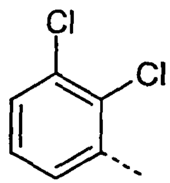 | $CH_3$ | $-(CH_2)_2-O-CH_2-$ | $-(CH_2)_2-$ | | H | H |
| 41 | 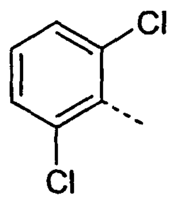 | $CH_3$ | $-(CH_2)_2-O-CH_2-$ | $-(CH_2)_2-$ | | H | H |
| 42 | 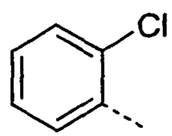 | $CH_3$ | $-(CH_2)_2-O-CH_2-$ | $-(CH_2)_2-$ | | H | H |
| 43 | 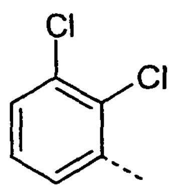 | $CH_3$ | $-(CH_2)_2-O-CH_2-$ | $-(CH_2)_2-$ | | H | 2-F |

(suite)

| Ex.* | $R_1$ | $R_2$ | Y | $R_3$ | $R_4$ | $R_5$ | $R_6$ |
|---|---|---|---|---|---|---|---|
| 44 chl | 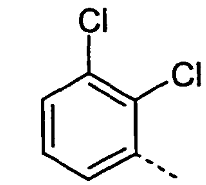 | $CH_3$ | 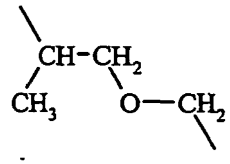 | $-(CH_2)_2-$ | | H | H |
| 45 | 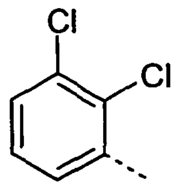 | $CH_3$ | $-(CH2)_2-O-CH_2$ | $-(CH_2)_3-$ | | H | H |
| 46 chl | 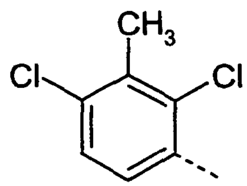 | $CH_3$ | $-(CH_2)_2-O-CH_2-$ | $-(CH_2)_2-$ | | $CH_3$ | H |
| 47 chl | 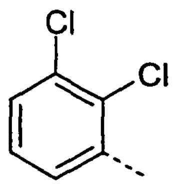 | $CH_3$ | $-(CH_2)_2-O-CH_2-$ | $-(CH_2)_2-$ | | $CH_3$ | H |
| 48 | 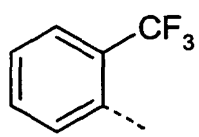 | $CH_3$ | $-(CH_2)_2-O-CH_2-$ | $-(CH_2)_2-$ | | H | H |

(suite)

| Ex.* | $R_1$ | $R_2$ | Y | $R_3$ | $R_4$ | $R_5$ | $R_6$ |
|---|---|---|---|---|---|---|---|
| 49 | 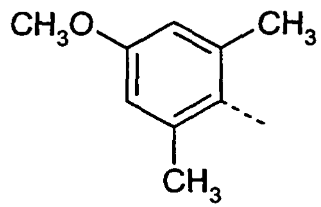 | $CH_3$ | $-(CH_2)_2-O-CH_2-$ | $-(CH_2)_2-$ | | H | H |
| 50 | 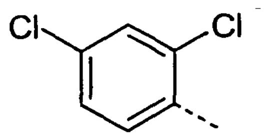 | $CH_3$ | $-(CH_2)_2-O-CH_2-$ | $-(CH_2)_2-$ | | H | H |
| 51 | 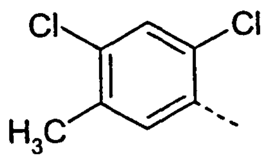 | $CH_3$ | $-(CH_2)_2-O-CH_2-$ | $-(CH_2)_2-$ | | H | H |
| 52 | 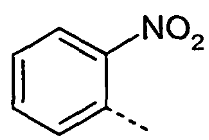 | $CH_3$ | $-(CH_2)_2-O-CH_2-$ | $-(CH_2)_2-$ | | H | H |
| 53 | 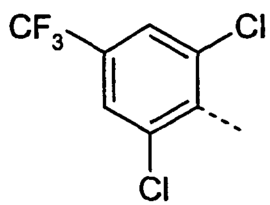 | $CH_3$ | $-(CH_2)_2-O-CH_2-$ | $-(CH_2)_2-$ | | H | H |

(suite)

| Ex.* | R1 | R2 | Y | R3 | R4 | R5 | R6 |
|---|---|---|---|---|---|---|---|
| 54 | 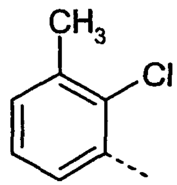 | $CH_3$ | $-(CH_2)_2-O-CH_2-$ | $-(CH_2)_2-$ | | H | H |
| 55 | 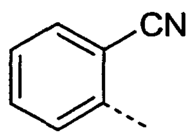 | $CH_3$ | $-(CH_2)_2-O-CH_2-$ | $-(CH_2)_2-$ | | H | H |
| 56 | 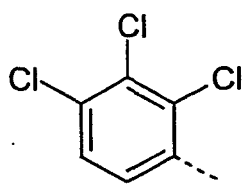 | $CH_3$ | $-(CH_2)_2-O-CH_2-$ | $-(CH_2)_2-$ | | H | H |
| 57 | 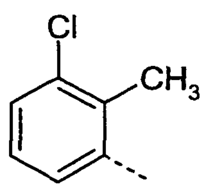 | $CH_3$ | $-(CH_2)_2-O-CH_2-$ | $-(CH_2)_2-$ | | H | H |
| 58 | 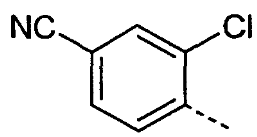 | $CH_3$ | $-(CH_2)_2-O-CH_2$ | $-(CH_2)_2-$ | | H | H |
| 59 | 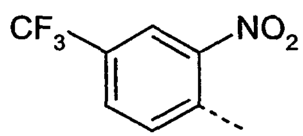 | $CH_3$ | $-(CH_2)_2-O-CH_2-$ | $-(CH)_2-$ | | H | H |

(suite)

| Ex.* | $R_1$ | $R_2$ | Y | $R_3$ | $R_4$ | $R_5$ | $R_6$ |
|---|---|---|---|---|---|---|---|
| 60 | 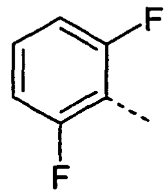 | $CH_3$ | $-(CH_2)_2-O-CH_2-$ | $-(CH_2)_2-$ | | H | H |
| 61 | 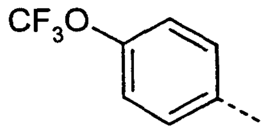 | $CH_3$ | $-(CH_2)_2-O-CH_2-$ | $-(CH_2)_2-$ | | H | H |
| 62 | 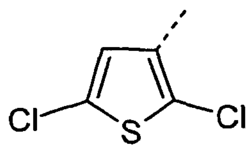 | $CH_3$ | $-(CH_2)-O-CH_2-$ | $-(CH_2)_2-$ | | H | H |
| 63 | 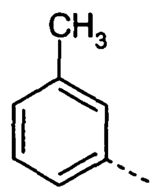 | $CH_3$ | $-(CH_2)_2-O-CH_2-$ | $-(CH_2)_2-$ | | H | H |
| 64 | 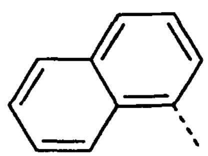 | $CH_3$ | $-(CH_2)_2-O-CH_2-$ | $-(CH_2)_2-$ | | H | H |

(suite)

| Ex.* | $R_1$ | $R_2$ | Y | $R_3$ | $R_4$ | $R_5$ | $R_6$ |
|---|---|---|---|---|---|---|---|
| 65 | 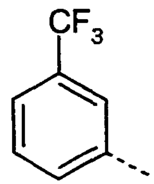 | $CH_3$ | $-(CH_2)_2-O-CH_2-$ | $-(CH_2)_2-$ | | H | H |
| 66 | 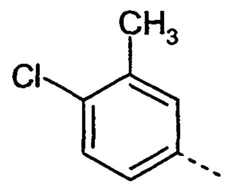 | $CH_3$ | $-(CH_2)_2-O-CH_2-$ | $-(CH_2)_2-$ | | H | H |
| 67 | 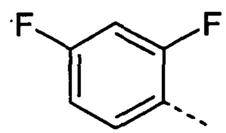 | $CH_3$ | $-(CH_2)_2-O-CH_2-$ | $-(CH_2)_2-$ | | H | H |
| 68 | 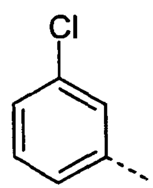 | $CH_3$ | $-(CH_2)_2-O-CH_2-$ | $-(CH_2)_2-$ | | H | H |
| 69 | 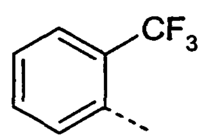 | $CH_3$ | $-(CH_2)_4-$ | $-(CH_2)_2-$ | | H | H |

(suite)

| Ex.* | $R_1$ | $R_2$ | Y | $R_3$ | $R_4$ | $R_5$ | $R_6$ |
|---|---|---|---|---|---|---|---|
| 70 | 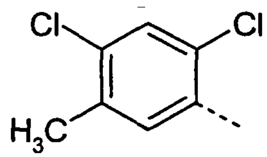 | $CH_3$ | $-(CH_2)_4-$ | $-(CH_2)_2-$ | | H | H |
| 71 | 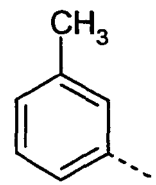 | $CH_3$ | $-(CH_2)_4-$ | $-(CH_2)_2-$ | | H | H |
| 72 | 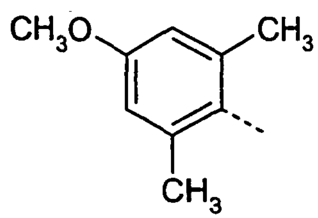 | $CH_3$ | $-(CH_2)_4-$ | $-(CH_2)_2-$ | | H | H |
| 73 | 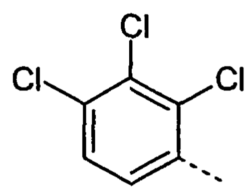 | $CH_3$ | $-(CH_2)_4-$ | $-(CH_2)_2-$ | | H | H |
| 74 | 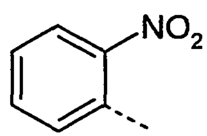 | $CH_3$ | $-(CH_2)_4-$ | $-(CH_2)_2-$ | | H | H |

(suite)

| Ex.* | $R_1$ | $R_2$ | Y | $R_3$ | $R_4$ | $R_5$ | $R_6$ |
|---|---|---|---|---|---|---|---|
| 75 | 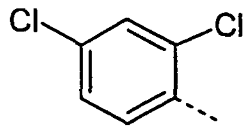 | $CH_3$ | $-(CH_2)_4-$ | $-(CH_2)_2-$ | | H | H |
| 76 | 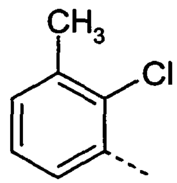 | $CH_3$ | $-(CH_2)_4-$ | $-(CH_2)_2-$ | | H | H |
| 77 | 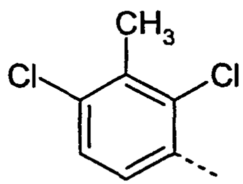 | $CH_3$ | $-(CH_2)_4-$ | OH | H | H | H |
| 78 | 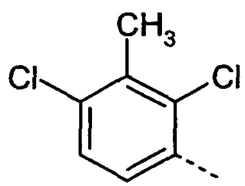 | $CH_3$ | $-(CH_2)_4-$ | H | H | H | H |
| 79 fum | 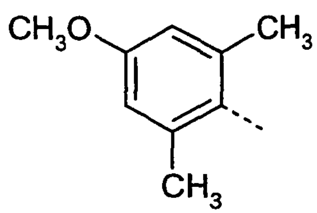 | $CH_3$ | $-(CH_2)_2-O-CH_2-$ | $-(CH_2)_2-$ | | H | H |

(suite)

| Ex.* | $R_1$ | $R_2$ | Y | $R_3$ | $R_4$ | $R_5$ | $R_6$ |
|---|---|---|---|---|---|---|---|
| 80 base | 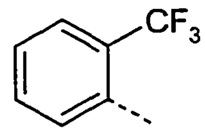 | $CH_3$ | $-CH_2-CH=CH-$ | $-(CH_2)_2-$. | | $CH_3$ | H |
| 81 fum | 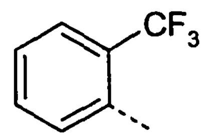 | $CH_3$ | $-CH_2-CH=CH-$ | $-(CH_2)_2-$ | | $CH_3$ | H |
| 82 | 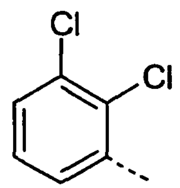 | $CH_3$ | $-CH_2-CH=CH-$ | $-(CH_2)_2-$ | | H | 2-F |
| 83 | 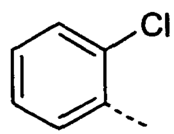 | $CH_3$ | $-CH_2-CH=CH-$ | $-(CH_2)_2-$ | | H | 2-F |
| 84 | 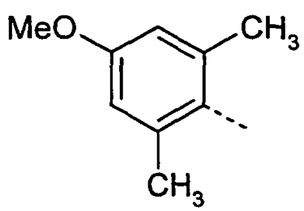 | $CH_3$ | $-(CH_2)_2-$ | $-(CH_2)_2-$ | | H | H |

(suite)

| Ex.* | $R_1$ | $R_2$ | Y | $R_3$ | $R_4$ | $R_5$ | $R_6$ |
|---|---|---|---|---|---|---|---|
| 85 base | 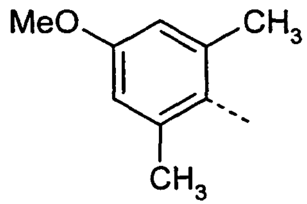 | $CH_3$ | $-(CH_2)_4-$ | $-(CH_2)_2-$ | | H | 2-Cl |
| 86 fum | 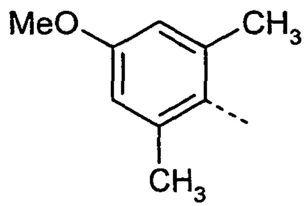 | $CH_3$ | $-(CH_2)_4-$ | $-(CH_2)_2-$ | | H - | 2-Cl |
| 87 base | 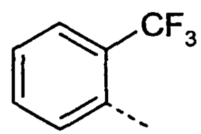 | $CH_3$ | $-(CH_2)_2-O-CH_2-$ | $-(CH_2)_2-$ | | $CH_3$ | H |
| 88 fum | 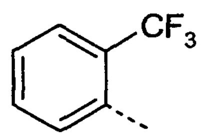 | $CH_3$ | $-(CH_2)_2-O-CH_2-$ | $-(CH_2)_2-$ | | $CH_3$ | H |
| 89 | 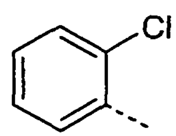 | $CH_3$ | $-(CH_2)_2-O-CH_2-$ | $-(CH_2)_2-$ | | H | 2-Cl |

(suite)

| Ex.* | $R_1$ | $R_2$ | Y | $R_3$ | $R_4$ | $R_5$ | $R_6$ |
|---|---|---|---|---|---|---|---|
| 90 | 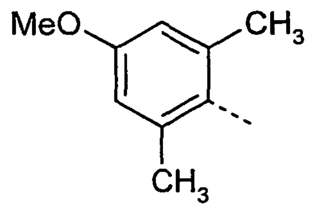 | $CH_3$ | $-(CH_2)_2-O-CH_2-$ | $-(CH_2)_2-$ | | H | 2-Cl |
| 91 base | 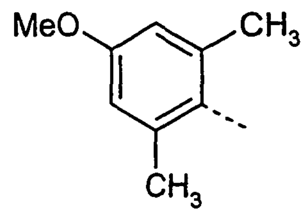 | $CH_3$ | $-(CH_2)_2-O-CH_2-$ | $-(CH_2)_2-$ | | iPr | H |
| 92 fum | 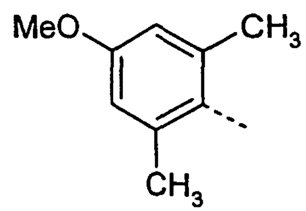 | $CH_3$ | $-(CH_2)_2-O-CH_2-$ | $-(CH_2)_2-$ | | iPr | H |
| 93 base | 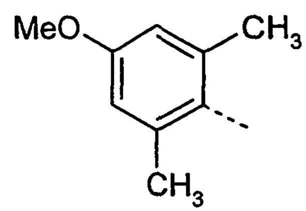 | $CH_3$ | $-(CH_2)_2-O-CH_2-$ | $-(CH_2)_2-$ | | $CH_3$ | 2-Cl |

(suite)

| Ex.* | $R_1$ | $R_2$ | Y | $R_3$ | $R_4$ | $R_5$ | $R_6$ |
|---|---|---|---|---|---|---|---|
| 94 fum | 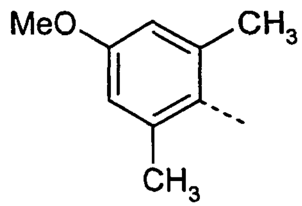 | $CH_3$ | $-(CH_2)_2-O-CH_2-$ | $-(CH_2)_2-$ | | $CH_3$ | 2-Cl |
| 95 base | 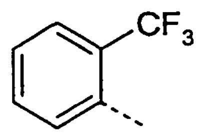 | $CH_3$ | $-(CH_2)_2-O-CH_2-$ | $-(CH_2)_2-$ | | $CH_3$ | 2-Cl |
| 96 fum | 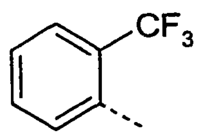 | $CH_3$ | $-(CH_2)_2-O-CH_2-$ | $-(CH_2)_2-$ | | $CH_3$ | 2-Cl |
| 97 base | 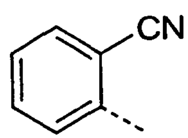 | $CH_3$ | $-(CH_2)_2-O-CH_2-$ | $-(CH_2)_2-$ | | $CH_3$ | H |
| 98 fum | 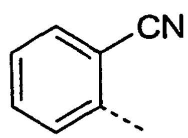 | $CH_3$ | $-(CH_2)_2-O-CH_2-$ | $-(CH_2)_2-$ | | $CH_3$ | H |
| 99 base | 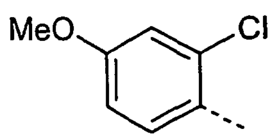 | $CH_3$ | $-(CH_2)_2-O-CH_2-$ | $-(CH_2)_2-$ | | $CH_3$ | H |

(suite)

| Ex.* | $R_1$ | $R_2$ | Y | $R_3$ | $R_4$ | $R_5$ | $R_6$ |
|---|---|---|---|---|---|---|---|
| 100 fum | 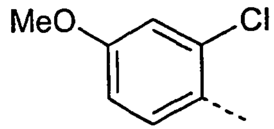 | $CH_3$ | $-(CH_2)_2-O-CH_2-$ | $-(CH_2)_2-$ | | $CH_3$ | H |
| 101 | 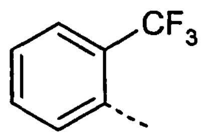 | $CH_3$ | $-(CH_2)_2-$ | $-(CH_2)_2-$ | | H | H |
| 102 base | 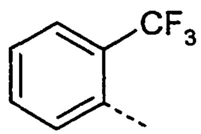 | $CH_3$ | $-(CH_2)_2-O-CH_2-$ | $-(CH_2)_2-$ | | Et | H |
| 103 base | 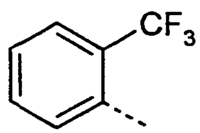 | $CH_3$ | $-(CH_2)_2-O-CH_2-$ | $-(CH_2)_2-$ | | iPr | H |
| 104 base | 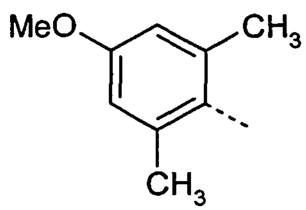 | $CH_3$ | $-(CH_2)_2-O-CH_2-$ | -N=CH- | | H | H |
| 105 | 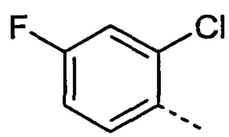 | $CH_3$ | $-CH_2-CH=CH-$ | $-(CH_2)_2-$ | | H | H |

(suite)

| Ex.* | $R_1$ | $R_2$ | Y | $R_3$ | $R_4$ | $R_5$ | $R_6$ |
|---|---|---|---|---|---|---|---|
| 106 | 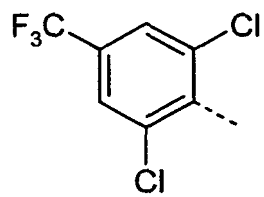 | $CH_3$ | $-CH_2-CH=CH-$ | $-(CH_2)_2-$ | | H | H |
| 107 | 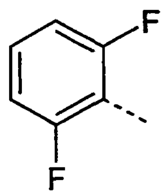 | $CH_3$ | $-CH_2-CH=CH-$ | $-(CH_2)_2-$ | | H | H |
| 108 | 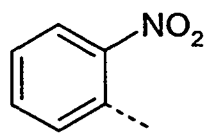 | $CH_3$ | $-CH_2-CH=CH-$ | $-(CH_2)_2-$ | | H | H |
| 109 | 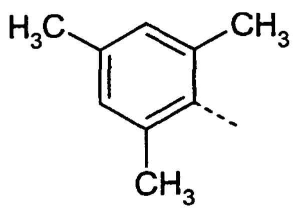 | $CH_3$ | $-CH_2-CH=CH-$ | $-(CH_2)_2-$ | | H | H |
| 110 | 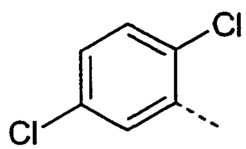 | $CH_3$ | $-CH_2-CH=CH-$ | $-(CH_2)_2-$ | | H | H |

(suite)

| Ex.* | $R_1$ | $R_2$ | Y | $R_3$ | $R_4$ | $R_5$ | $R_6$ |
|---|---|---|---|---|---|---|---|
| 111 | 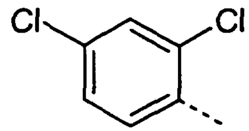 | $CH_3$ | $-CH_2-CH=CH-$ | $-(CH_2)_2-$ | | H | H |
| 112 | 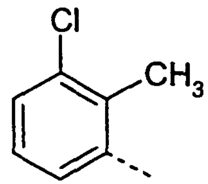 | $CH_3$ | $-CH_2-CH=CH-$ | $-(CH_2)_2-$ | | H | H |
| 113 | 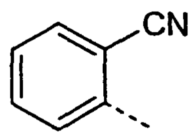 | $CH_3$ | $-CH_2-CH=CH-$ | $-(CH_2)_2-$ | | H | H |
| 114 | 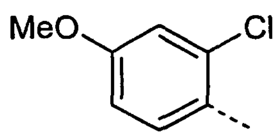 | $CH_3$ | $-CH_2-CH=CH-$ | $-(CH_2)_2-$ | | H | H |
| 115 | 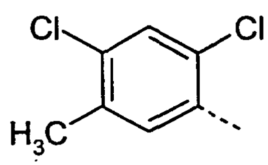 | $CH_3$ | $-CH_2-CH=CH-$ | $-(CH_2)_2-$ | | H | H |
| 116 | 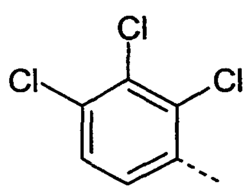 | $CH_3$ | $-CH_2-CH=CH-$ | $-(CH_2)_2-$ | | H | H |

(suite)

| Ex.* | $R_1$ | $R_2$ | Y | $R_3$ | $R_4$ | $R_5$ | $R_6$ |
|---|---|---|---|---|---|---|---|
| 117 | 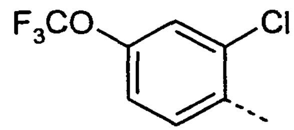 | $CH_3$ | $-CH_2-CH=CH-$ | $-(CH_2)_2-$ | | H | H |
| 118 | 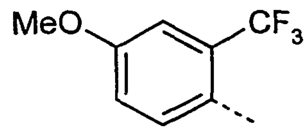 | $CH_3$ | $-CH_2-CH=CH-$ | $-(CH_2)_2-$ | | H | H |
| 119 | 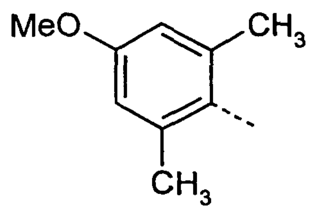 | $CH_3$ | $-CH_2-CH=CH-$ | $-(CH_2)_2-$ | | H | H |
| 120 | 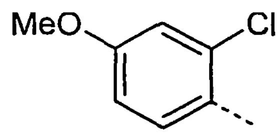 | $CH_3$ | $-(CH_2)_4-$ | $-(CH_2)_2-$ | | H | H |
| 121 | 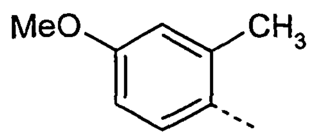 | $CH_3$ | $-(CH_2)_4-$ | $-(CH_2)_2-$ | | H | H |
| 122 | 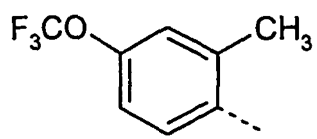 | $CH_3$ | $-(CH_2)_4-$ | $-(CH_2)_2-$ | | H | H |

(suite)

| Ex.* | $R_1$ | $R_2$ | Y | $R_3$ | $R_4$ | $R_5$ | $R_6$ |
|---|---|---|---|---|---|---|---|
| 123 | 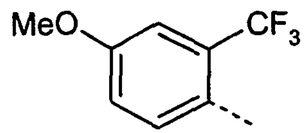 | $CH_3$ | $-(CH_2)_4-$ | $-(CH_2)_2-$ | | H | H |
| 124 | 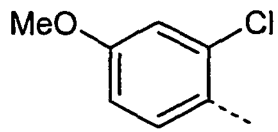 | $CH_3$ | $-(CH_2)_2-O-CH_2-$ | $-(CH_2)_2-$ | | H | H |
| 125 | 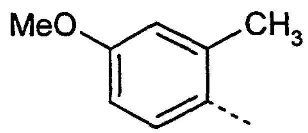 | $CH_3$ | $-(CH_2)_2-O-CH_2-$ | $-(CH_2)_2-$ | | H | H |

Et signifie Ethyl
cPr signifie Cyclopropyl
iPr signifie isopropyl (1-méthyléthyl)
Ph signifie Phényl
*Tous les composés décrits dans le tableau sont sous forme de sel avec l'acide trifluoroacétique, à l'exception des composés marqués :
(chl) sel avec l'acide chlorhydrique
(bas) non salifiés
(fum) sel avec l'acide fumarique

**Activité biologique**

Les composés de la présente invention ont été évalués pour leur propriété analgésique dans le test de douleur induite par le formaldéhyde chez la souris (Shibata, M., Ohkubo, T., Takahashi, H. & R. Inoki. Modified formalin test: characteristic biphasic pain response. Pain, 38, 347-352). En résumé, une administration de formaldéhyde (0,92 % dans le sérum physiologique) est effectuée dans la patte arrière et la durée de léchage, qui reflète l'intensité de la douleur, est enregistrée de 0 à 5 min (1$^{ère}$ phase) et de 15 à 30 min (2$^{nde}$ phase) après l'injection. Le pourcentage d'inhibition de la seconde phase de léchage induite par le formaldéhyde est donné, pour quelques composés selon l'invention, dans le tableau suivant :

| Exemple | Dose (mg/kg) | Voie d'administration | % d'inhibition de la 2$^{nde}$ phase de léchage |
|---|---|---|---|
| 16 | 100 | p.o. | 80 |
| 9 | 100 | p.o. | 60 |
| 19 | 10 | i.p. | 42 |
| 49 | 1 | i.v. | 40 |
| p.o. : voie orale<br>i.p. : voie intrapéritonéale<br>i.v. : voie intraveineuse | | | |

Ces résultats témoignent d'une baisse très sensible de la douleur après administration des composés.

Suite aux résultats de l'essai précédent, les composés selon l'invention ont été soumis à un test visant à démontrer leur mode d'action et mettant en jeu le récepteur $B_1$ de la bradykinine.

Ce test utilise la veine ombilicale humaine et est réalisé selon le protocole suivant :

Des cordons ombilicaux humains de 15-25 cm de long sont récupérés juste après la délivrance et placés immédiatement dans un flacon contenant une solution de Krebs de composition (en mM) : NaCl 119, KCl 4,7, $KH_2PO_4$ 1,18, $MgSO_4$ 1,17, $NaHCO_3$ 25, $CaCl_2$ 2,5, Glucose 5,5, EDTA 0,026 puis stockés à 4°C.

Le cordon est disséqué sous solution de Krebs afin de dégager la veine ombilicale. La veine est nettoyée de tout tissu adhérent et coupée en petits anneaux de 3-4 mm de large. L'endothélium est enlevé précautionneusement par introduction dans la lumière du vaisseau d'un fin cathéter n°1, rendu légèrement abrasif.

Afin d'induire l'expression du récepteur $B_1$ de la bradykinine, les segments de veine sont mis à incuber à 37°C dans une cuve de 25 ml pendant 16 heures dans un milieu de culture EMEM oxygéné par un mélange 95% $O_2$ + 5% $CO_2$ auquel on ajoute des antibiotiques : pénicilline 10 000 UI/ml et streptomycine 10 000 UI/ml. Le lendemain, les anneaux de veine sont montés sur un support en acier inoxydable, relié à un capteur isométrique et placés dans une cuve à organes isolés de 8 ml thermostatée à 37°C, contenant de la solution de Krebs oxygénée par un mélange 95% $O_2$ + 5% $CO_2$.

Après une période de repos d'une heure pendant laquelle les anneaux sont rincés 5 à 6 fois avec la solution de Krebs (maintenue à 37°C pendant toute la manipulation et oxygénée par le mélange 95% $O_2$ + 5% $CO_2$ ), la veine est soumise progressivement à une tension de 1 g. Lorsque la tension est stable, après 45 minutes environ, la solution de Krebs est remplacée par une solution hyperpotassique (KPSS : à température de 37°C) de même composition, mais contenant du KCl 125 mM et pas de NaCl.

Après une série de rinçages, repos et réajustement de la tension, la contraction maximale de chaque segment est déterminée par une nouvelle dépolarisation avec la solution de KPSS.

Après une nouvelle période de repos pendant laquelle la tension à 1 g est réajustée constamment, les composés suivants sont ajoutés dans le bain d'organe isolé : Mépyramine (1μM), Atropine (1μM), Indométacine (3μM), LNA (30μM), Captopril (10μM), DL-Thiorphan (1μM) et Nifédipine (0,1 μM).

20 minutes après, la molécule à tester ou le solvant de la molécule est ajouté dans le bain d'organe isolé. Les molécules sont étudiées à 10 μM ; si une molécule présente un degré d'activité suffisant, elle est étudiée à des concentrations plus faibles (ex : 1 - 0,1-0,01 μM).

Après 15 minutes d'incubation, les segments de veine sont contractés par l'ajout de concentrations croissantes de des-Arg$^{10}$-Kallidin (0,1 nM à 30 000 nM) dans la cuve.

Les $EC_{50}$ (concentrations effectives d'agonistes requises pour produire 50% de la réponse maximale obtenue avec le KPSS) sont calculées par la méthode des moindres carrés.

Le $pK_B = [-\log K_B]$ est obtenue à partir de l'équation :

$$K_B = [A] / (\text{concentration ratio-1})$$

où [A] est la concentration d'antagoniste et la (concentration ratio) représente le rapport entre l'$EC_{50}$ en présence d'antagoniste, et l'$EC_{50}$ en l'absence d'antagoniste.

Selon ce test, les composés selon l'invention cités dans la description présentent un $pK_B$ compris entre 7 et 10, ce qui tend à démontrer que le mode d'action de ces composés fait intervenir un antagonisme au récepteur B1 de la bradykinine.

Les composés de la présente invention, sont utiles pour le traitement de diverses formes de douleur telles que l'hyperalgésie inflammatoire, l'allodynie, la douleur neuropathique associées, par exemple, au diabète, à des neuropathies (constriction du nerf sciatique, lombalgies), à toute forme de traumatisme, à une intervention chirurgicale (extraction dentaire, ablation des amygdales), à une cystite interstitielle, à une maladie inflammatoire du colon, à un cancer.

Les composés de la présente invention peuvent aussi être utiles pour traiter toute pathologie associée à un recrutement de neutrophiles comme par exemple, le syndrome de détresse respiratoire aiguë, le psoriasis, les obstructions pulmonaires chroniques, les maladies inflammatoires du colon, la polyarthrite rhumatoïde.

L'activité des composés selon l'invention, mise en évidence au cours des tests biologiques, est significative de propriétés antalgiques et permet d'envisager leur utilisation en thérapeutique.

Selon l'invention, on préconise l'utilisation des composés définis par la formule I, ainsi que de leurs sels avec des acides non toxiques, de préférence leurs sels pharmaceutiquement acceptables, en tant que principes actifs de médicaments destinés à un traitement chez les mammifères, notamment chez l'homme, vis à vis de la douleur ou de certaines maladies généralement caractérisées par une migration massive de neutrophiles.

Parmi les maladies qui peuvent être traitées au moyen d'une administration d'une quantité thérapeutiquement efficace d'au moins l'un des composés de formule I, on peut citer les hyperalgésies inflammatoires, les douleurs neuropathiques, les douleurs associées à un traumatisme ou à un cancer, les maladies inflammatoires du côlon, la polyarthrite rhumatoïde, le psoriasis, les obstructions pulmonaires chroniques ou le syndrome de détresse respiratoire aiguë.

Les composés de la présente invention, en raison de leur mode d'action, trouvent aussi leur utilité pour traiter ou prévenir tout état pathologique dans lequel les récepteurs B1 de la bradykinine sont impliqués et notamment surexprimés. En plus des diverses formes de douleur et des maladies inflammatoires déjà citées, les composés de l'invention peuvent être utiles pour traiter :

- certains problèmes respiratoires tels que l'asthme, la bronchite, la pleurésie ou les rhinites d'origine allergique ou virale,
- certaines formes de diabète,
- certaines maladies de la peau telles que les dermatites, l'eczéma, le psoriasis,
- les maladies des yeux tels que le glaucome, la rétinite,
- la maladie d'Alzheimer,
- le choc septique,
- les chocs traumatiques, notamment au niveau du crâne,
- certains cancers notamment en ralentissant ou en inhibant la prolifération des cellules cancéreuses et plus particulièrement le cancer de la prostate.

La dose de principe actif dépend du mode d'administration et du type de pathologie ; elle est généralement comprise entre 0,05 et 10 mg/kg du sujet à traiter. En fonction du traitement envisagé, les composés de formule I ou leurs sels pourront être associés à d'autres principes actifs, et seront formulés avec des excipients couramment utilisés.

Dans le but d'obtenir une action rapide, notamment lorsqu'il s'agit de traiter une douleur aiguë, le mode d'administration du médicament se fera de préférence par injection, par exemple par voie intramusculaire ou sous-cutanée. Dans le cas de douleurs chroniques, l'administration du médicament peut être faite par le moyen de formulations galéniques communes, par exemple par voie orale sous forme de gélules ou de comprimés dans lesquels un composé selon l'invention est associé à des excipients connus de l'homme du métier, ou sous forme d'un patch adhésif dans lequel un composé selon l'invention est formulé avec des excipients connus de l'homme du métier pour favoriser le passage transdermique du principe actif.

## Revendications

1. Composé dérivé d'arylsulfonamide, **caractérisé en ce qu'**il est choisi parmi l'ensemble constitué par :

a) les produits de formule:

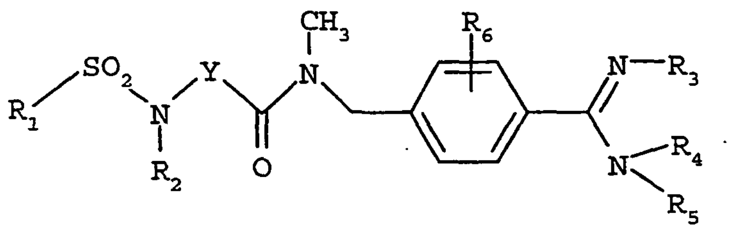

I

dans laquelle

$R_1$ représente un noyau aromatique non substitué ou substitué par un ou plusieurs des atomes ou groupes d'atomes choisi(s) parmi les halogènes, les groupes alkyle en $C_1$-$C_3$, alcoxy en $C_1$-$C_3$, nitro, cyano, trifluorométhyl ou trifluorométhoxy,
$R_2$ représente un atome d'hydrogène ou une chaîne hydrocarbonée ayant de 1 à 4 atomes de carbone, linéaire, ramifiée, ou bien encore cyclique éventuellement substitué par un groupe phényle, par un groupe $CONH_2$ ou par un ou plusieurs atomes de fluor,
$R_3$ représente un atome d'hydrogène, un groupe hydroxy, ou forme avec $R_4$ un groupe -CH=N- ou un groupe alkylène en $C_2$-$C_4$ linéaire ou ramifié,
$R_4$ représente un atome d'hydrogène ou forme avec $R_3$ un groupe -CH=N- ou un groupe alkylène en $C_2$-$C_4$ linéaire ou ramifié,
$R_5$ représente un atome d'hydrogène ou un groupe alkyle en $C_1$-$C_3$,
$R_5$ représente un atome d'hydrogène ou un halogène,
Y représente un groupe alkylène en $C_2$-$C_4$, saturé ou insaturé, linéaire ou ramifié, éventuellement interrompu entre deux atomes de carbone par un atome d'oxygène,

b) les sels d'addition des composés de formule I ci-dessus avec un acide.

2. Composé selon la revendication 1, **caractérisé en ce que** $R_1$ représente un groupe phényle substitué par un ou plusieurs atomes ou groupes d'atomes choisis parmi un atome d'halogène, de préference l'atome de chlore, et les groupes alkyle en $C_1$-$C_3$ et alcoxy en $C_1$-$C_3$.

3. Composé selon la revendication 1 ou 2, **caractérisé en ce que** $R_2$ représente un groupe alkyle en $C_1$-$C_4$.

4. Composé selon l'une des revendications 1 à 3, **caractérisé en ce que** $R_3$ et $R_4$ forment ensemble un groupe alkylène en $C_2$-$C_3$.

5. Composé selon l'une des revendications 1 à 4, **caractérisé en ce que** $R_5$ et $R_6$ représentent chacun un atome d'hydrogène.

6. Composé selon l'une des revendications 1 à 5, **caractérisé en ce que** Y représente une chaîne alkylène en $C_2$-$C_4$ saturée éventuellement interrompue par un atome d'oxygène.

7. Composé selon la revendication 6, **caractérisé en ce que** Y représente un groupe $-(CH_2)_4-$.

8. Composé selon la revendication 6, **caractérisé en ce que** Y représente un groupe $-(CH_2)_2-O-CH_2-$.

9. Procédé de préparation d'un composé de formule I tel que défini à la revendication 1, et de ses sels d'addition, comprenant les étapes consistant à :

a) faire réagir un acide de formule :

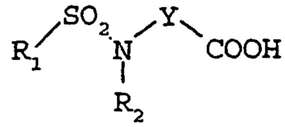

II

dans laquelle

$R_1$ représente un noyau aromatique non substitué ou substitué par un ou plusieurs des atomes ou groupes d'atomes choisi(s) parmi les halogènes, les groupes alkyle en $C_1$-$C_3$, alcoxy en $C_1$-$C_3$, nitro, cyano, trifluorométhyl ou trifluorométhoxy,
$R_2$ représente un atome d'hydrogène, un groupe alkyle en $C_1$-$C_4$ éventuellement substitué par un groupe phényle, par un groupe $CONH_2$ ou par un ou plusieurs atomes de fluor,
et Y représente un groupe alkylène en $C_2$-$C_4$, saturé ou insaturé, linéaire ou ramifié, éventuellement interrompu entre deux atomes de carbone par un atome d'oxygène,
avec une amine de formule :

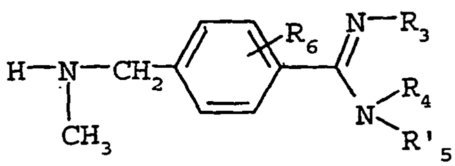

III

dans laquelle
$R_3$ représente un atome d'hydrogène ou forme avec $R_4$ un groupe alkylène en $C_2$-$C_4$ linéaire ou ramifié,
$R_4$ représente un atome d'hydrogène ou forme avec $R_3$ un groupe alkylène en $C_2$-$C_4$ linéaire ou ramifié,
$R'_5$ représente un groupe alkyle en $C_1$-$C_3$, un atome d'hydrogène ou un groupe amino-protecteur,
$R_6$ représente un atome d'hydrogène ou un halogène,
la réaction étant conduite dans un solvant en présence d'au moins un agent activateur à une température généralement comprise entre la température ambiante et 60°C et pendant environ 2 à 15 heures pour obtenir l'amide de formule

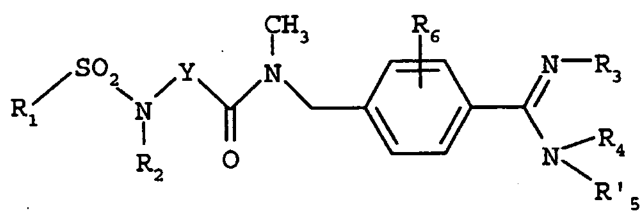

IV

dans laquelle $R_1$, $R_2$, $R_3$, $R_4$, $R'_5$, $R_6$ et Y conservent la même signification que dans les produits de départ,

b) si nécessaire, lorsque le substituant $R'_5$ est un groupe amino-protecteur, faire réagir le composé de formule IV de façon à éliminer le groupe amino-protecteur et le remplacer par un atome d'hydrogène, et ainsi obtenir le composé de formule I dans lequel $R_5$ représente un atome d'hydrogène,
c) si nécessaire, faire réagir le composé de formule IV ou I obtenu ci-dessus, avec un acide minéral ou organique, pour obtenir le sel d'addition du composé de formule IV ou I.

**10.** Composition thérapeutique, **caractérisée en ce qu'**elle renferme, en association avec au moins un excipient physiologiquement acceptable, au moins un composé de formule I selon l'une des revendications 1 à 8, ou l'un de ses sels d'addition pharmaceutiquement acceptables avec un acide.

**11.** Utilisation d'un composé de formule I selon l'une des revendications 1 à 8 ou de l'un de ses sels d'addition pharmaceutiquement acceptables avec un acide, pour la préparation d'un médicament destiné au traitement de la douleur.

**12.** Utilisation d'un composé de formule I selon l'une des revendications 1 à 8 ou de l'un de ses sels d'addition pharmaceutiquement acceptables avec un acide, pour la préparation d'un médicament destiné au traitement de maladies inflammatoires.

## Claims

**1.** Arylsulphonamide derivative, **characterized in that** it is chosen from among the group consisting of:

a) products of formula:

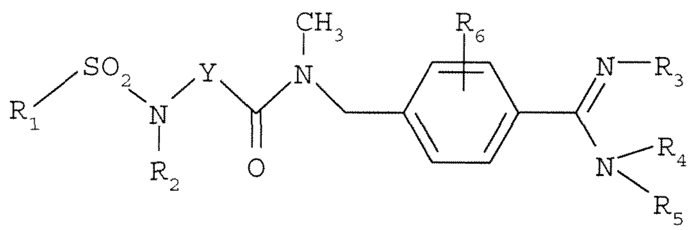

I

in which

$R_1$ represents an aromatic ring that is non-substituted or substituted by one or more atoms or groups of atoms chosen from among the halogens, $C_1$-$C_3$ alkyl groups, $C_1$-$C_3$ alcoxy groups, nitro, cyano, trifluoromethyl or trifluoromethoxy groups,
$R_2$ represents a hydrogen atom or a straight, branched or cyclic hydrocarbon chain having 1 to 4 carbon atoms optionally substituted by a phenyl group, by a $CONH_2$ group or by one or more fluorine atoms,
$R_3$ represents a hydrogen atom, a hydroxy group, or with $R_4$ forms a -CH=N-group or a straight or branched $C_2$-$C_4$ alkylene group,
$R_4$ represents a hydrogen atom or with $R_3$ forms a -CH=N- group or a straight or branched $C_2$-$C_4$ alkylene group,
$R_5$ represents a hydrogen atom or a $C_1$-$C_3$ alkyl group,
$R_6$ represents a hydrogen atom or a halogen,
Y represents a $C_2$-$C_4$ alkylene group, saturated or unsaturated, straight or branched, optionally interrupted between two carbon atoms by an oxygen atom,

b) the addition salts of the above formula I compounds with an acid.

**2.** Compound according to claim 1, **characterized in that** $R_1$ represents a phenyl group substituted by one or more atoms or groups of atoms chosen from among a halogen atom, preferably the chlorine atom, and $C_1$-$C_3$ alkyl groups and $C_1$-$C_3$ alkoxy groups.

**3.** Compound according to claim 1 or 2, **characterized in that** $R_2$ represents a $C_1$-$C_4$ alkyl group.

**4.** Compound according to any of claims 1 to 3, **characterized in that** $R_3$ and $R_4$ together form a $C_2$-$C_3$ alkylene group.

**5.** Compound according to any of claims 1 to 4, **characterized in that** $R_5$ and $R_6$ each represent a hydrogen atom.

**6.** Compound according to any of claims 1 to 5, **characterized in that** Y represents a saturated $C_2$-$C_4$ alkylene chain optionally interrupted by an oxygen atom.

**7.** Compound according to claim 6, **characterized in that** Y represents a $-(CH_2)_4-$ group.

**8.** Compound according to claim 6, **characterized in that** Y represents a $-(CH_2)_2-O-CH_2-$ group.

**9.** Method for preparing a formula I compound such as defined in claim 1, and its addition salts, comprising the steps consisting of:

a) reacting an acid of formula:

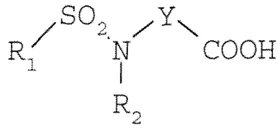

II

in which

$R_1$ represents an aromatic ring that is non-substituted or substituted by one or more atoms or groups of atoms chosen from among the halogens, $C_1$-$C_3$ alkyl groups, $C_1$-$C_3$ alkoxy groups, nitro, cyano, trifluoromethyl or trifluoromethoxy groups,
$R_2$ represents a hydrogen atom, a $C_1$-$C_4$ alkyl group optionally substituted by a phenyl group, by a $CONH_2$ group or by one or more fluorine atoms,
and Y represents a $C_2$-$C_4$ alkylene group, saturated or unsaturated, straight or branched, optionally interrupted between two carbon atoms by an oxygen atom,
with an amine of formula:

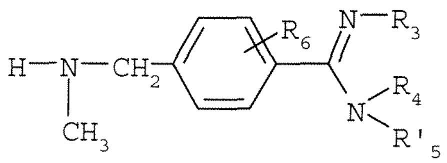

III

in which
$R_3$ represents a hydrogen atom or with $R_4$ forms a straight or branched $C_2$-$C_4$ alkylene group,
$R_4$ represents a hydrogen atom or with $R_3$ forms a straight or branched $C_2$-$C_4$ alkylene group,
$R'_5$ represents a $C_1$-$C_3$ alkyl group, a hydrogen atom or an amino-protecting group,
$R_6$ represents a hydrogen atom or a halogen,
the reaction being conducted in a solvent in the presence of at least one activator agent at a temperature generally lying between room temperature and 60°C and for approximately 2 to 15 hours to obtain the amide of formula:

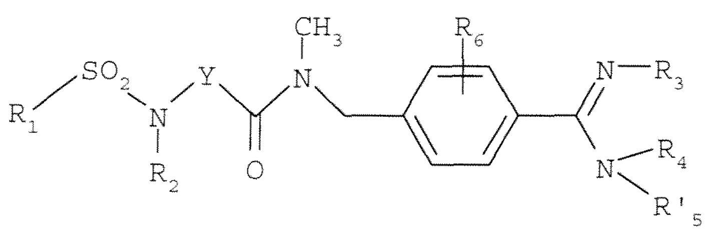

IV

in which $R_1$, $R_2$, $R_3$, $R_4$, $R'_5$, $R_6$ and Y maintain the same meanings as in the starting products,

b) if necessary, when the substituent $R'_5$ is an amino-protecting group, reacting the formula IV compound so as to remove the amino-protecting group and replace it by a hydrogen atom, thereby obtaining the formula I

compound in which $R_5$ represents a hydrogen atom,
c) if necessary, reacting the formula IV or formula I compound obtained above with a mineral or organic acid to obtain the addition salt of the formula IV or formula I compound.

**10.** Therapeutic composition, **characterized in that**, in association with at least one physiologically suitable excipient, it contains at least one formula I compound according to any of claims 1 to 8, or one of its pharmaceutically acceptable addition salts with an acid.

**11.** Use of a formula I compound as in any of claims 1 to 8, or of one of its pharmaceutically acceptable addition salts with an acid, for the preparation of a medicinal product intended to treat pain.

**12.** Use of a formula I compound as in any of claims 1 to 8, or of one of its pharmaceutically acceptable addition salts with an acid, for the preparation of a medicinal product intended to treat inflammatory diseases.

## Patentansprüche

**1.** Abgeleitete Arylsulfonamidverbindung, **dadurch gekennzeichnet, daß** sie aus der Einheit ausgewählt ist, die besteht aus:

a) den Produkten der Formel:

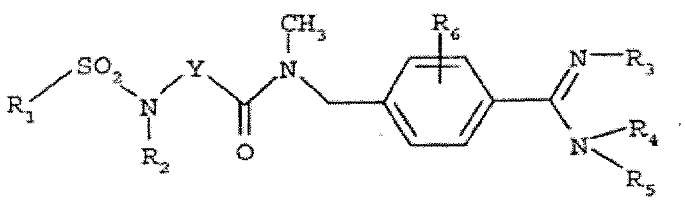

I

worin

$R_1$ einen aromatischen Kern, unsubstituiert oder substituiert durch ein oder mehrere der Atome oder Atomgruppen, ausgewählt unter den Halogenen, den $C_1$-$C_3$-Alkyl-, $C_1$-$C_3$-Alkoxy-, Nitro-, Cyano-, Trifluormethyl- oder Trifluormethoxygruppen, darstellt,
$R_2$ ein Wasserstoffatom oder eine lineare, verzweigte oder aber zyklische Kohlenwasserstoffkette mit 1 bis 4 Kohlenstoffatomen, eventuell substituiert durch eine Phenylgruppe, durch eine $CONH_2$-Gruppe oder durch ein oder mehrere Fluoratome, darstellt,
$R_3$ ein Wasserstoffatom, eine Hydroxygruppe darstellt oder mit $R_4$ eine -CH=N-Gruppe oder eine lineare oder verzweigte $C_2$-$C_4$-Alkylengruppe bildet,
$R_4$ ein Wasserstoffatom darstellt oder mit $R_3$ eine -CH=N-Gruppe oder eine lineare oder verzweigte $C_2$-$C_4$-Alkylengruppe bildet,
$R_5$ ein Wasserstoffatom oder eine $C_1$-$C_3$-Alkylgruppe darstellt,
$R_6$ ein Wasserstoffatom oder ein Halogen darstellt,
Y eine $C_2$-$C_4$-Alkylengruppe, gesättigt oder ungesättigt, linear oder verzweigt, eventuell zwischen zwei Kohlenstoffatomen durch ein Sauerstoffatom unterbrochen, darstellt,

b) den Additionssalzen der Verbindungen der obigen Formel I mit einer Säure.

**2.** Verbindung nach Anspruch 1, **dadurch gekennzeichnet, daß** $R_1$ eine Phenylgruppe, substituiert durch ein oder mehrere Atome oder Atomgruppen, ausgewählt unter einem Halogenatom, vorzugsweise Chloratom, und den $C_1$-$C_3$-Alkyl-und $C_1$-$C_3$-Alkoxygruppen darstellt.

**3.** Verbindung nach Anspruch 1 oder 2, **dadurch gekennzeichnet, daß** $R_2$ eine $C_1$-$C_4$-Alkylgruppe darstellt.

**4.** Verbindung nach einem der Ansprüche 1 bis 3, **dadurch gekennzeichnet, daß** $R_3$ und $R_4$ zusammen eine $C_2$-$C_3$-Alkylengruppe bilden.

5. Verbindung nach einem der Ansprüche 1 bis 4, **dadurch gekennzeichnet, daß** $R_5$ und $R_6$ jeweils ein Wasserstoffatom darstellen.

6. Verbindung nach einem der Ansprüche 1 bis 5, **dadurch gekennzeichnet, daß** Y eine gesättigte, eventuell durch ein Sauerstoffatom unterbrochene $C_2$-$C_4$-Alkylenkette darstellt.

7. Verbindung nach Anspruch 6, **dadurch gekennzeichnet, daß** Y eine $-(CH_2)_4$-Gruppe darstellt.

8. Verbindung nach Anspruch 6, **dadurch gekennzeichnet, daß** Y eine $-(CH_2)_2-O-CH_2$-Gruppe darstellt.

9. Verfahren zur Herstellung einer Verbindung der Formel I wie sie in Patentanspruch 1 definiert ist, sowie ihrer Additionssalze, das die Schritte umfaßt, die darin bestehen:

a) eine Säure der Formel:

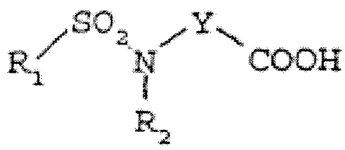

II

worin

$R_1$ einen aromatischen Kern, unsubstituiert oder substituiert durch ein oder mehrere der Atome oder Atomgruppen, ausgewählt unter den Halogenen, den $C_1$-$C_3$-Alkyl-, $C_1$-$C_3$-Alkoxy-, Nitro-, Cyano-, Trifluormethyl- oder Trifluormethoxygruppen, darstellt,
$R_2$ ein Wasserstoffatom, eine $C_1$-$C_4$-Alkylgruppe eventuell substituiert durch eine Phenylgruppe, durch eine $CONH_2$-Gruppe oder durch ein oder mehrere Fluoratome, darstellt, und
Y eine $C_2$-$C_4$-Alkylengruppe, gesättigt oder ungesättigt, linear oder verzweigt, eventuell zwischen zwei Kohlenstoffatomen durch ein Sauerstoffatom unterbrochen, darstellt,
mit einem Amin folgender Formel reagieren zu lassen:

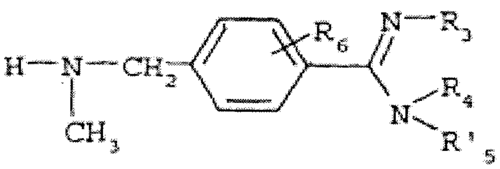

III

worin
$R_3$ ein Wasserstoffatom darstellt oder mit $R_4$ eine lineare oder verzweigte $C_2$-$C_4$-Alkylengruppe bildet,
$R_4$ ein Wasserstoffatom darstellt oder mit $R_3$ eine lineare oder verzweigte $C_2$-$C_4$-Alkylengruppe bildet,
$R'_5$ eine $C_1$-$C_3$-Alkylgruppe, ein Wasserstoffatom oder eine Aminoschutzgruppe darstellt,
$R_6$ ein Wasserstoffatom oder ein Halogen darstellt,
wobei die Reaktion in einem Lösungsmittel in Gegenwart wenigstens eines Aktivierungsmittels bei einer Temperatur im allgemeinen zwischen der Umgebungstemperatur und 60°C und für etwa 2 bis 15 Stunden durchgeführt wird, um das Amid folgender Formel zu erhalten

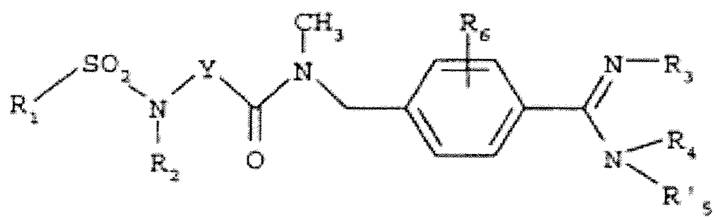

IV

worin $R_1$, $R_2$, $R_3$, $R_4$, $R'_5$, $R_6$ und Y die gleiche Bedeutung wie bei den Ausgangsprodukten beibehalten,

b) falls erforderlich, wenn der Substituent $R'_5$ eine Aminoschutzgruppe ist, die Verbindung der Formel IV derart reagieren zu lassen, daß die Aminoschutzgruppe beseitigt und sie durch ein Wasserstoffatom ersetzt wird und daß so die Verbindung der Formel I erhalten wird, worin $R_5$ ein Wasserstoffatom darstellt,
c) falls erforderlich die oben erhaltene Verbindung der Formel IV oder I mit einer mineralischen oder organischen Säure reagieren zu lassen, um das Additionssalz der Verbindung der Formel IV oder I zu erhalten.

**10.** Therapeutische Zusammensetzung, **dadurch gekennzeichnet, daß** sie in Verbindung mit wenigstens einem physiologisch akzeptablen Bindemittel wenigstens eine Verbindung der Formel I nach einem der Ansprüche 1 bis 8 oder eines ihrer pharmazeutisch akzeptablen Additionssalze mit einer Säure enthält.

**11.** Verwendung einer Verbindung der Formel I nach einem der Ansprüche 1 bis 8 oder eines ihrer pharmazeutisch akzeptablen Additionssalze mit einer Säure für die Herstellung eines Medikaments, das für die Schmerzbehandlung bestimmt ist.

**12.** Verwendung einer Verbindung der Formel I nach einem der Ansprüche 1 bis 8 oder eines ihrer pharmazeutisch akzeptablen Additionssalze mit einer Säure für die Herstellung eines Medikaments, das für die Behandlung von Entzündungskrankheiten bestimmt ist.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 236163 A
- EP 236164 A
- EP 614911 A
- EP 558961 A
- WO 9216549 A1
- WO 9725315 A
- WO 0034313 A
- WO 9640639 A
- WO 9724349 A
- WO 9803503 A
- WO 9824783 A
- WO 9900387 A


### Littérature non-brevet citée dans la description


- *Pharmazie,* 1984, vol. 39 (5), 315-317
- *Pharmazie,* 1986, vol. 41 (4), 233-235
- *J. Chem. Soc., Perkin Trans.,* 1986, vol. 1 (9), 1655-64
- **SHIBATA, M. ; OHKUBO, T. ; TAKAHASHI, H. ; R. INOKI.** Modified formalin test: characteristic biphasic pain response. *Pain,* vol. 38, 347-352